# EUROPEAN PATENT APPLICATION

(11) **EP 4 328 311 A1**
(43) Date of publication of application: **28.02.2024**
(21) Application number: 22791116.1
(22) Date of filing: 21.04.2022
(51) Int. Cl.: C12N 15/82, C12N 15/40, A01H 5/00, A01H 4/00

(54) **NEGATIVE-STRAND RNA VIRAL VECTOR AND PLANT GENOME EDITING METHOD WITHOUT TRANSFORMATION**

(30) Priority: 21.04.2021 CN 202110431464
(71) Applicant: Zhejiang University, Hangzhou, Zhejiang 310058 (CN)
(72) Inventor: LI, Zhenghe, Hangzhou, Zhejiang 310058 (CN); LIU, Qian, Hangzhou, Zhejiang 310058 (CN); ZHAO, Chenglu, Hangzhou, Zhejiang 310058 (CN)
(74) Representative: van Dam, Vincent
(86) International application number: PCT/CN2022/088299
(87) International publication number: WO 2022/223010

(57) **Abstract**

Provided are a method for carrying out site-directed modification on a plant genetic material by using a negative-strand RNA viral vector and a viral vector system. Also provided is a method for performing site-directed editing on DNA of genomic interest of a plant without the need for the stable genetic transformation of a plant to be modified, comprising the steps of: infecting a plant with a tomato spotted wilt viral vector and transiently expressing a sequence-specific nucleic acid modification enzyme which targets a target site of the infected plant genome and cleaves or modifies the target site, a DNA repair mechanism of the plant completing targeted modification of the target site. The delivery of sequence-specific nucleic acid modification enzymes by using RNA viral vectors not only improves the editing efficiency of target sites, but can also obtain non-transgenic editing plants.

## Description

### TECHNICAL FIELD OF THE DISCLOSURE

The present disclosure belongs to the field of plant genetic engineering and relates to methods for non-transgenic plant genome editing. More particularly, but not exclusively, the present disclosure relates to methods of generating targeted mutation in plant genome by delivering CRISPR/Cas nuclease to plants using engineered plant negative-stranded RNA viral vector systems, whereby plants with modified genetic material and their progeny are produced.

### BACKGROUND OF THE DISCLOSURE

The development of sequence-specific endonucleases (SSNs) technologies has made targeted genome editing possible. SSNs comprise a sequence-specific DNA-binding domain and a non-specific DNA cleavage domain. Upon introduction into cells, SSNs target specific sites in the genome, creating double-strand breaks (DSBs) in DNA. This activates the cell's non-homologous end joining (NHEJ) mechanism to repair DSBs. This repair process often leads to base insertions or deletions, resulting in mutations in the target sequence. Additionally, when homologous DNA sequences are present within the cell, the homologous recombination (HR) machinery can utilize homologous templates to repair DSBs, enabling genetic modifications, allelic gene replacement, or targeted insertion of exogenous genes into the target sequence. Furthermore, SSN variants that cannot generate DSBs can be fused with different effector proteins, e.g., transcriptional regulatory proteins, cytosine deaminases, adenine deaminases, reverse transcriptases, to enable targeted gene transcription regulation, base editing, insertion, or deletion of single or multiple bases.

Commonly used SSNs include zinc finger nucleases (ZFNs), transcription activator-like effector nucleases (TALENs), and the Clustered Regularly Interspaced Short Palindromic Repeats/CRISPR-associated proteins (CRISPR/Cas) system. Among these, CRISPR/Cas technology requires only the introduction of Cas nucleases and target site-specific guide RNA into cells. It is widely utilized in animal and plant genome editing due to its ease of use and high efficiency.

One of the critical steps in genome editing technology is the delivery of sequence-specific nucleases (SSNs), i.e., the methods and means to introduce SSNs into recipient cells. The rigid plant cell walls hinder the direct transfection of SSN molecules, necessitating genetic transformation methods, such as *Agrobacterium-mediated* transformation or gene gun bombardment, to deliver SSNs in the form of transgenes into plant cells. However, this method presents several challenges when applied in crop genetic improvement: i) Most crop species or varieties exhibit low transformation efficiency; ii) The segregation of SSN transgenes in the progeny of edited plants relies on sexual hybridization, which is time-consuming and labor-intensive, especially for polyploid or asexually reproduced plants, or plants with long breeding cycle; iii) The stable genetic transformation process can cause unpredictable changes and damage to plant chromosomal DNA; iv) Products involving the genetic transformation process may be categorized as genetically modified organisms (GMOs) and face regulatory scrutiny in certain countries.

Viral vectors are natural biomacromolecule delivery devices widely used for introducing and transiently expressing foreign genes in plants (e.g., Donson et al., Proc Natl Acad Sci U S A, 1991, 88: 7204-7208; Wang et al., PLoS Pathog, 2015, 11: e1005223). Particularly, RNA viral vectors, not involving a DNA stage during replication, bear negligible risk of integrating foreign nucleic acids into the host genome. Infection of plants with plant viruses is faster and simpler than stable genetic transformation and viral replication can result in high levels of foreign gene expression. Moreover, infectious viral vectors can achieve in planta delivery of foreign genes within the infected plant tissues without the need for isolating specific recipient cells or tissues. Currently, various plant viruses have been engineered as vectors for the direct delivery of nucleic acids or expression of foreign proteins within the plant (Gleba et al., Curr Opin Plant Biol, 2004, 7: 182-188; Cody and Scholthof, Annu Rev Phytopathol, 2019, 57: 211-230).

In plant genome editing, the use of viral vectors as a delivery tool is limited by the large sizes of CRISPR/Cas nucleases. The sizes of commonly used Cas9 and Cas12a nucleases (approximately 4 kb), together with regulatory elements, can reach 5-7 kb, presenting a significant challenge to the packaging capacity of viral vectors. Due to the cargo size limitation, some infectious plant viral vectors have been used to deliver the smaller CRISPR guide RNAs (gRNAs), and the list includes the single-stranded DNA cabbage leaf curl virus (Yin et al., SciRep, 2015, 5: 14926), andthepositive-senseRNAvirustobacco rattle virus (Ali et al., Mol Plant, 2015, 8: 1288-1291; patents WO 2015189693; WO 2016084084), tobacco mosaic virus (Cody et al., Plant Physiol, 2017, 175: 23-35), pea early browning virus (Ali et al., Virus Res, 2018, 244: 333-337), beet necrotic yellow vein virus (Jiangetal., PlantBiotechnolJ, 2019, 17: 1302-1315), and foxtail mosaic virus (Mei et al., Plant Direct, 2019, 3: 1-16) . In these cases, the guide RNAs delivered by these viral vectors collaborate with the Cas9 protein provided by an integrated transgene to achieve genome editing. Additionally, some plant viral vectors with relatively larger packaging capacities have been used to deliver other nuclease components . For example, tobacco rattle virus has been used to deliver meganucleases of approximately 1 kb (Honig et al., Mol Plant, 2015, 8: 1292-1294) or zinc finger nucleases of approximately 2 kb (Marton et al., Plant Physiol, 2010, 154: 1079-1087; patent WO 2009130695), or split CRISPR/Cas nuclease components (U.S. Patent Application US 20190359993).

A viral replicon system is a class of vectors with a large cargo capacity that can replicate but cannot infect plants systematically. Baltes et al. (Plant Cell, 2014, 151-163) disclose the delivery of CRISPR/Cas nucleases using a geminivirus replicon derived from bean yellow dwarf virus (BeYDV) . Wang et al. (Mol Plant, 2017, 10: 1007-1010; Chinese patent CN 109880846 A) disclose the delivery of CRISPR/Cas nucleases and DNA repair templates using a wheat dwarf virus (WDV) replicon. A technique for delivering CRISPR/Cas nuclease utilizing the PVX vector or tomato mosaic virus vector is describedbyAirga et al. (Plant Cell Physiol, 2020, 61: 1946-1953) and a US patent (US 20190359993) . A technique for delivering CRISPR/Cas nucleases into plant single cells via a barley yellow streak mosaic virus vector is described in Gao et al. (New Phytol, 2019, 223: 2120-2133) and a Chinese patent (CN 110511955 A). The above viral replicon vectors are unable to infect plants systemically and need to be introduced into cells by means of *Agrobacterium* transformation or biolistic transformation, which can express the complete CRISPR/Cas nuclease and enable gene editing in the inoculated cells.

Plant negative-stranded RNA viruses mainly include the non-segmented negative-stranded RNA viruses in the family *Rhabdoviridae* and the segmented viruses in the order *Bunyaviridae.* A few reports documented the development of plant negative-stranded RNA virus vectors. Ma et al. (Nature Plants, 2020, 6: 773-779) disclose a method to systematically deliver CRISPR/Cas nucleases and achieve DNA-free gene editing in *N*. *benthamiana* using Sonchus yellow net rhabdovirus (SYNV) vector. However, there is little report on the delivery of Cas nucleases in different crops due to the limitation of the SYNV host range. Feng et al. (Proc Natl Acad Sci USA, 2020, 117: 1181-1190) report a method for reverse genetic manipulation of tomato spotted wilt virus.

### SUMMARY OF THE DISCLOSURE

To overcome the current technical challenges in achieving non-transgenic targeted genome editing in plants using plant viral vectors, the present invention utilizes a tospovirus, tomato spotted wilt virus (TSWV)-based vector to deliver sequence-specific nucleic acid modifying enzyme into plants, wherein said nucleic acid modifying enzyme targets specific DNA sequences in the plant genome and cleaves or modifies the target sites. Precise modifications at the target sites are achieved through the plant's endogenous DNA repair mechanisms. Furthermore, the TSWV vectors employed in this invention are capable of systemic infection to enable the delivery of sequence-specific nucleases in infected whole plants. The procedure results in gene-edited plant parts without the integration of exogenous nucleotide sequences. Additionally, a method is provided to obtain non-transgenic plants with heritable gene edits from the edited plant parts (such as plant cells) through tissue culture and plant regeneration. Furthermore, the edited plant parts (such as plant cells) without integrated exogenous DNA sequences, when subjected to antiviral drug treatment during the tissue culture process, result in the recovery of non-transgenic, virus-free plants with heritable targeted gene modifications.

In one aspect of the present disclosure, a method is provided to modify the genetic material of a plant cell without the need for introducing exogenous genetic material into the plant genome, comprising: a) providing at least one plant cell to be modified; b) providing a tospovirus vector comprising at least one polynucleotide sequence encoding a sequence-specific nucleic acid modifying enzyme; c) infecting the plant cell with the tospovirus vector to allow transient expression of the sequence-specific nucleic acid modifying enzyme, wherein the nucleic acid modifying enzyme targets and cleaves or modifies a genomic sequence in the infected plant cells, and wherein the plant DNA repair machinery completes the modification of genetic material of the plant cells.

In certain embodiments, the present disclosure also provides a method for generating genetically modified plants without the need for introducing exogenous DNA into the plant genome, comprising: a) providing at least one plant cell to be modified; b) providing a tospovirus vector comprising at least one polynucleotide sequence encoding a sequence-specific nucleic acid modifying enzyme; c) infecting said plant cell with said tospovirus vector to allow transient expression of said nucleic acid modifying enzyme, wherein the nucleic acid modifying enzyme targets and cleaves or modifies a genomic sequence in the infected plant cells, and wherein the plant DNA repair machinery completes the modification of genetic material of the plant cells; d) obtaining a genetically modified plant from the modified plant cell.

In some embodiments, the method further comprises selecting a genetically modified plant cell or plant, wherein the selection does not require a selectable marker.

In some embodiments, said genetic modifications comprise nucleotide deletion, insertion or substitution of one or more nucleotides, or a combination thereof, at the target DNA sequence.

In some embodiments, the sequence-specific nucleic acid modifying enzyme is a nuclease, its mutated forms or derivatives capable of modifying genomic DNA sequence, i.e., CRISPR/Cas nucleases, TALEN nucleases, zinc finger nucleases, meganuclease. In certain embodiments, the nuclease is the *Streptococcus pyogenes* CRISPR/SpCas9. In certain embodiments, the nuclease is the *Lachnospiraceae bacterium* CRISPR/LbCas12a (LbCpf1).

In some embodiments, the sequence-specific nucleic acid modifying enzyme is a nucleotide base-modifying enzyme, for example, an adenine base editor comprising a Cas polypeptide fused to an adenine deaminase, a cytidine base editor comprising a Cas polypeptide with a cytidine deaminase . In certain preferred embodiments, the Cas polypeptide is a nickase variant of *Streptococcus pyogenes* SpCas9, and the adenine deaminase is an artificially evolved *E. coli* tRNA adenine deaminase A (ecTadA) variant (TadA8e V106W), and the cytidine deaminase is a human-derived Apo B messenger RNA cytidine deaminase catalytic subunit 3A (hAPOBEC3A; hA3A).

In some embodiments, the recombinant tospovirus vector system is the TSWV vector comprising the S, M, and L genome segments having at least 70%, at least 80%, at least 90%, at least 95%, at least 99% or 100% sequence homologies to the nucleic acid sequences as set forth in SEQ ID NO: 1, SEQ ID NO: 2, and SEQ ID NO: 3, respectively.

In some embodiments, the TSWV vectors provided by the present disclosure contain one or more (e.g., 2, 3, 4, 5, 6, 7, 8, 9, 10, or more) exogenous gene insertion sites. In certain embodiments, the recombinant TSWV vector carries a reporter gene in the S genomic segment and another reporter gene in the M genomic segment, with the reporter gene being operably linked to viral mRNA transcription regulatory cis-elements. Preferably, the reporter genes are the green fluorescent protein gene (*GFP*) and red fluorescent protein gene (*RFP*). After introducing the recombinant viral vector constructs into plant cells, virus replication, and systemic infection can be tracked by the expressed fluorescent reporters.

In some embodiments, the TSWV S segment comprises at least one heterologous polynucleotide sequence encoding a sequence-specific nuclease component being operably linked to viral mRNA transcription regulatory cis-elements; preferably, the nuclease component is a chimeric CRISPR guide RNA polynucleotide molecule.

In some embodiments, the TSWV non-structural protein gene in the S segment (NSs) is replaced by heterologous polynucleotide sequences encoding a fusion of the sequence-specific nuclease component to heterologous sequences encoding, for example, a fluorescent reporter. In some embodiments, the sequence-specific nuclease component is a guide RNA. In certain embodiments, the heterologous polynucleotide sequence inserted into said S genome encodes one guide RNA. In other embodiments, the heterologous polynucleotide sequence encodes two or more guide RNAs.

In some embodiments, the TSWV M segment comprises at least one heterologous polynucleotide sequence operably linked to viral transcription regulatory elements and encoding a Cas polypeptide, optionally selected from the group consisting of *Streptococcus pyogenes* SpCas9, *Lachnospiraceae bacterium* LbCas12a (LbCpf1), or an adenine base editor or cytosine base editor comprising a Cas9 variant fused to a base deaminase. In some preferred embodiments, the coding sequences of LbCas12a, SpCas9, adenine base editor, and cytosine base editor polypeptides are plant codon-optimized sequences; preferably, as set forth in SEQ ID NO: 10, 11, 84, 85, respectively.

In some embodiments, the viral glycoprotein precursor (GP) gene sequence in the TSWVM segment is replaced by a heterologous polynucleotide sequence encoding a sequence-specific nuclease component.

In some embodiments, the viral RNA-dependent RNA polymerase (RdRp) gene sequence in the TSWV L segment is plant codon-optimized as set forth in SEQ ID NO: 5.

In some embodiments, the tospovirus vector is an infectious TSWV vector devoid of a functional NSs protein. In some embodiments, the TSWV vector is a non-insect-transmissible tospovirus vector devoid of a functional GP protein.

In another aspect, the present disclosure provides methods for introducing an infectious TSWV vector into plant cells for the transient expression of a sequence-specific nucleic acid modifying enzyme. In certain embodiments, the nucleic acid modifying enzyme is a CRISPR/LbCas12a or CRISPR/SpCas9 nuclease . In certain embodiments, the nucleic acid modifying enzyme is an adenine base editor or cytosine base editor.

In some embodiments, the method for infecting a plant is through introducing viral nucleic acid constructs into plant tissues under conditions sufficient to initial recombinant virus infection. In certain embodiments, the viral nucleic acid constructs are *Agrobacterium* binary plasmids containing transcriptional regulatory cis-elements, including a promoter and a terminator, and the constructs are delivered into plant tissue through agroinfiltration.

In some embodiments, the viral nucleic acid constructs being introduced into the plant tissues further comprise helper constructs for expressing one or more viral suppressors of RNA silencing suppressors (VSRs). In certain embodiments, the VSR is selected from the group consisting of tomato bushy stunt virus (TBSV) p19, tobacco etch virus (TEV) Pl/Hc-Pro, barley stripe mosaic virus BSMV γb, or TSWV NSs.

In some embodiments, the binary constructs produce the positive- or negative-sense viral RNA transcripts corresponding to the chimeric sequence of the recombinant TSWV S, M, and L segments. In certain preferred embodiments, the binary constructs produce positive-sense RNA transcripts.

In some embodiments, the polynucleotide constructs comprising the TSWV L, M, and S segments are delivered into a plant cell in relative molar ratios of 1-2 (L): 2-10 (M): 1-2 (S), preferably in a relative ration of 1: 10: 2. In certain embodiments, the ratios of the nucleic acid constructs are the ratios of the relative concentration (OD₆₀₀) of *Agrobacterium* strains carrying different binary plasmids.

In some embodiments, the method for infecting a plant with the TSWV vector is to use recombinant virus particles through mechanical rubbing, pressurized spraying, and plant grafting.

In some embodiments, the plant to be genetically modified is selected from the group consisting of natural or experimental host plants that can be systemically or locally infected by a tospovirus, including, but are not limited to, *Nicotiana benthamiana,* common tobacco (*N. tabacum*)*,* tomato (*Solanum lycopersicum*), sweet pepper (*Capsicum annuum*)*,* chili pepper (*C. frutescens*), habanero pepper (*C. chinense*) , peanut (*Arachis hypogaea*), potato (*Solanum tuberosum*), ground cherry (*Physalis alkekengi*), eggplant (*Solanummelongena*), soybean (*Glycinemax*), cotton (*Gossypium hirsutum*), lettuce (*Lactuca sativa*)*,* spinach (*Spinacia oleracea*), watermelon (*Citrullus lanatus*), cucumber (*Cucumis sativus*)*,* broad bean (*Vicia faba*), black mung bean (*Vigna mungo*), green bean (*Vigna radiata*) or cowpea (*Vigna unguiculata*)*.*

In some embodiments, the plants to be genetically modified comprise different varieties or genotypes of viral host species . In some preferred embodiments, the plant varieties include but are not limited to, a plurality of peanut varieties, such as Huayu 25, Huayu 32, Huayu 39, Huayu 48, Huayu 9616, Fengyou 68, Honghua 1, Dabaisha, Silihong, and Shuofeng 518. In other preferred embodiments, the plant varieties include but are not limited to, a plurality of sweet pepper varieties, such as Zhoula 2, Zhoula 3, Zhoula 4, Fuxiang Xiuli, Bola 5, Bola 7, Bola 15, Xingshu 201, Xingshu 301, and Xingshu Guiyan.

In some embodiments, the infectious TSWV vectors introduced into plant cells express the LbCas12a polypeptide and a single guide RNA forms a nuclease complex targeting the *Nicotiana benthamiana* crFucT (SEQ ID NO: 86) or the crDCL2 target sites (SEQ ID NO:87), or the conserved crPDS1 target site in *N*. *benthamiana* and *N*. *tabacum* ( SEQ ID NO: 12), or the tomato crPDS2 target site (SEQ ID NO: 13), or the pepper crPDS3 (SEQ ID NO: 14) or crPDS6 target sites (SEQ ID NO: 99), or the peanut and soybean crPDS4 target site (SEQ ID NO: 15), or the peanut crPDS5 target site (SEQ ID NO: 98), or the ground cherry crER target site (SEQ ID NO: 88) .

In some embodiments, the infectious TSWV vectors express the LbCas12a polypeptide and multiple guide RNAs (e.g., 2, 3, 4, 5, 6, 7, 8, 9, 10, or more) in tandem to form a plurality of nuclease complexes targeting a plurality (e.g., 2, 3, 4, 5, 6, 7, 8, 9, 10, or more) of target site sequences. In some preferred embodiments, the two guide RNAs are expressed in tandem to target the *N*. *benthamiana* crPDS1 and crFucT target site, or the three guide RNAs are expressed in tandem to target the *N. benthamiana* crPDS1, crFucT, and crDCL2 target sites, respectively.

In some embodiments, the infectious TSWV vectors express the SpCas9 polypeptide and a single guide RNA to form a nuclease complex targeting the gGFP target site (SEQ ID NO: 16) in the GFP-transgenic *N*. *benthamiana* or the *N*. *benthamiana* gPDS2 (SEQ ID NO: 89), gRDR6 ( SEQ ID NO: 90), or gSGS3 (SEQ ID NO: 91) target sites, or the gPDS1 target sequence (SEQ ID NO: 17) conserved in both *N*. *benthamiana* and *N*. *tabacum.*

In some embodiments, the infectious TSWV vectors express a SpCas9 polypeptide and multiple guide RNAs (e.g., 2, 3, 4, 5, 6, 7, 8, 9, 10, or more) in tandem, forming a plurality of nuclease complexes targeting a plurality (e.g., 2, 3, 4, 5, 6, 7, 8, 9, 10, or more) of target sequences. In some preferred embodiments, the two guide RNAs are expressed in tandem targeting the *N*. *benthamiana* gPDS2 and gRDR6 targets, respectively, or the three guide RNAs are expressed in tandem targeting the gPDS2, gRDR6, and gSGS3 targets, respectively.

In some embodiments, the infectious TSWV vectors express an adenine base editor polypeptide and a guide RNA, forming an adenine base editor complex targeting the *N*. *benthamiana* gPDSa (SEQ ID NO: 92), or gFucTa1 (SEQ ID NO: 93), or gBBLd (SEQ ID NO: 97) target site sequences.

In some embodiments, infectious TSWV vectors express a cytosine base editor polypeptide and a guide RNA forming a base editor nuclease complex targeting the *N*. *benthamiana* gFucTa2 (SEQ ID NO: 94) or gRDR6a1 (SEQ ID NO: 95), or gRDR6a2 (SEQ ID NO: 96) target site sequences.

In another aspect, the present disclosure provides methods for generating plants from viral vector-infected plant cells with modified genetic material and for further selecting plants with modified genetic material. The plant cells in the present disclosure may be cells from an intact plant or isolated plant cells, such as callus tissue, suspension cells, and protoplasts. In some preferred embodiments, the plant cells are derived from infected intact plants.

In some embodiments, virus-infected plant cells or tissues are used as explants for tissue culture and plant regeneration on media without a selection marker (e.g., antibiotics, herbicides, etc.) to obtain plants with modified genetic material.

In some embodiments, the method further comprises selecting the plants with modified genetic material from the regenerated plants without using a selection maker (e.g., antibiotics, herbicides). In certain embodiments, the method of selecting plants with modified genetic material involves the use of restriction endonuclease protection analysis, Sanger sequencing analysis, and high-throughput sequencing (HTS) analysis to identify plants with genetic modifications.

In some embodiments, cells from the upper leaf tissues of *N. benthamiana* plants infected with TSWV vectors are cultured in vitro to regenerate plants with modified genetic material but without exogenous DNA.

In some embodiments, cells from the upper leaf tissues of tobacco plants infected with TSWV vectors are cultured in vitro to regenerate plants with modified genetic material but without exogenous DNA.

In some embodiments, cells from the upper leaf tissues of tomato plants infected with TSWV vectors are cultured in vitro to regenerate plants with modified genetic material but without exogenous DNA.

In some embodiments, the present disclosure further compares the effects of the TSWV vector with or without the *NSs* gene on plant cell differentiation and regeneration and finds that NSs has an inhibitory effect on plant regeneration. In some preferred embodiments, cells infected with the TSWV vectors deficient in *NSs* are used to regenerate plants with modified genetic material by tissue culture.

In another aspect of the present invention there is provided a method for generating virus-free plants from cells infected with a tospovirus by tissue culture, comprising: a) providing at least one plant cell infected with a tospovirus; b) in vitro culturing the infected plant cells on a medium containing an antiviral compound; c) obtaining and identifying virus-free regenerated plants.

In some embodiments, treatments with the antiviral compound ribavirin result in a 100% TSWV clearance rate, compared with a 4.4% clearance rate without treatments without an antiviral.

In some embodiments, treatments with the antiviral compound favipiravir result in a 66.7% TSWV clearance rate.

In some embodiments, treatments with the antiviral compound remdesivir result in a 15.6% TSWV clearance rate.

In another aspect of the present invention, there is provided a recombinant tospovirus vector system, comprising: a) a polynucleotide sequence of a viral vector of the tospovirus, and b) a heterologous polynucleotide sequence encoding at least one sequence-specific nucleic acid modifying enzyme being operably linked to a viral transcription regulatory cis-element, wherein the tospovirus vector is infectious and capable of directing transient expression of said sequence-specific nucleic acid modifying enzyme in a plant cell.

In some embodiments, the sequence-specific nucleic acid modifying enzyme is a nuclease, its mutated forms or derivatives capable of modifying genomic DNA sequence, i.e., CRISPR/Cas nucleases, TALEN nucleases, zinc finger nucleases, meganuclease. In certain embodiments, the nuclease is the *Streptococcus pyogenes* CRISPR/SpCas9. In certain embodiments, the nuclease is the *Lachnospiraceae bacterium* CRISPR/LbCas12a (LbCpf1).

In some embodiments, the sequence-specific nucleic acid modifying enzyme is an adenine base editor. In some embodiments, the sequence-specific nucleic acid modifying enzyme is a cytidine base editor.

In some embodiments, the recombinant tospovirus vector system is the TSWV vector comprising the S, M, and L genome segments having at least 70%, at least 80%, at least 90%, at least 95%, at least 99% or 100% sequence homologies to the nucleic acid sequences as set forth in SEQ ID NO: 1, SEQ ID NO: 2, and SEQ ID NO: 3, respectively.

In some embodiments, the TSWV S segment comprises at least one heterologous polynucleotide sequence encoding a sequence-specific nuclease component being operably linked to viral mRNA transcription regulatory cis-elements; preferably, the nuclease component is a chimeric CRISPR guide RNA polynucleotide molecule.

In some embodiments, the TSWV non-structural protein gene in the S segment (NSs) is replaced by heterologous polynucleotide sequences encoding a fusion of the sequence-specific nuclease component to heterologous sequences encoding, for example, a fluorescent reporter. In some embodiments, the sequence-specific nuclease component is a guide RNA. In certain embodiments, the heterologous polynucleotide sequence inserted into said S genome encodes one guide RNA. In certain embodiments, the heterologous polynucleotide sequence encodes two or more guide RNAs.

In some embodiments, the TSWV M segment comprises at least one heterologous polynucleotide sequence operably linked to viral transcription regulatory elements and encoding a Cas polypeptide, optionally selected from the group consisting of *Streptococcus pyogenes* SpCas9, *Lachnospiraceae bacterium* LbCas12a (LbCpf1), or an adenine base editor or cytosine base editor comprising a Cas9 variant fused to a base deaminase.

In some preferred embodiments, the coding sequences of LbCas12a, SpCas9, adenine base editor, and cytosine base editor polypeptides are plant codon-optimized sequences; preferably, as set forth in SEQ ID NO: 10, 11, 84, 85, respectively.

In some embodiments, the viral glycoprotein precursor (GP) gene sequence in the TSWV M segment is replaced by a heterologous polynucleotide sequence encoding a sequence-specific nuclease component.

In some embodiments, the viral RNA-dependent RNA polymerase (RdRp) gene sequence in the TSWV L segment is plant codon-optimized as set forth in SEQ ID NO: 5.

In some embodiments, the tospovirus vectors are viral nucleic acid constructs capable of initiating infection upon introduction into a plant; preferably, the viral constructs are *Agrobacterium* binary plasmids containing transcriptional regulatory cis-elements, including a promoter and a terminator, and the constructs are delivered into plant tissue through agroinfiltration.

In some embodiments, the viral nucleic acid constructs being introduced into the plant tissues further comprise helper constructs for the expression of one or more VSRs. In certain embodiments, the VSR is selected from the group consisting of TBSV p19, TEV Pl/Hc-Pro, BSMV γb, or TSWV NSs.

In some embodiments, the binary constructs produce the positive- or negative-sense viral RNA transcripts corresponding to the chimeric sequence of the recombinant TSWV S, M, and L segments. In certain preferred embodiments, the binary constructs produce positive-sense RNA transcripts.

In some embodiments, the polynucleotide constructs comprising the TSWV L, M, and S segments are delivered into a plant cell in relative molar ratios of 1-2 (L): 2-10 (M): 1-2 (S), preferably in a relative ration of 1: 10: 2. In certain embodiments, the ratios of the nucleic acid constructs are the ratios of the relative concentration (OD₆₀₀) of *Agrobacterium* strains carrying different binary plasmids.

In some embodiments, the method for infecting a plant with a TSWV vector is to use recombinant virus particles through mechanical rubbing, pressurized spraying, plant grafting.

In some embodiments, the tospovirus vector is an infectious TSWV vector devoid of a functional NSs protein. In some embodiments, the TSWV vector is non-insect-transmissible tospovirus vector devoid of a functional GP protein.

In another aspect of the present invention, there is also provided a bacterial strain or recombinant viral particle comprising the recombinant tospovirus vector system.

In another aspect, the disclosure also provides a kit of the recombinant tospovirus vector systems, wherein the reagents containing the viral vectors may be provided individually or in combination, including: a) a composition comprising nucleic acid constructs comprising the polynucleotide sequence of the TSWV S, M and L segments and at least one heterologous polynucleotide sequence encoding a sequence-specific nuclease; (b) a helper expression vector encoding one or VSRs selected from the group consisting of TBSV p19, TEV Pl/Hc-Pro, BSMV γb, or TSWV NSs. The kit further comprises a user manual or instructions.

In another aspect, the present disclosure also relates to the application of recombinant tospovirus vector systems, strains or recombinant viruses, kits, or methods as described above to the editing of plant genomes.

In another aspect, the present disclosure also provides genetically modified plants and their offspring produced by using the recombinant tospovirus vector system described above or produced according to the method described above.

### BRIEF DESCRIPTION OF THE DRAWINGS

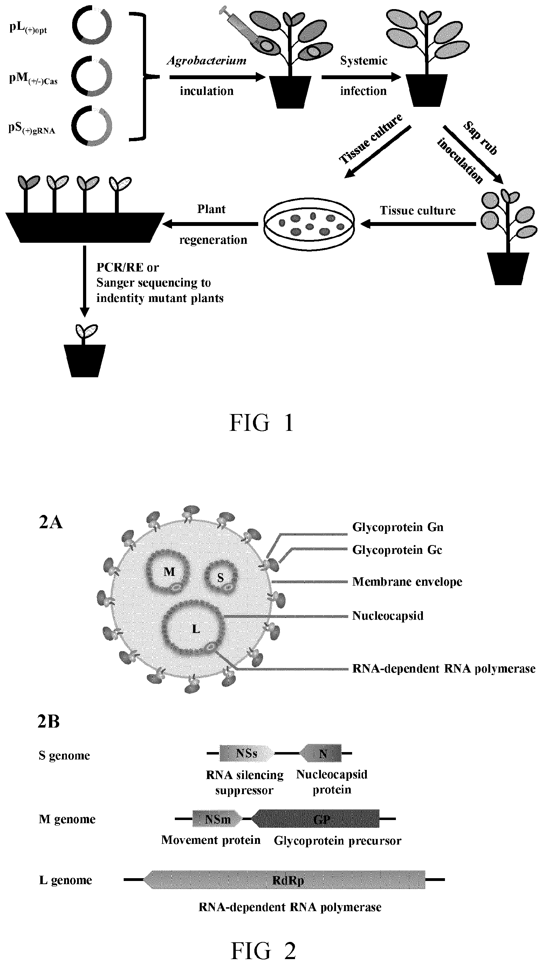

**FIG. 1** illustrates the plant genome editing procedure using the TSWV vector-mediated delivery of CRISPR/Cas nuclease. *N*. *benthamiana* plants were agroinoculated with the TSWV vectors carrying a CRISPR/Cas nuclease to initiate systemic infection of the recombinant virus. Leaf saps containing the recombinant virus particles are used as inoculum to infect additional host plants. The virus vector expresses the nuclease in the infected cells to induce gene editing, and the infected plant tissues were used as explants to regenerate plants in vitro by tissue culture. Targeted sequence modifications in the regenerated plants and their offspring are identified by endonuclease protection test or Sanger sequencing.
FIG. **2** illustrates the morphology and genomic structure of TSWV. FIG. 2A shows the quasi-spherical, enveloped particle with viral Gn and Gc glycoprotein spikes embedded in the host-derived lipidmembrane. Inside the viral particle are three single-stranded, negative-sense RNA genomes (L, M, and S) encapsidated by the nucleocapsid protein (N) and RNA-dependent RNA polymerase (RdRp) . FIG. 2B shows the TSWV genome structure. The L genome segment encodes RdRp in the negative-sense RNA, the ambisense M segment encodes the movement protein NSm and GP, and the ambisense S segment encodes the N and NSs proteins.

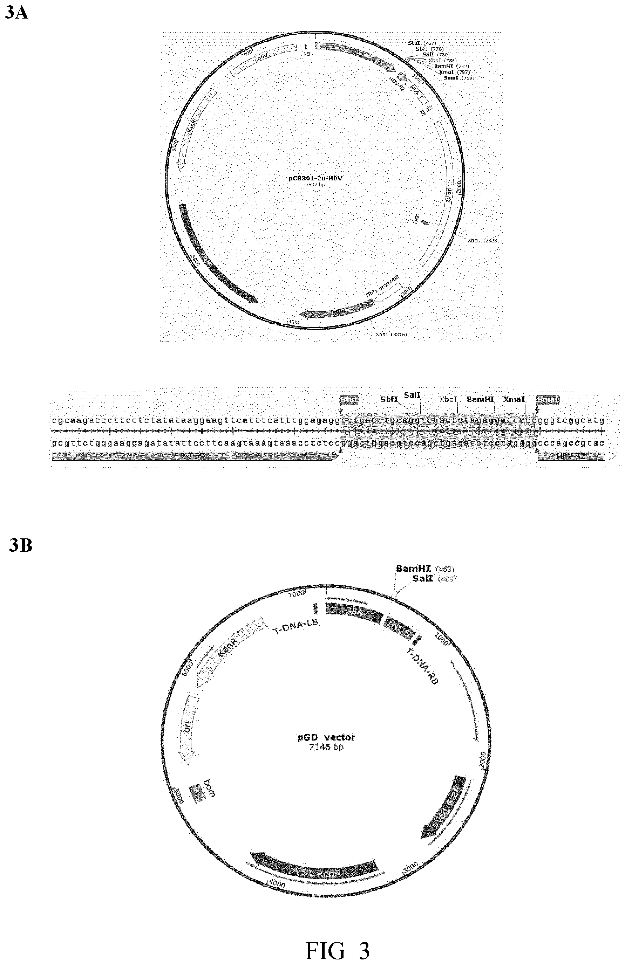

FIG. **3** shows the maps of the empty binary vectors. FIG. **3**A shows the map of pCB301-2µ-HDV plasmid used for the construction of TSWV cDNA clones. The plasmid above contains a doubled 35S promoter (2×35S) (SEQ ID NO: 9), hepatitis D virus ribozyme (HDV-Rz), NOS terminator, T-DNA left arm (LB) and right arm (RB), yeast replication origin (2µ ori), yeast TRP1 autotrophic marker, bacterial replication origin (oriV), kanamycin resistance gene (KanR), and the replicase gene trfA. Viral cDNA sequences are inserted into the StuI and SmaI sites to produce viral RNAs with precise ends. FIG. 3B illustrates the map of the pGD binary vector used to construct transient expression vectors.

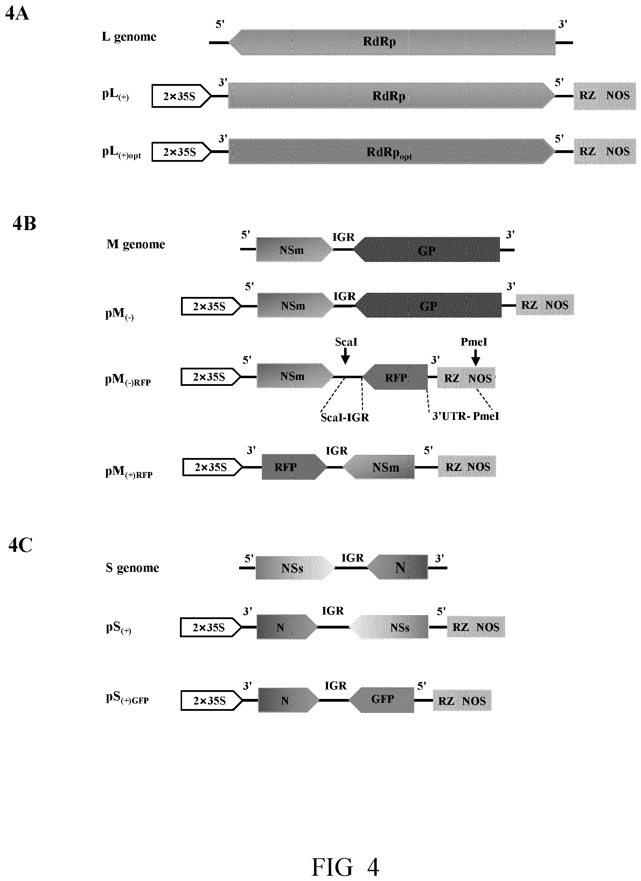

FIG. **4** is a schematic illustration showing the strategy for engineering the TSWV vector to express two reporter proteins simultaneously. FIG. 4A shows the pL₍₊₎ construct encoding the L antigenomic (positive sense) RNA, and the pL₍₊₎ₒₚₜ in which the RdRp coding sequence (SEQ ID NO: 4) is replaced by a plant codon-optimized sequence (SEQ ID NO: 5) . FIG. 4B shows the pM₍₋₎ construct encoding theM genomic (negative sense) RNA, the pM_{(-)RFP} and pM_{(+)RFP} constructs encoding the M genomic and antigenomic RNA, respectively, and containing an *RFP* gene substituting for the GP coding sequence (SEQ ID NO: 6) . FIG. 4C shows the pS₍₊₎ construct encoding the S antigenomic RNA and the pS_{(+)GFP} containing a *GFP* gene substituting for the NSs coding sequence. 2×35S, double 35S promoter; Rz: hepatitis D virus ribozyme; NOS: NOS terminator; 5' and 3', 5' and 3' untranslated region (UTR); IGR, intergenic region.

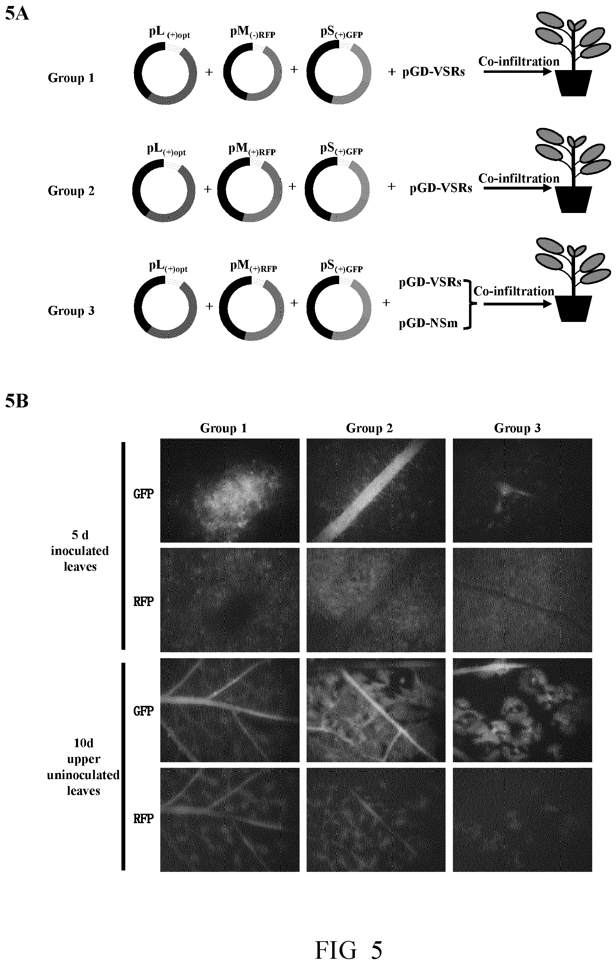

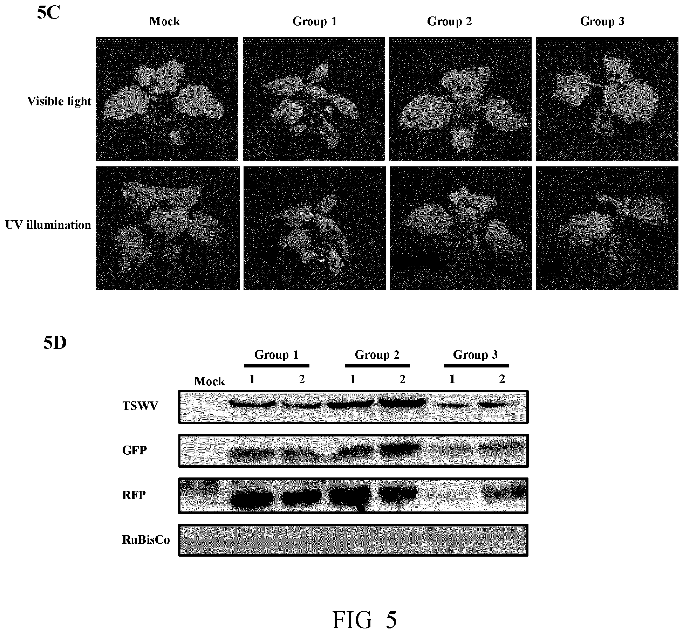

FIG. **5** shows the simultaneous expression of two fluorescent reporter proteins with the TSWV vector. FIG. 5A illustrates the combinations of *Agrobacterium* inoculation mixtures containing different viral constructs. FIG. 5B shows fluorescent images of GFP and RFP in infiltrated leaves and upper uninoculated leaves of *N*. *benthamiana* plants. FIG. **5**C shows symptoms and fluorescence produced by recombinant TSWV vectors in agroinoculated *N*. *benthamiana* plants. Mock: mock-inoculated plant. FIG. 5D shows protein gel blots of the TSWVN, GFP, and RFP protein expressed in virus-infected systemic leaves.

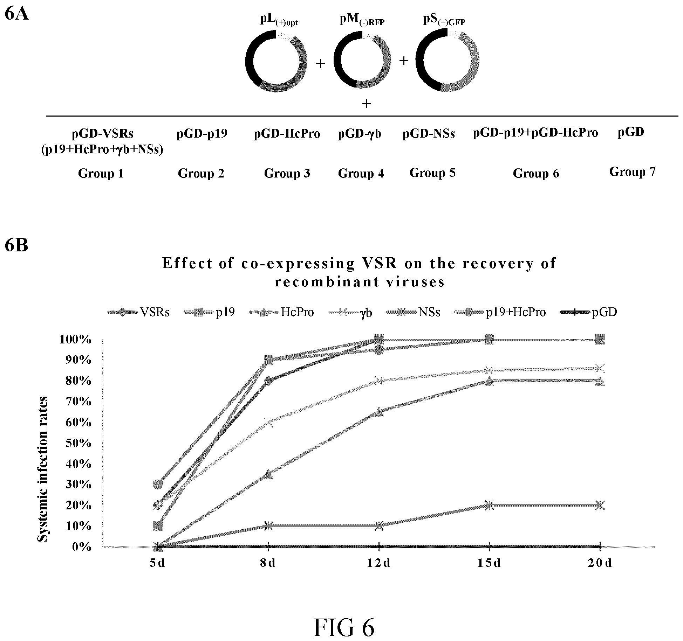

FIG. **6** shows the effect of expressing different viral suppressors of RNA silencing (VSRs) on the recovery of recombinant TSWV vectors. FIG. 6A illustrates the combination of *Agrobacterium* mixtures containing one or more VSRs and the TSWV vector constructs. FIG. 6B is a graph showing the TSWV vector's recovery efficiency upon co-delivering with one or more VSRs.

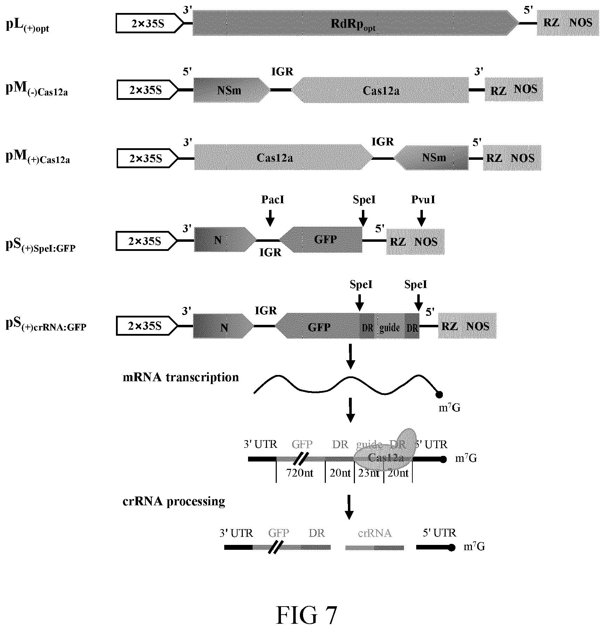

FIG. **7** is a scheme illustrating the strategy for engineering the TSWV-Cas12a-crRNAvector. pL₍₊₎ₒₚₜ is an L antigenomic strand cDNA clone containing codon-optimized RdRp; pM_{(-)Cas12a} and pM_{(+)Cas12a} are the constructs containing the M genomic and antigenomic cDNA, respectively, in which the *GP* gene is replaced by the sequence encoding *Cas12a*; pS_{(+)crRNA:GFP} is a construct containing the S antigenomic cDNA with *NSs* gene being replaced by a fusion sequence comprising the crRNA sequence and the GFP coding sequence. 2×35S, double 35S promoter; Rz, hepatitis D virus ribozyme; NOS, NOS terminator; 5' and 3', 5' and 3' untranslated region (UTR); IGR, intergenic region. m7G: mRNA cap; DR, crRNA direct repeat region (direct repeat); guide, crRNA guide sequence.

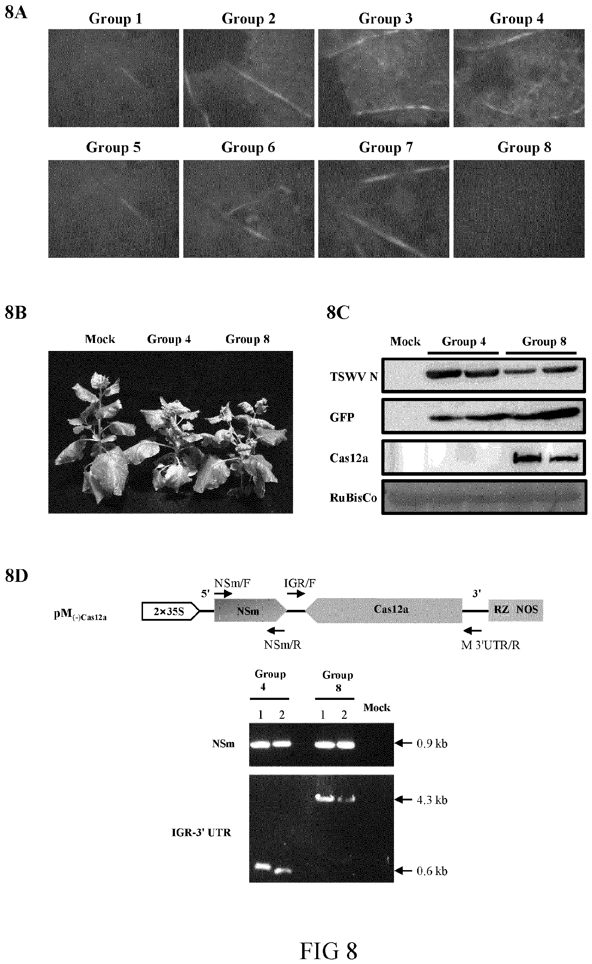

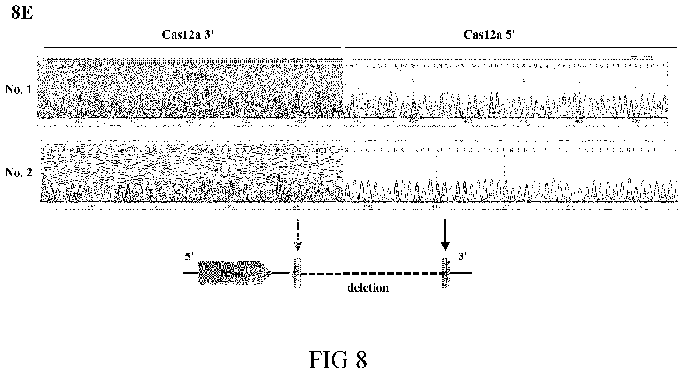

FIG. **8** shows the recovery of the TSWV-Cas12a-crRNA vector from cloned cDNAs and the stability of the Cas12a insert. FIG. 8A shows GFP fluorescence produced by the recombinant TSWV vectors in *N*. *benthamiana* leaves agroinfiltrated with different combinations of agroinfiltration mixtures. FIG. 8B shows the symptoms of the TSWV vectors. Mock: mock-inoculated control. FIG. 8C displays the expression of TSWV N protein, GFP, and Cas12a in systemically infected tissues. FIG. 8D illustrates the RT-PCR assays for detecting the integrity of *Cas12a* gene in the M segment of the recombinant TSWV vectors. The top panel displays the positions of primer-binding sites for detecting the *NSm* and *Cas12a* genes, and the bottom panel shows the gel electrophoresis of PCRproducts. FIG. 8E shows Sanger sequencing chromatographs di deletion of the majority portion of *Cas12a* fragment in the M genome.

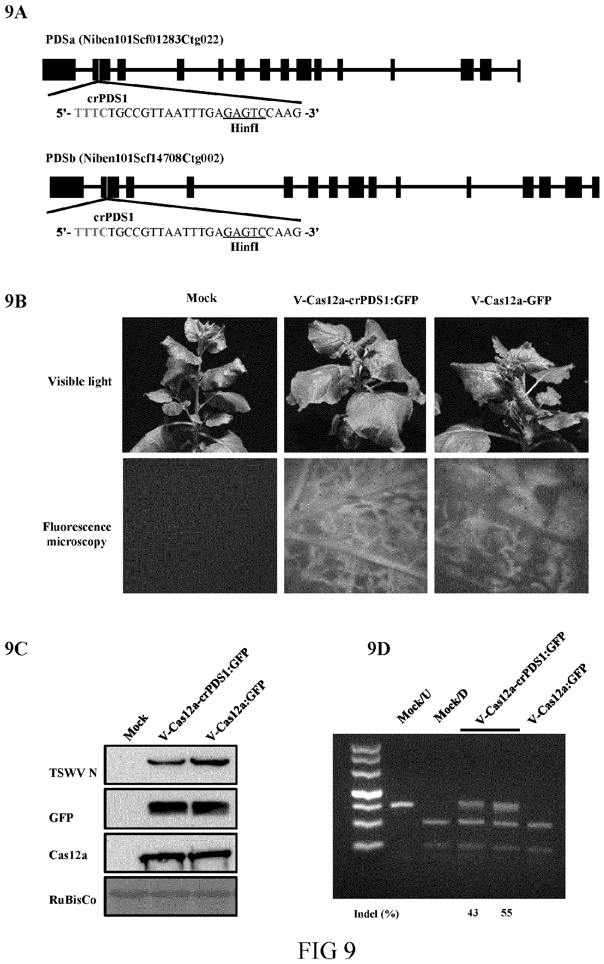

FIG. **9** shows TSWV-Cas12a-crRNA-indcued targeted gene editing in infected *N*. *benthamiana* plants. FIG. 9A are diagrams showing the *PDS* gene structures and the crPDS1 target site sequences in *N*. *benthamiana.* The selected crPDS1 target site is conserved in both *PDS* (a and b) homoeologs of the allotetraploid *N*. *benthamiana.* Underlined letters denote the restriction endonuclease recognition sequence contained in the target sequence, and bold letters represent the PAM sequence. FIG. 9B shows symptoms and GFP fluorescence in upper leaf tissues of systemically infected *N*. *benthamiana* plants. FIG. 9C depicts the expression of the TSWV N, GFP, and Cas12a proteins. FIG. 9D shows a PCR/RE assay to detect the indel frequency at the crPDS1 target site. Mock/U and Mock/D denote mock-infected plants without or with restriction digestion. Indel (%) indicates the percentage of PCR products resistant to HinfI restriction digestion.

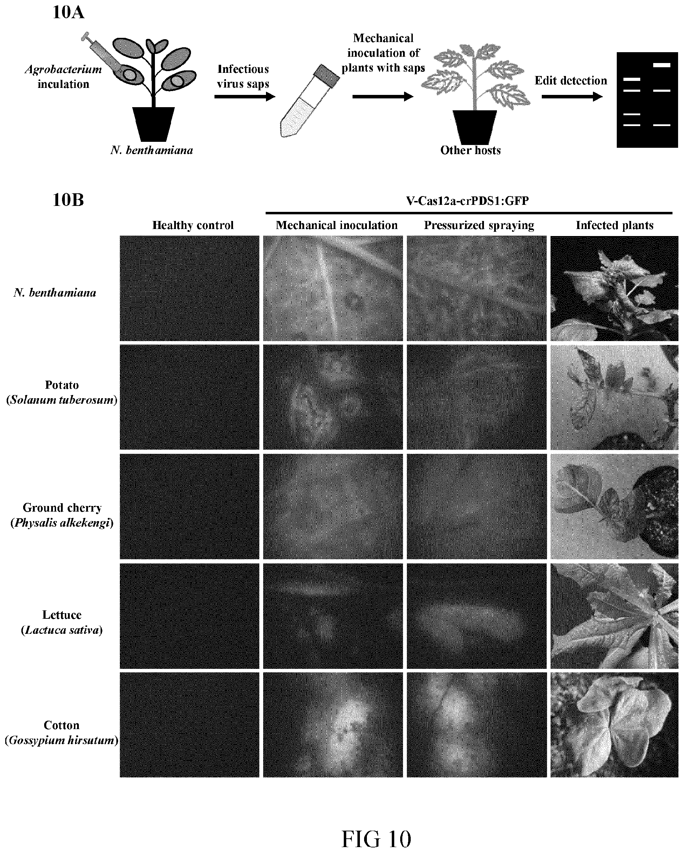

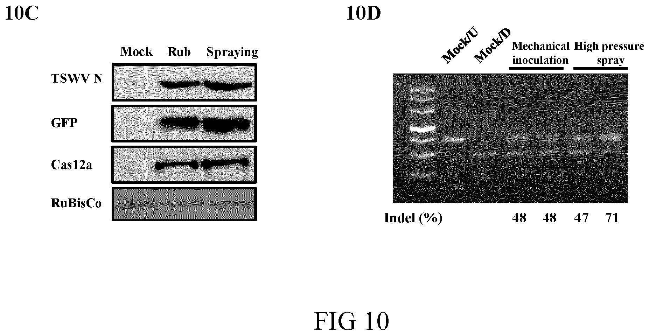

FIG. **10** shows targeted gene editing in multiple plant hosts infected with TSWV-Cas12a-crRNA leaf saps extracted from infected *N*. *benthamiana.* FIG. 10A depicts the scheme of the experimental procedure. FIG. 10B depicts GFP fluorescence and symptoms in sap-inoculated *N*. *benthamiana,* potato, ground cherry, lettuce, and cotton plants. **10**C depicts the expression of the TSWV N, GFP, and Cas12a proteins in systemic leaves of the infected *N*. *benthamiana.* FIG. 10D depicts PCR/RE assay for detecting the indel frequency of the sap-inoculated *N*. *benthamiana* plants.

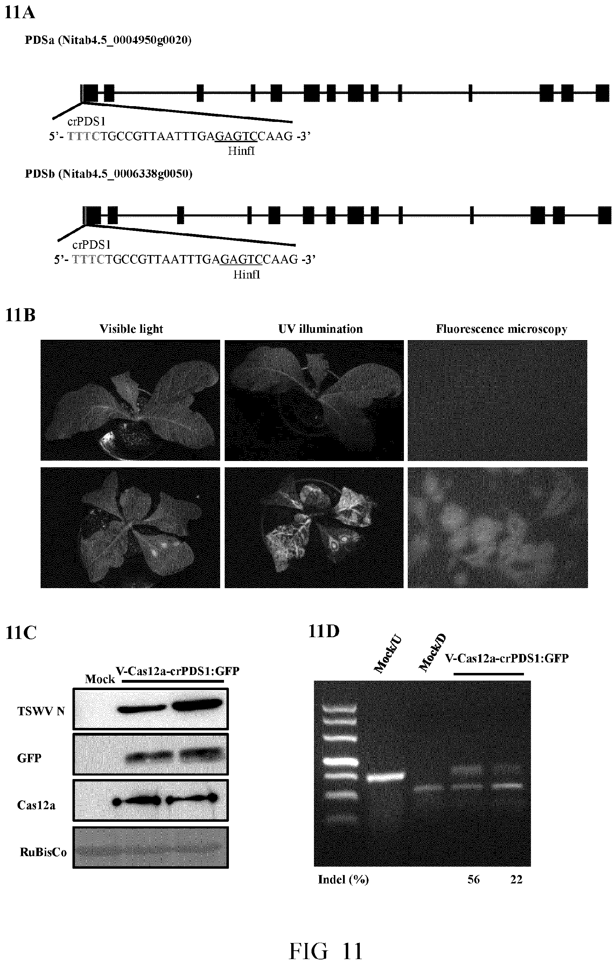

FIG. **11** shows TSWV-Cas12a-crRNA-induced targeted gene editing in infected common tobacco (*N. tabacum*). FIG. 11A are diagrams showing the *PDS* gene structures and the crPDS1 target site sequences in tobacco. The selected crPDS1 is conserved in tobacco *PDSa* and *PDSb.* Underlined letters denote the restriction endonuclease recognition sequence contained in the target sequence, and bold letters represent the PAM sequence. FIG. 11B shows symptoms and GFP fluorescence in upper leaf tissues of systemically infected tobacco plants. Upper panels are mock-inoculated control plants, and lower panels show plants inoculated with TSWV-Cas12a-crRNA. FIG. 11C shows a PCR/RE assay to detect the indel frequency at the crPDS1 target site.

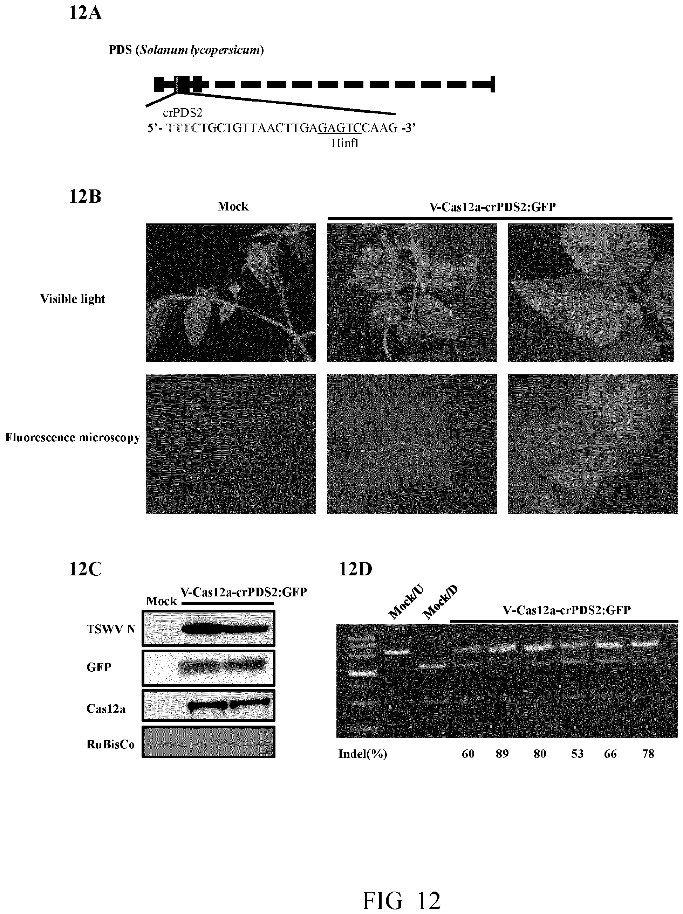

FIG. **12** shows TSWV-Cas12a-crRNA-induced gene editing in infected tomato plants. FIG.12A shows a schematic diagram of the tomato *PDS* gene structure and the crPDS2 target site sequence. FIG. 12B shows symptoms and GFP fluorescence in the upper leaves of infected tomato plants. FIG. 12C shows the expression of the TSWV N protein, GFP, and Cas12a proteins. FIG. 12D shows the indel frequency at the crPDS2 target site.

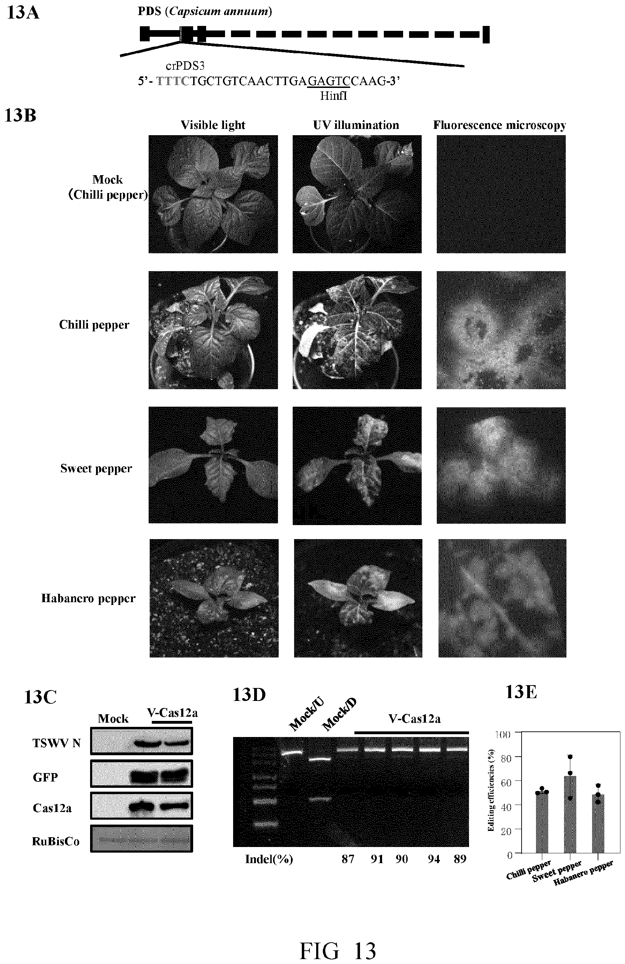

FIG. **13** shows TSWV-Cas12a-crRNA-induced gene editing in infected peppers. FIG. 13A is a schematic diagram showing the tomato *PDS* gene structure and the crPDS3 target site sequence, which is conserved in chili pepper (*C*. *frutescens*), sweet pepper (*C*. *annuum*) and habanero pepper (*C*. *chinense*). FIG. 13B depicts symptoms and GFP fluorescence in infected peppers. Top panels show a healthy control plant, and bottom panels show a virus-infected plant. FIG. 13C illustrates the expression of the TSWV N protein, GFP, and Cas12a proteins. FIG. 13D showing a PCR/RE assay for detecting the indel frequency at the crPDS2 target site in infected chili peppers (*C*. *frutescens*)*.* FIG. 13E is a graph showing the percentage of reads with targeted mutations in virus-infected tissues of the chili pepper, sweet pepper, and habanero pepper.

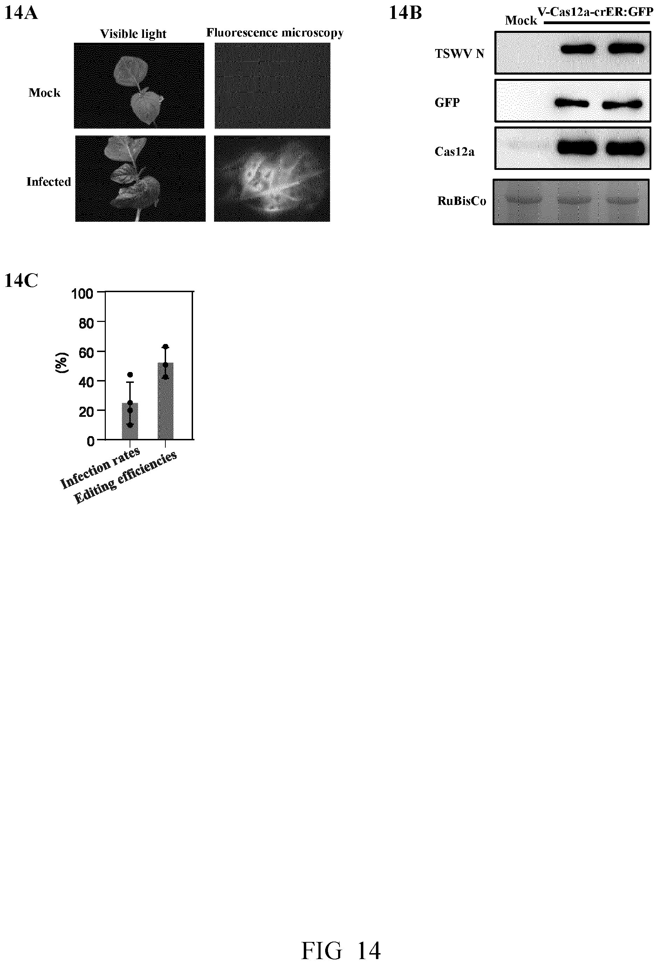

FIG. **14** shows TSWV-Cas12a-crRNA-induced gene editing in infected ground cherry. FIG. 14A shows symptoms and GFP fluorescence in infected ground cherry. Mock: moc-inoculation control plant. FIG. 14B shows the expression of the viral N protein, GFP, and Cas12a proteins. FIG. 14C shows the percentage of reads with targeted mutations in virus-infected tissues.

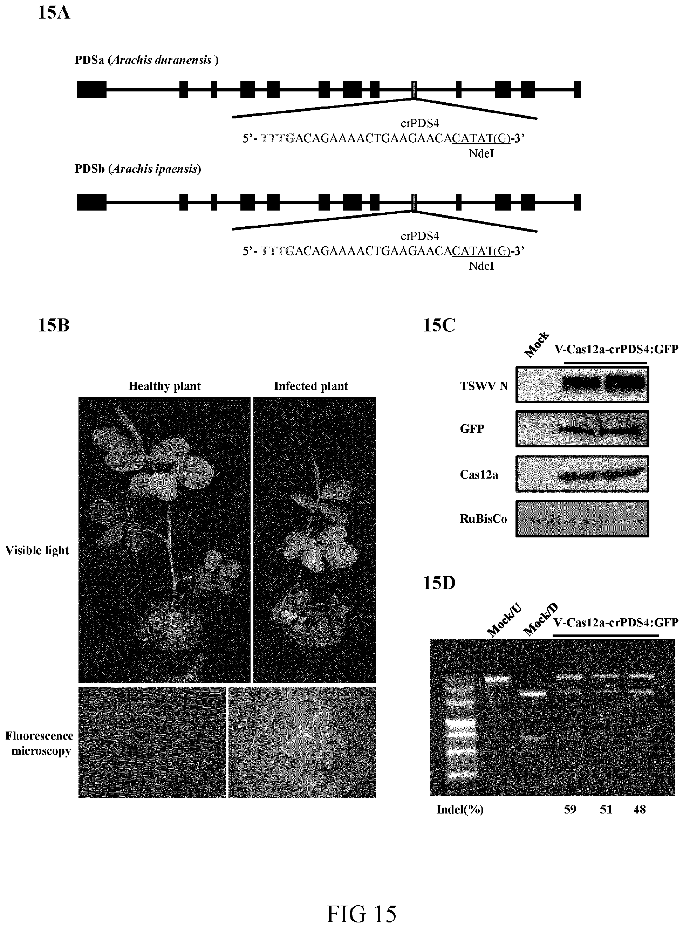

FIG. **15** shows TSWV-Cas12a-crRNA-induced gene editing in infected peanut plants. FIG. 15A is a schematic diagram showing the peanut *PDS* gene structure and the crPDS4 target site sequence that is conserved in both homoeologs in the allotetraploid genome of peanuts. FIG. 15B shows symptoms and GFP fluorescence in infected peanuts . The left picture is a mock-inoculated control plant, and the right picture is the virus-infected plant. FIG. 15C shows the expression of the viral N protein, GFP, and Cas12a proteins. FIG. 15D depicts a PCR/RE assay for detecting indel frequency in infected peanut tissues.

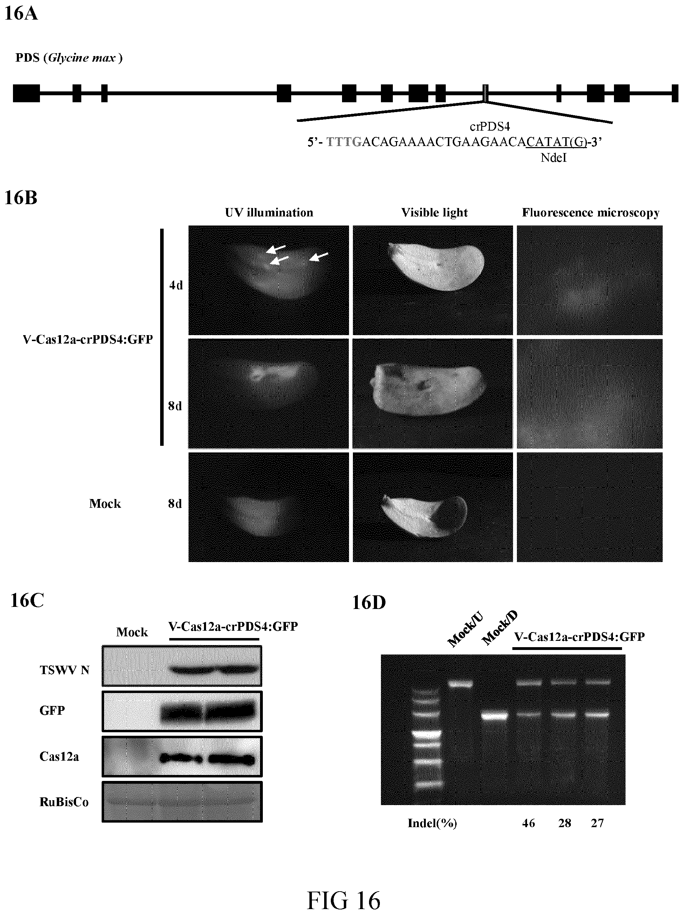

FIG. **16** shows TSWV-Cas12a-crRNA-induced gene editing in infected soybean plants. FIG. 16A is a schematic diagram showing the soybean *PDS* gene structure and the crPDS4 target site sequence. FIG. 16B shows symptoms and GFP fluorescence in infected peanuts . Images of a mock-inoculated plant are shown in the images at the bottom panels. FIG. 16C shows the expression of the viral N protein, GFP, and Cas12a proteins. FIG. 16D shows a PCR/RE assay for detecting indel frequency in infected soybean tissues .

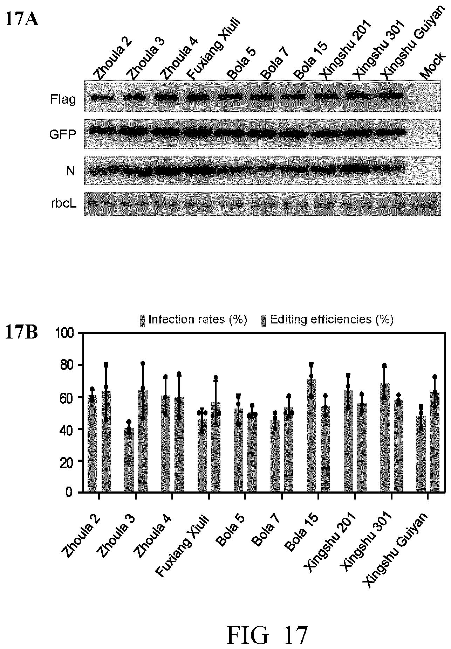

FIG. **17** shows TSWV-Cas12a-crRNA-induced gene editing in different sweet pepper varieties. FIG. 17A depicts the expression of the TSWV N, GFP, and Cas12a proteins in upper young leaf tissues of systemically infected pepper plants . FIG. 17B depicts systemic infection rates and indel frequencies in infected sweet pepper varieties.

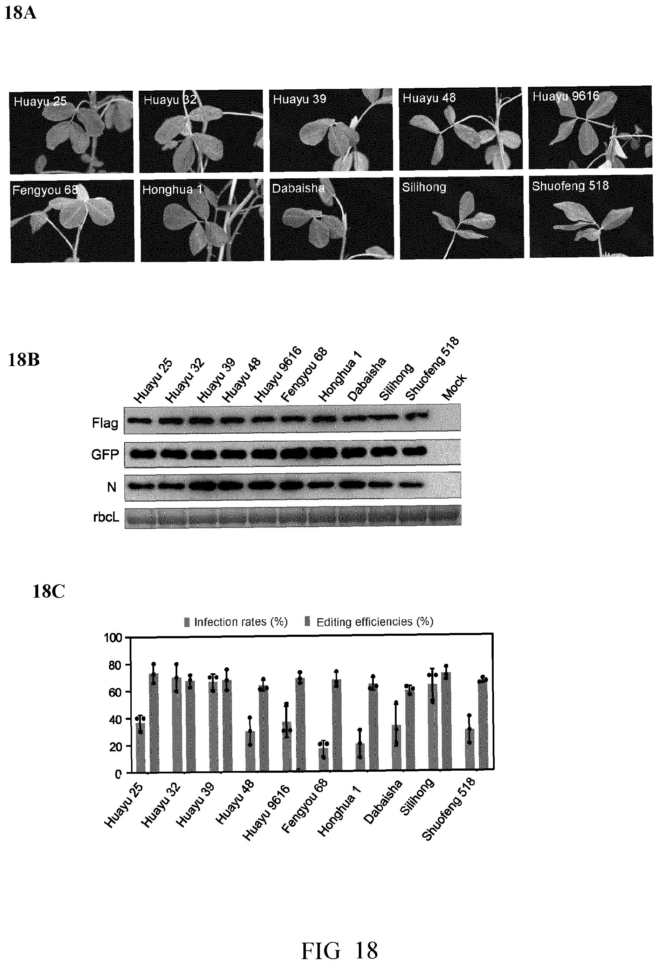

FIG. **18** shows TSWV-Cas12a-crRNA-induced gene editing in different peanut varieties. FIG. 18A FIG depicts symptoms of infected peanut plants. **18**B depicts the expression of the TSWV N, GFP, and Cas12a proteins in upper young leaf tissues of systemically infected peanut plants. FIG. 18C depicts systemic infection rates and indel frequencies in infected peanut varieties.

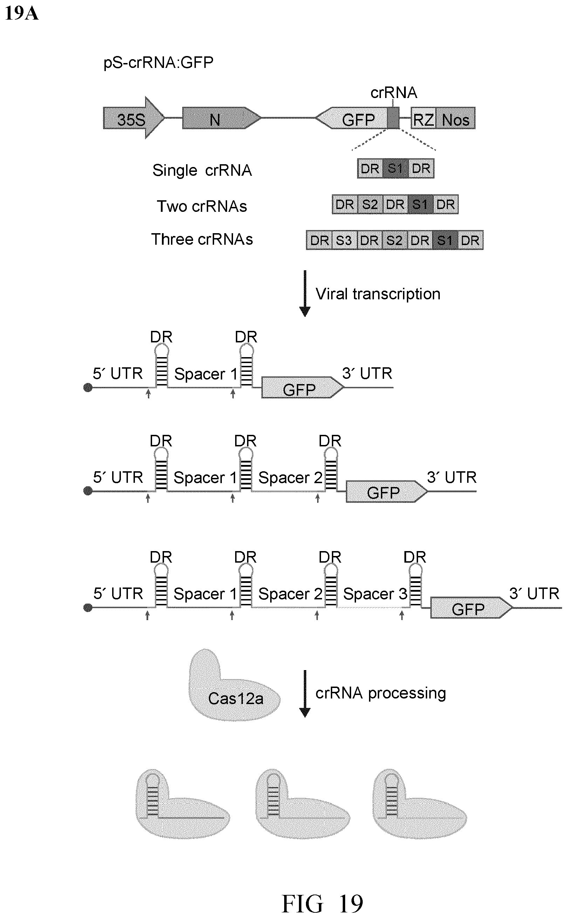

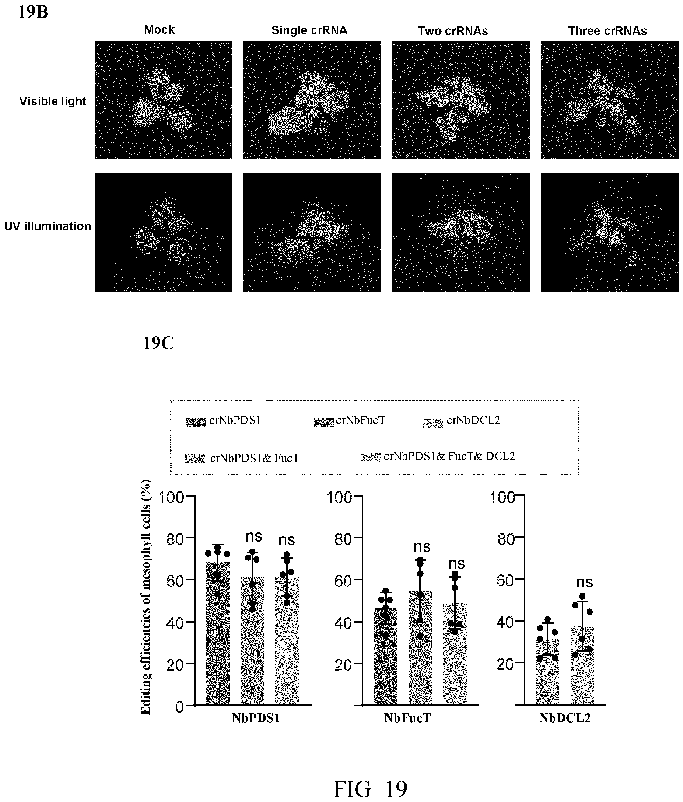

FIG. **19** shows multiplexed gene editing with pS-crRNA:GFP expressing one or multiple gRNAs . FIG. 19A are schematic diagrams illustrating the strategy for expressing one or multiple crRNAs . S1, S2, and S3 are Spacer 1, Spacer 2, and Spacer 3 sequences; DR is direct repeat; Red arrows indicate Cas12a cleavage sites that mediate the release of one or more mature crRNAs. FIG. 19B shows symptoms of TSWV-Cas12a-crRNAvectors containing one, two, or three crRNAs in infected *N*. *benthamiana* plants. FIG. 19C shows indel frequencies at each target site induced by the TSWV vectors with single or multiple gRNAs . ns, no statistically significant differences (n = 6 biological replicates, two-tailed Student's t-test).

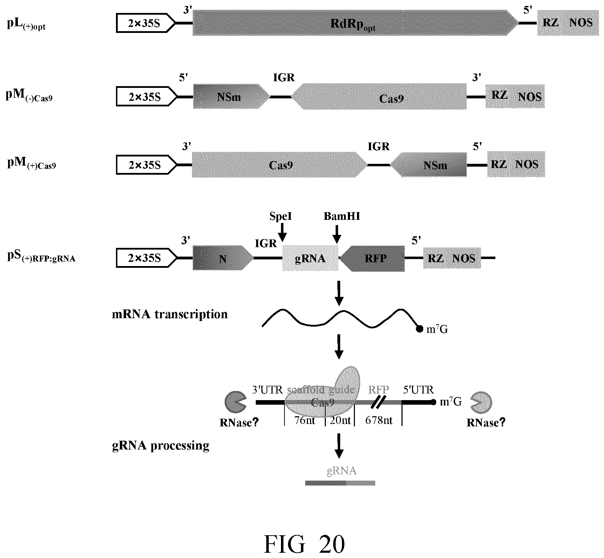

FIG. **20** is a schematic illustration showing the engineering of the TSWV-Cas9-gRNA vector. pL₍₊₎ₒₚₜ is an L antigenomic strand cDNA clone containing codon-optimized RdRp; pM_{(-)cas9} and pM_{(+)Cas9} are the constructs containing the M genomic and antigenomic cDNA, respectively, in which the *GP* gene is replaced by the sequence encoding *Cas9;* pS_{(+)RFP:gRNA} is a construct containing the S antigenomic cDNA with *NSs* gene being replaced by a fusion sequence comprising the RFP coding sequence and a crRNA sequence . Upon viral replication and transcription, the S genome segment's RFP and gRNA fusion sequence is transcribed into mRNA. The Cas9 protein expressed from the M segment binds the gRNA sequence, and the plant endogenous RNA enzymes remove the extra sequences at both ends. 2×35S, double 35S promoter; Rz, hepatitis D virus ribozyme; NOS, NOS terminator; 5' and 3' , 5' and 3' untranslated region (UTR) ; IGR, intergenic region. m7G: mRNA cap; scaffold, gRNA scaffold sequence; guide: gRNA guide sequence.

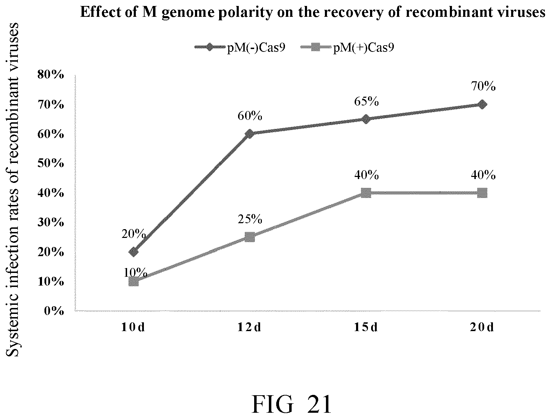

FIG. **21** is a graph comparing the efficiencies of recombinant TSWV-CRISPR/Cas9 recovery using the pM_{(-)Cas9} and pM_{(+)cas9} constructs.

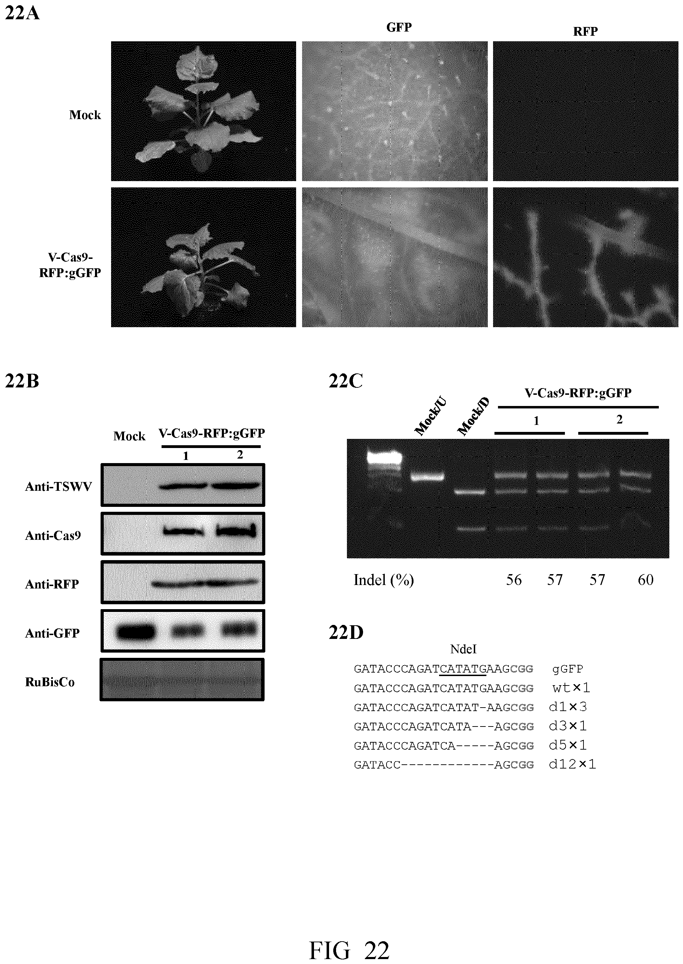

FIG. **22** shows TSWV-Cas9-gRNA-induced gene editing at the gGFP target loci in the *GFP*-transgenic *N*. *benthamiana* plants. FIG. 22A depicts symptoms and GFP fluorescence in infected *N*. *benthamiana* 16C plants. FIG. 22B depicts the expression of TSWV N, RFP, GFP, and Cas9 proteins. FIG. 22C shows indel frequency at the gGFP target loci determined by PCR/RE assay. FIG. **22**D shows Sanger sequencing results of the targeted mutations . wt, wild type; d#, # bases deletion; ×#, # clones.

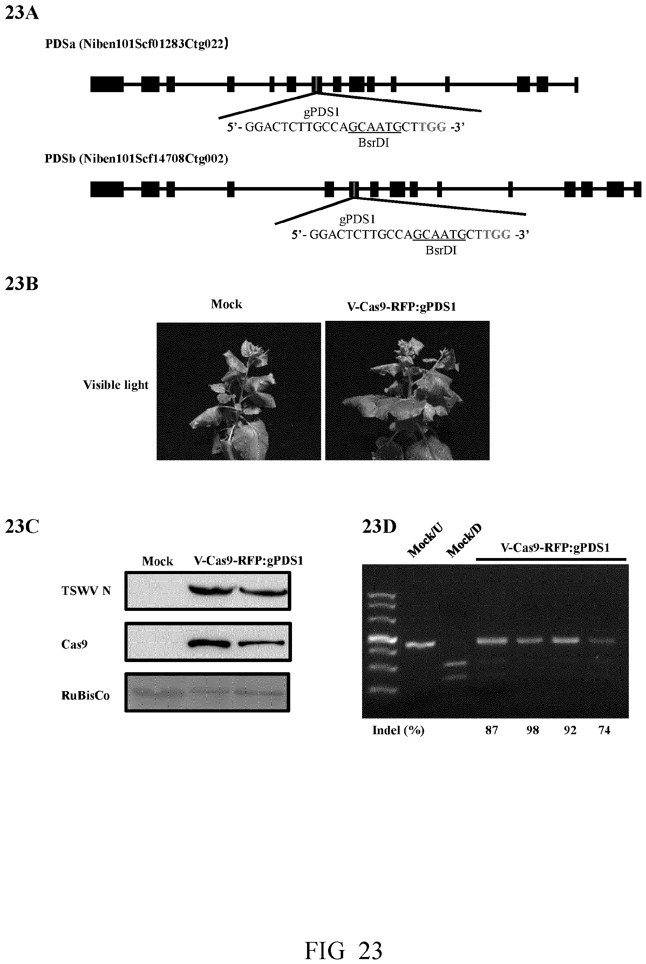

FIG. **23** shows TSWV-Cas9-gRNA-indcued targeted gene editing in infected *N*. *benthamiana* plants. FIG. 23A are diagrams showing the *PDS* gene structures and the gPDS1 target site sequences in *N*. *benthamiana.* The selected gPDS1 target site is conserved in both *PDS* (a and b) homoeologs of *N*. *benthamiana.* Underlined letters denote the restriction endonuclease recognition sequence contained in the target sequence, and bold letters represent the PAM sequence. FIG. 23B shows symptoms in infected *N. benthamiana* plants. FIG. 23C depicts the expression of the TSWV N, GFP, and Cas12a proteins. FIG. 23D shows a PCR/RE assay to detect the indel frequency at the gPDS1 target site. Mock/U and Mock/D denote mock-infected plants without or with restriction digestion.

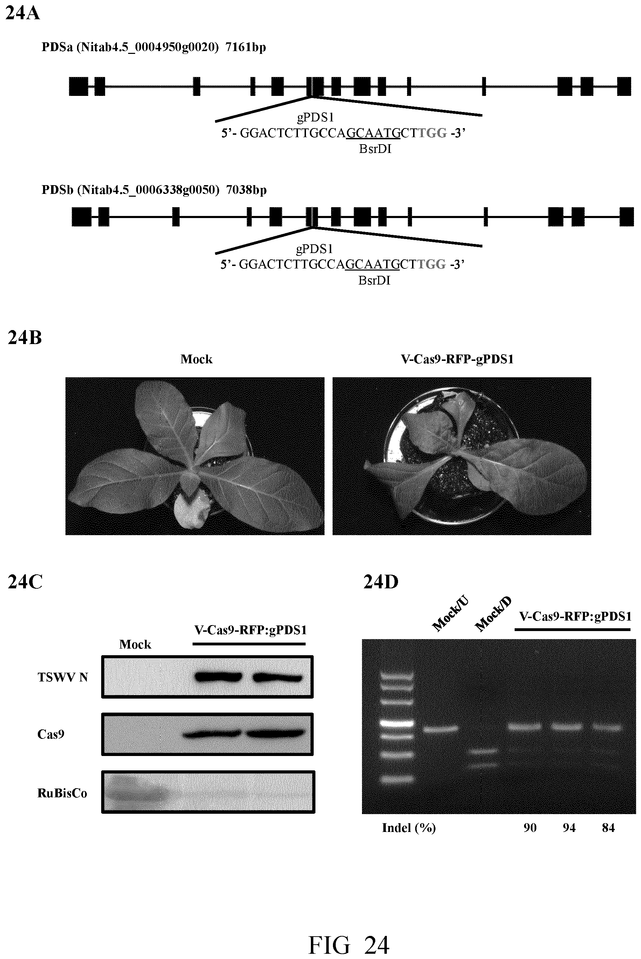

FIG. **24** shows TSWV-Cas9-gRNA-indcued targeted gene editing in sap-inoculated *N*. *tabacum* plants. FIG. 24A are diagrams showing the *PDS* gene structures and the gPDS1 target site sequences in tobacco. The selected gPDS1 target site is conserved in both *PDS* (a and b) homoeologs of tobacco. Underlined letters denote the restriction endonuclease recognition sequence contained in the target sequence, and bold letters represent the PAM sequence. FIG. 23B shows symptoms in infected tobacco plants. FIG. 24C depicts the expression of the TSWV N, GFP, and Cas12a proteins. FIG. 24D shows a PCR/RE assay to detect the indel frequency at the gPDS1 target site.

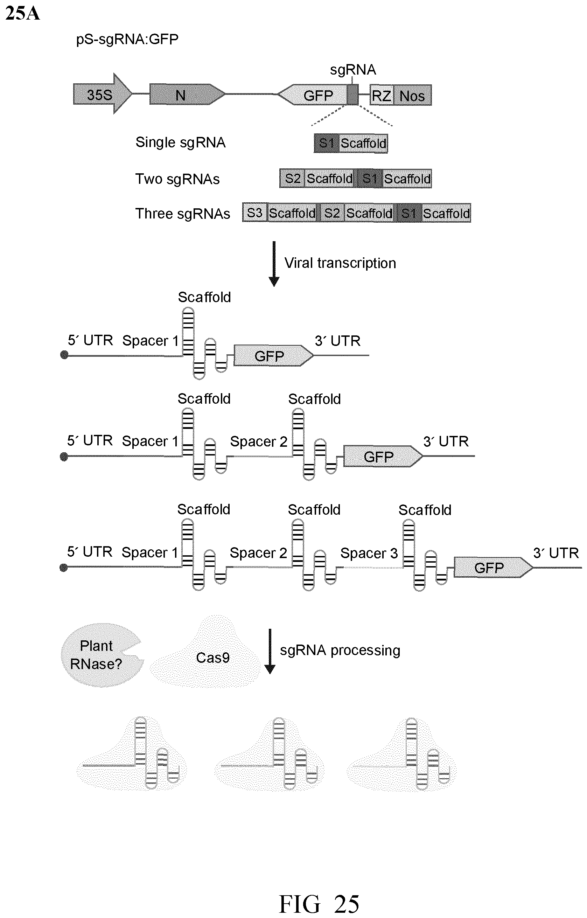

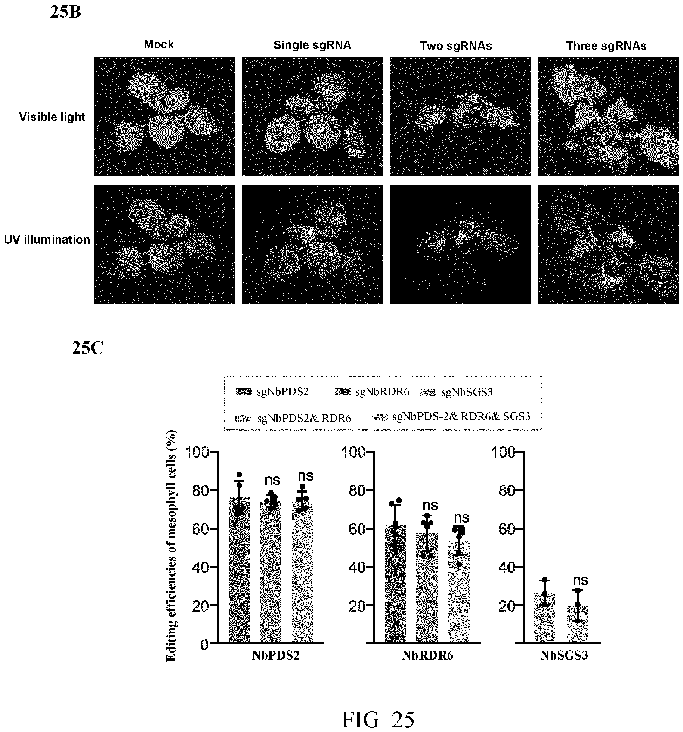

FIG. **25** shows multiplexed gene editing with pS-sgRNA:GFP expressing one or multiple gRNAs . FIG. 25A are schematic diagrams illustrating the strategy for expressing one or multiple gRNAs . S1, S2, and S3 are Spacer 1, Spacer 2, and Spacer 3 sequences; Scaffold, sgRNA scaffold sequence. Processing of gRNAs embedded in viral mRNA is dependent on Cas9 protein binding and plant endogenous RNase to remove additional sequences at both ends. FIG. 25B depicts symptoms of TSWV-Cas9-gRNA vectors containing one, two, or three gRNAs in infected *N*. *benthamiana* plants. FIG. 25C shows that the TSWV vectors with single or multiple gRNAs induce indel frequencies at each target site. ns, no statistically significant differences.

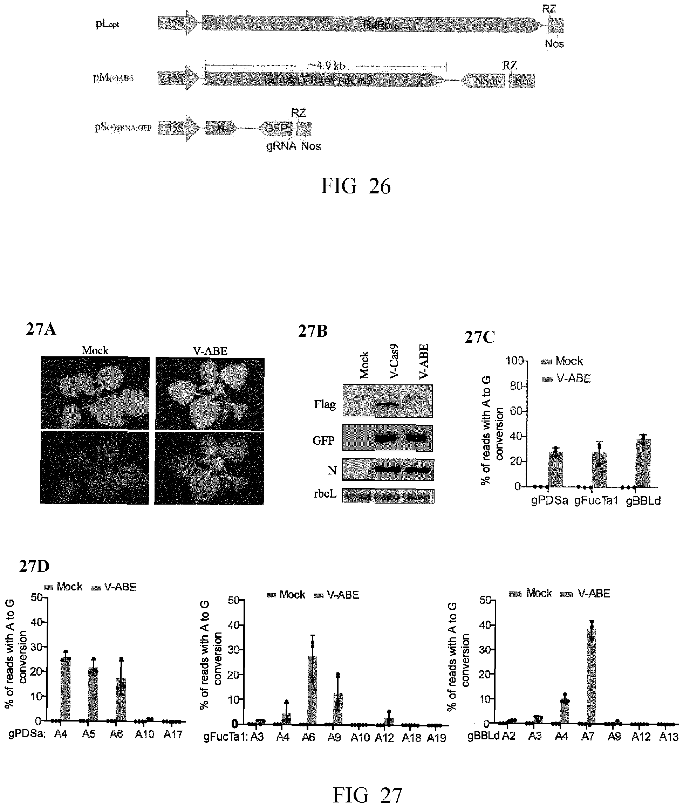

FIG. **26** are diagrams illustrating the constructs for the recovery of TSWV-ABE-gRNA. pM_{(+)ABE} is a construct containing the M antigenomic cDNA clone with the GP gene being replaced by the ABE coding sequence; 2×35S, double 35S promoter; Rz, hepatitis D virus ribozyme; NOS, NOS terminator.
FIG. **27** shows TSWV-ABE-gRNA-induced base conversion at the target loci in infected *N*. *benthamiana* plants. FIG. 27A shows the symptoms and GFP fluorescence. FIG. 27B depicts the expression of TSWV N, GFP, and ABE fusion proteins. FIG. **27C** are bar graphs showing base conversion (A to G or T to C) frequencies at the *PDSa, FucTa,* and *BBLd* target sites. FIG. **27D** are bar graphs depicting the base-editing windows within the 20-bp protospacer sequence.

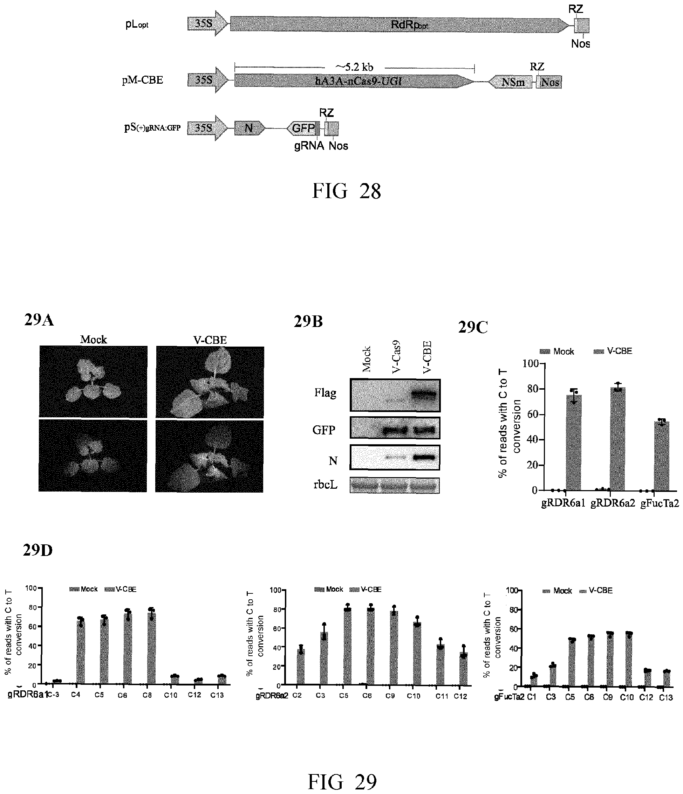

FIG. **28** are diagrams illustrating the constructs for the recovery of TSWV-CBE-gRNA. pM_{(+)CBE} is a construct containing the M antigenomic cDNA clone with the *GP* gene being replaced by the CBE coding sequence; 2×35S, double 35S promoter; Rz, hepatitis D virus ribozyme; NOS, NOS terminator.
FIG. **29** shows TSWV-CBE-gRNA-induced base conversion at the target loci in infected *N*. *benthamiana* plants. FIG. 27A depicts the symptoms and GFP fluorescence. FIG. 29B depicts the expression of TSWV N, GFP, and CBE fusion proteins. FIG. **29C** are bar graphs showing base conversion (C to T or G to A) frequencies at the *RDR6a1, RDR6a1,* and *FucTa* target sites. FIG. **29D** are bar graphs depicting the base-editing windows within the 20-bp protospacer sequence.

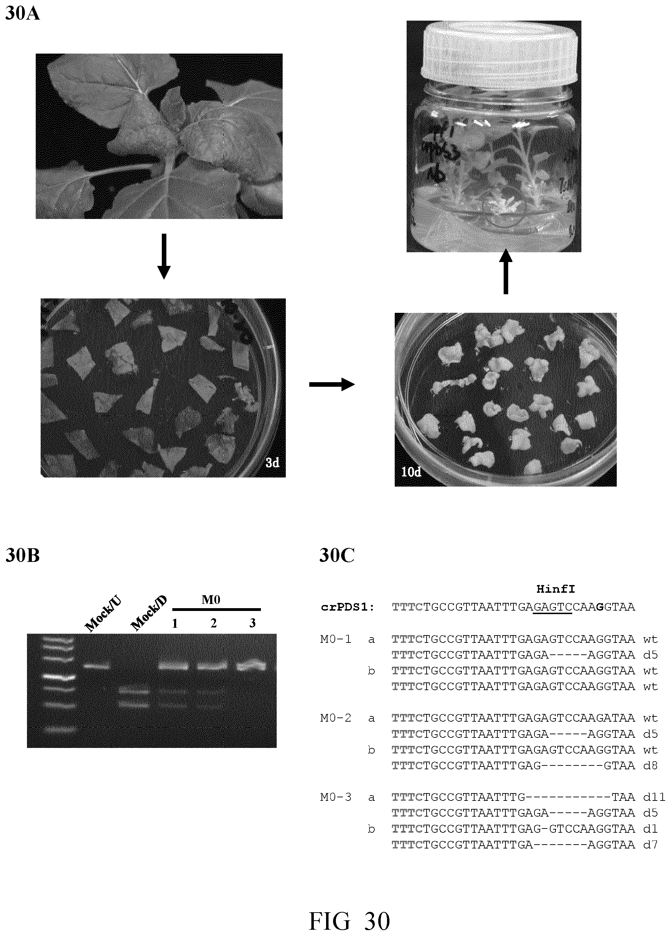

FIG. **30** shows regeneration of mutant plants from *N*. *benthamiana* cells infected with TSWV-Cas12a-crRNA by tissue culture. FIG. 30A is a flow chart depicting the experimental procedure. Upper leaves of TSWV-Cas12a-crRNA-infected N. *benthamiana* were used as explants and cultured in vitro to regenerate plants. Red circle labels an albino seedling due to *PDS* mutations. FIG. 30B are PCR/RE results showing the indel frequencies at the *PDS* target loci in three regeneratedM0 plants, including an albino seedling (M0-3) . Wt, wild type; d#, deletion of # bases. FIG. 30B depicts the target site sequences in the three M0 plants as genotyped by Sanger sequencing.

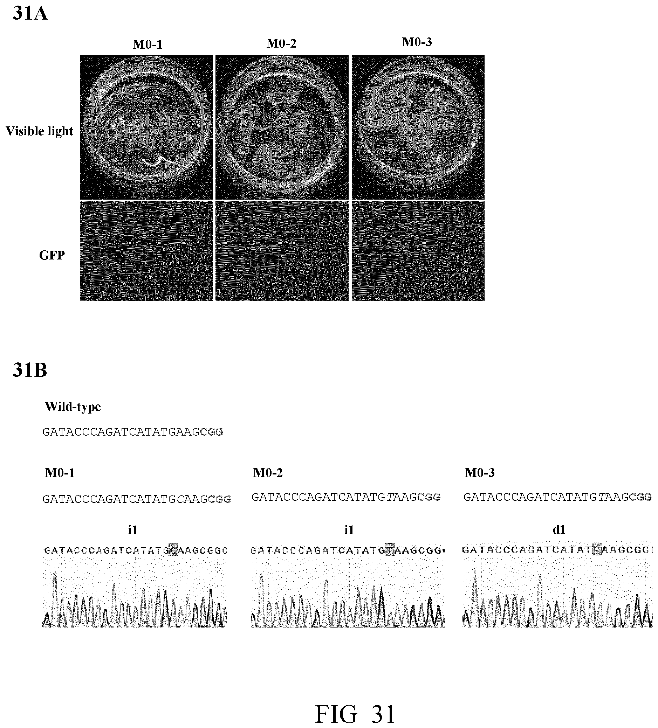

FIG. **31** shows the regeneration of N. *benthamiana* plants from leaf tissues infected with TSWV-Cas9-gRNA. FIG. 31A are images showing the regenerated plants and GFP fluorescence in symptomatic leaves. FIG. 31B depicts the sequence mutations at the gGFP locus in three regenerated plants. Italicized letters indicate base insertion, "-" indicates base deletion, and bold letters indicate PAM sequence. i1, one base insertion; d1, one base deletion.

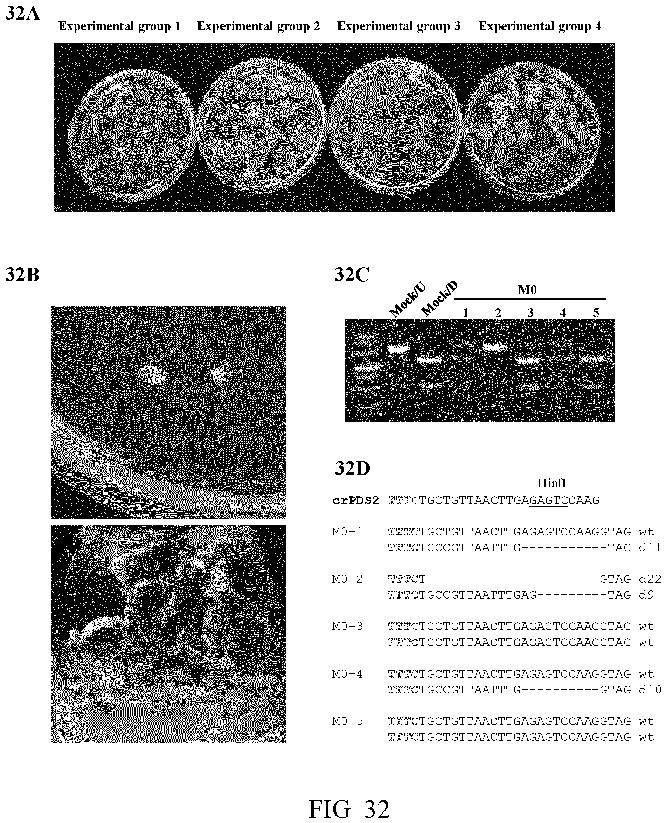

FIG. **32** shows the regeneration of tomato plants from leaf tissues infected with TSWV-Cas12a-crRNA. FIG. 32A depicts callus and shoots (red circles) induced from tomato leaf explants after three weeks of tissue culture. FIG. 32B depicts albino callus (top panel) and rooted seedlings (bottom panel). FIG. 32C depicts the indel frequencies at the *PDS* target site in five regenerated M0 plants. FIG. 32D depicts the sequence mutations at the target locus. Wt, wild type; d#, deletion of # bases.

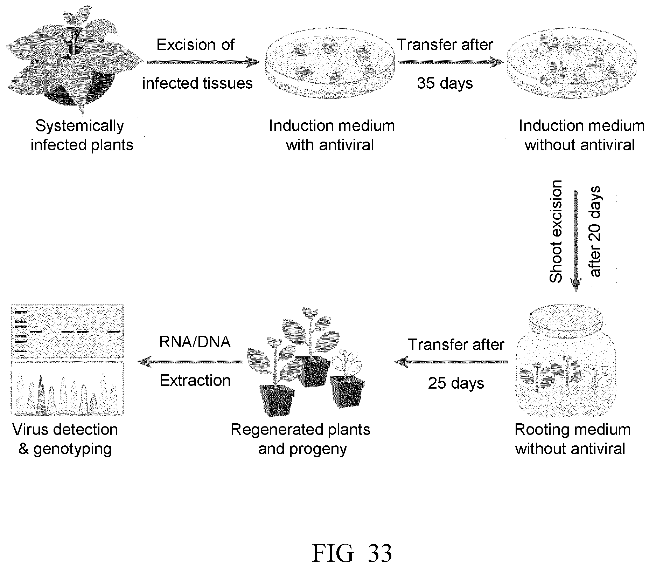

FIG. **33** is a flow chart showing the experimental procedure to eliminate the TSWV vectors during tissue culture through antiviral treatment.

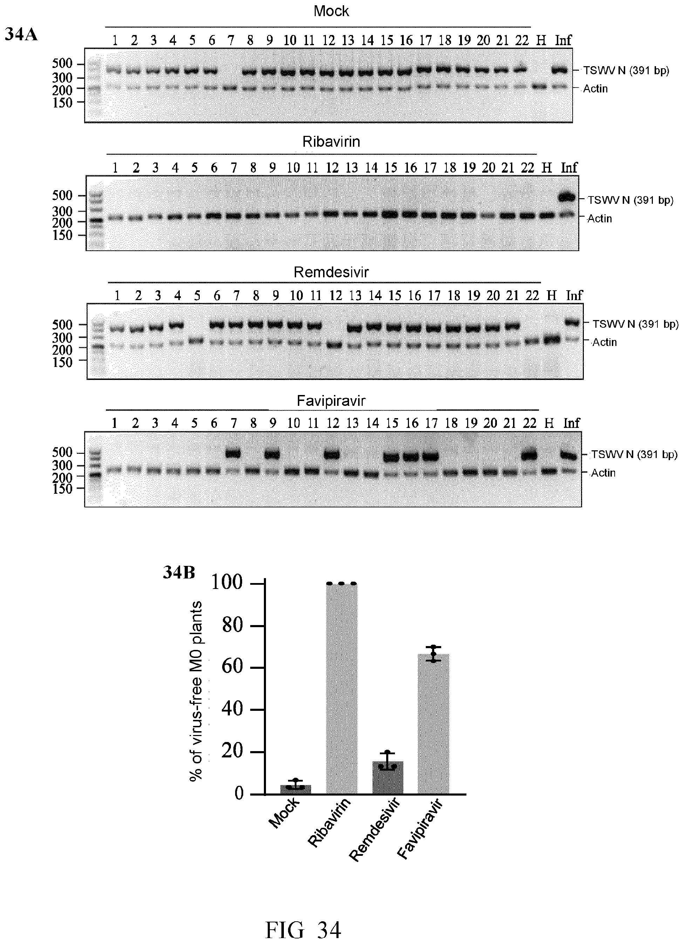

FIG. **34** shows the efficiency of TSWV clearance from regenerated *N*. *benthamiana* plants upon antiviral treatment. FIG. 34A shows the results of RT-PCR detection of virus in regenerated plants . The presence of the TSWV vector was detected using the *N*-specific primers, while the endogenous *Actin*-specific primers were used as an internal reference. FIG. 34B is a bar chart showing the percentage of virus-free regenerated plants after various drug treatments.

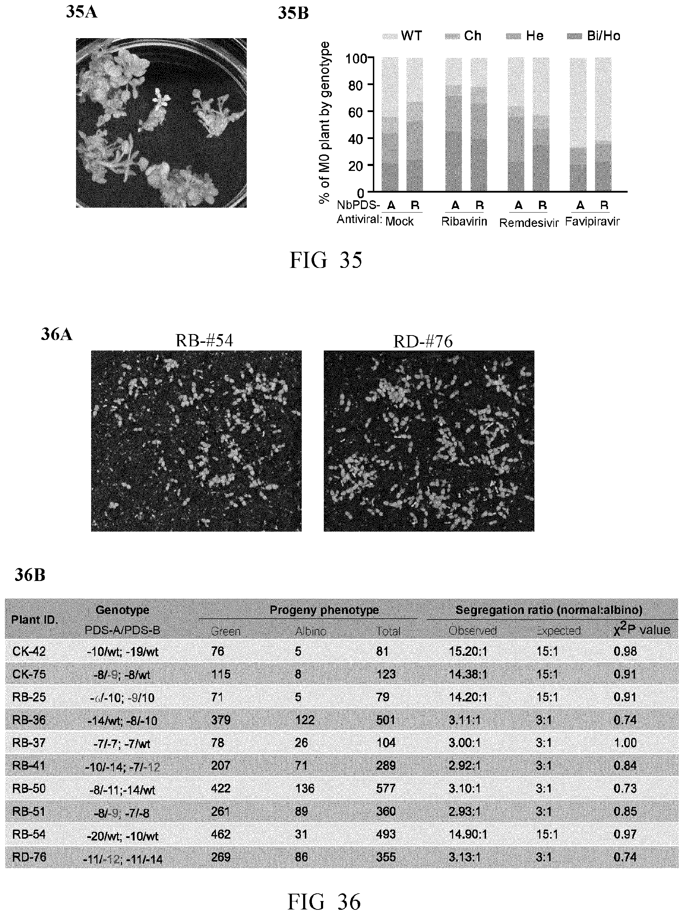

FIG. **35** shows the effect of antiviral treatment on the recovery of regenerated seedlings with targeted mutations in *N. benthamiana.* FIG. 35A shows vigorously growing regenerated *N. benthamiana* seedlings in ribavirin-containing media. One regenerated seedling showed an albino phenotype due to the knocking-out of the four alleles of *PDS.* FIG. 35B shows the percentage of regenerated seedlings by mutation genotypes after different antiviral treatments.
FIG. **36** shows the inheritance of TSWV-induced mutations from regenerated plants to the next generation. FIG. 36A are two images showing phenotypic segregation ratios in M1 progeny in accordance with 15:1 (RB-#54) and 3:1 (RD-#76). FIG. **36**B summarizes the M1 progeny phenotype segregation for 10 regeneratedM0 lines. Statistical analyses were performed using the chi-square goodness-of-fit test to an expected segregation ratio of 3:1 (M0 lines with one functional PDS homoeoallele) or 15:1 (M0 lines with two functional PDS homoeoalleles) . CK, RB, and RD in plant ID indicate the antiviral treatment: mock, ribavirin, and remdesivir, respectively. -# in genotype denotes the number of nucleotide deletions, with the in-frame deletion types (-6, -9, and -12) labeled in green letters. wt, wild-type.

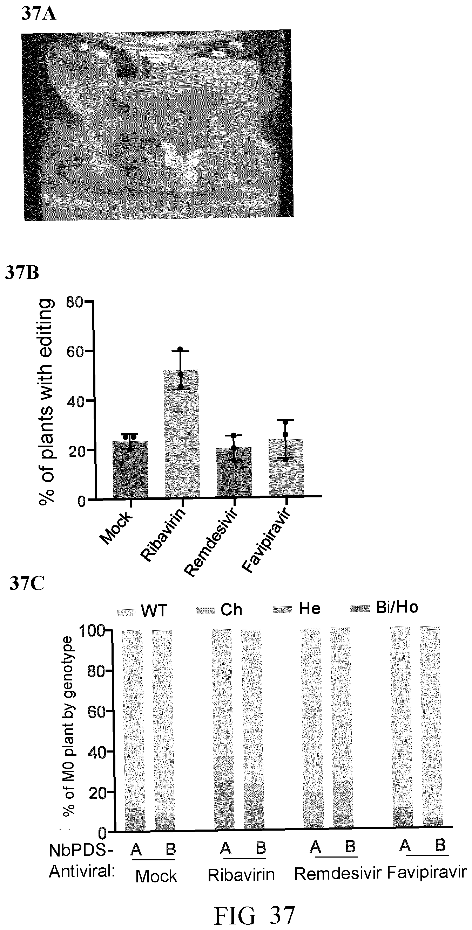

FIG. **37** shows the effect of antiviral treatment on TSWV clearance and the recovery of regenerated tobacco seedlings with mutations . FIG. 37A is an image showing regenerated tobacco seedlings. FIG. 37B shows the percentage of plants containing gene mutations across each treatment group. FIG. 37C shows percentages of regenerated plantlets with each mutation genotype.

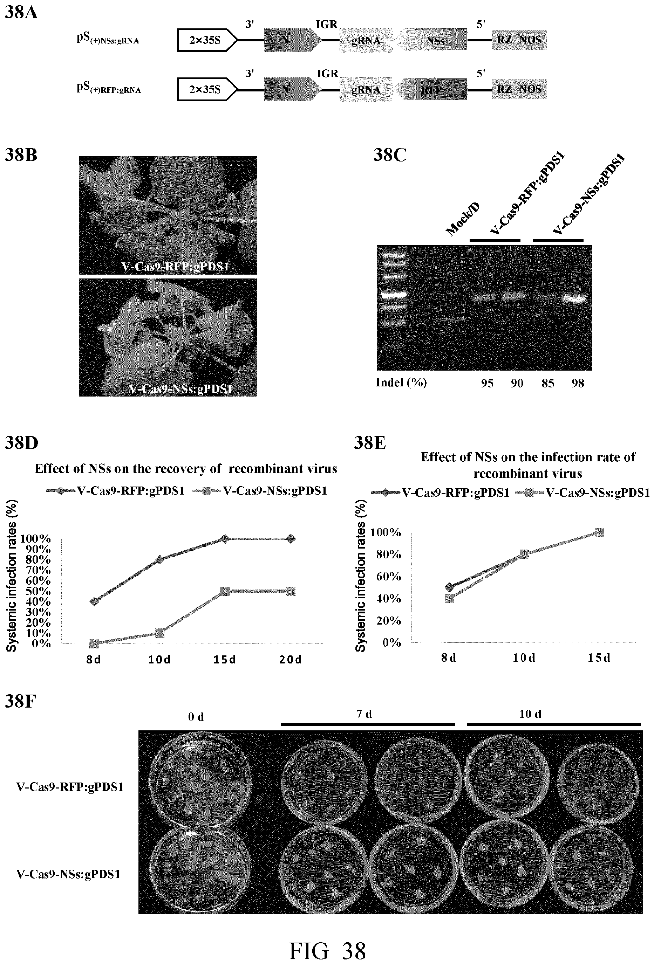

FIG. **38** shows the effects of NSs on targeted mutations induced by the TSWV-Cas9-gRNA vector and on plant regeneration from virus-infected leaf tissues. FIG. 38A shows schematic representation of the pS_{(+)NSs:gPDS1} (with NSs) and pS_{(+)RFP:gPDS1} (without NSs) constructs. FIG. 38B shows symptoms of *N*. *benthamiana* infected with TSWV-Cas9-gRNA with or without the *NSs.* FIG. 38C shows indel frequencies induced by TSWV-Cas9-gRNA with or without the *NSs.* FIG. 38D depicts the reduced recovery rate of the recombinant TSWV-Cas9-gRNA vector containing NSs. FIG. 38E is a graph showing similar infection rates of the recombinant TSWV-Cas9-gRNA vector with or without *NSs* after mechanical inoculation. FIG. 38F are images comparing the ability of *N*. *benthamiana* leaf explants infected with TSWV-Cas9-gRNA vectors with or without the *NSs* gene to differentiate into calli.

### DESCRIPTION OF EMBODIMENTS

Before explaining in detail at least one embodiment of the present invention, it should be understood that the present invention is not necessarily limited in its application to the details shown in the following specification or exemplified by the embodiments. The present invention may have other embodiments or may be implemented or accomplished in various ways. It is also to be understood that the phrases and terms used herein are for descriptive purposes and are not to be construed as limiting.

The following definitions and methods are provided to define the present disclosure better and to guide those of ordinary skill in the art in the practice of the present disclosure. Unless specified otherwise, all technical and scientific terms used herein have the same meanings as commonly understood by those skilled in the field. Moreover, the terms and laboratory procedures related to protein and nucleic acid chemistry, molecular biology, cell and tissue culture, microbiology, and virology used herein are widely used terms and routine procedures in the corresponding fields. For example, the standard recombinant DNA and molecular cloning techniques used in the present invention are well-known to those skilled in the art and are more comprehensively described in the following literature: Michael R. Green and Joseph Sambrook, Molecular Cloning: A Laboratory Manual (4th edition) ; Cold Spring Harbor Laboratory Press: Cold Spring Harbor, 2012. Also, for a better understanding of the present invention, definitions, and explanations of the relevant terms are provided below.

### Definition

The terms "contains", "includes", "has" and their synonyms as used herein refer to "including but not limited to". The scope of this terminology includes the scope of the terminology "composed of " and "essentially composed of ".

The phrase "essentially composed of " means that the composition andmethodmay include other components and/or steps, as long as the other components and/or steps do not substantially alter the basis and novel features of the required composition or method.

As used herein, the singular forms "one" and "the" include plural referents unless the context clearly indicates otherwise. For example, the terms "one compound" or "at least one compound" may include a number of compounds, including mixtures thereof.

As used herein, the term "plant" refers to whole plants, any progeny, and the parts that make up the plant. As used herein, "plant parts" include but are not limited to plant cells, plant cell cultures, plant tissues, plant tissue cultures, plant cuttings, plant organs (such as fruits, seeds, embryos, meristematic regions, callus tissue, leaves, roots, shoots, flowers, gametophytes, sporophytes, pollen, and microspores) . Plant cells can be individual cells or cell aggregates (e.g., callus tissues and cultured cells), or they can be protoplasts, gametophyte-producing cells, or cells or collections of cells capable of regenerating into complete plants. The plant cell cultures or tissue cultures can regenerate plants, and the regenerated plants have the physiological or morphological characteristics and substantially the same genotype as the plant from which the cell or tissue originated. The regenerable cells in the plant cell cultures or tissue cultures may be embryos, protoplasts, meristematic cells, callus tissues, pollens, leaves, anthers, roots, root tips, filaments, flowers, kernels, spikes, rachises, husks, or stems. Plant parts include harvestable parts and parts that can be used to propagate offspring plants. Plant parts that can be used for propagation include, but are not limited to, seeds, fruits, cuttings, seedlings, tubers, and rootstocks. A harvestable part of a plant may be any useful part of a plant, including, but not limited to, flowers, pollen, seedlings, tubers, leaves, stems, fruits, seeds, and roots.

The term "plant cell" refers to the structural and physiological unit of a plant. As used herein, plant cells include protoplasts and protoplasts with partial cell walls . Plant cells can be in the form of isolated individual cells or cell aggregates (e.g., loose callus tissues and cultured cells) and can be part of tissue units (e.g., plant tissues, plant organs, and plants) . Thus, a plant cell may be a protoplast, a cell-producing gamete, or a cell or collection of cells capable of regenerating into a complete plant. Therefore, in the embodiments herein, a seed containing a plurality of plant cells and capable of regenerating into a whole plant is considered a "plant part" . Plant "progeny" includes any subsequent generations of plants.

The terms "variety", "line", "cultivar", "germplasm" and "genotype" are used interchangeably in the present invention to refer to plant species that taxonomically belong to the same species and whose genetic combinations and phenotypes have stable characteristics that distinguish them from other plant kinds.

"Gene" is the entire nucleotide sequence required to produce a polypeptide chain or functional RNA.

The term "genome" when used concerning plant cells, covers not only chromosomal DNA present in the nucleus but also organelle DNA present in subcellular components of the cell (e.g., mitochondria, plastids). "Polynucleotides", "nucleic acid sequence", "nucleotide sequence" or "nucleic acid fragment" are used interchangeably and refer to a single- or double-stranded RNA or DNA polymer and may optionally contain synthetic, non-natural, or altered nucleotide bases. Nucleotides are referred to by their individual letter names as follows: "A" for adenosine or deoxyadenosine (corresponding to RNA or DNA, respectively), "C" for cytidine or deoxycytidine, "G " denotes guanosine or deoxyguanosine, "U" denotes uridine, "T" denotes thymine, "I" denotes inosine, and "R" indicates purine (A or G), "Y" indicates pyrimidine (C or T), "K" indicates G or T, "H " denotes A or C or T, and "N" denotes any nucleotide.

The term "genetic material" is the DNA that transmits genetic information between the parent and the offspring.

The term "sequence" refers to a nucleotide sequence of any length, which may be DNA or RNA; it may be straight-stranded, circular, or branched, and may be single- or double-stranded.

The term "expression" refers to the conversion of sequence information into the corresponding expression product, including direct transcription products (e.g., mRNA, tRNA, rRNA, antisense RNA, nuclease, structural RNA, or any other type of RNA) or proteins produced by translation of mRNA. Expression products also include RNAmodifiedby, for example, cap addition, polyadenylation, methylation, and proteins modified by, for example, methylation, acetylation, phosphorylation, ubiquitination, ADP-ribosylation, myristoylation, and glycosylation.

The term "encoding" refers to a DNA polynucleotide sequence that can be transcribed into RNA that translates a protein (mRNA) or a DNA polynucleotide sequence that can be transcribed into RNA that does not translate a protein (non-coding RNA such as tRNA, rRNA, etc.); or an RNA polynucleotide sequence that can be translated into a protein.

The terms "polypeptide", "peptide", and "protein" are used interchangeably in the present invention to refer to polymers of amino acid residues.

The term "fusion protein" refers to a polypeptide chain resulting from the expression of two or more genes encoding polypeptides linked together, which may be connected by a specific linker peptide.

"Codon-optimization" refers to replacing one or more codons in the encoding sequence with synonymous codons to enhance the expression of the target protein in a specific host cell. Each organism shows some degree of codon utilization difference or preference, and to improve the expression level of the gene in the target organism, replacing underutilized or rare codons in the target gene with codons preferred by the target organism without changing amino acids. In addition, codon-optimization includes inactivation of suspicious polyadenylation sites, exon-intron splicing sites, sequences that can form secondary structures, and balancing GC content to improve gene expression at the transcriptional level. "Codon-optimization" is a conventional means of gene design, and there are published patents and public computer programs in the field for optimizing codons of genes intended for expression in specific plant species, for example, codon optimization software available from several commercial nucleic acid synthesis companies, for example, codon usage database available at www.kazusa.orjp/codon/, "Method for achieving improved polypeptide expression" (U.S. patents 881,224,7), and "Method and device for optimizing a nucleotide sequence for the purpose of expression of a protein" (U.S. patents 8224578), Murray et al. (Nucleic Acids Res 1989, 17:477-497) and Nakamura et al. (Nucleic Acids Res, 2000, 28: 292).

The percentage of "identity" describes the extent to which a polynucleotide or peptide segment is identical in a sequence (e.g., nucleotide sequence or amino acid sequence) alignment. Sequence alignment is generated by manually comparing two sequences, such as the sequence shown herein as a reference and another sequence, to produce the highest number of matching elements, such as individual nucleotides or amino acids, while allowing gaps to be introduced into either sequence. The "identity score" of a sequence compared to a reference sequence is the number of matching elements divided by the entire length of the reference sequence, excluding gaps introduced into the reference sequence by the matching process. As used herein, the "percent identity" ("% identity") is the identity score multiplied by 100.

As used herein, "recombinant" nucleic acids refer to non-naturally occurring nucleic acids, such as two otherwise separate nucleic acid fragments joined together by artificial intervention to produce a combination of nucleic acid fragments with the intended function. This artificial combination is often accomplished by chemical synthesis or by artificial manipulation of separate segments of the nucleic acid (e.g., by genetic engineering techniques).

As used herein, the term "chimeric sequence" as applied to a nucleic acid or polypeptide refers to the combination of two or more sequences of different origins into a single recombinant sequence by manual intervention or a regulatory sequence and nucleotide sequence of interest of the same origin but arranged in a manner different from that typically found in nature.

The terms "heterologous" and "exogenous" are used interchangeably herein and refer to polynucleotide or polypeptide sequences that are not naturally present in a nucleic acid or protein. Heterologous nucleic acid sequences may be linked to naturally occurring nucleic acid sequences (or variants thereof) (e.g., by means of genetic engineering) to produce chimeric nucleic acid sequences.

The term "vector" refers to a self-replicating nucleic acid molecule, such as a plasmid, phage, virus, or cosmid, that is transferred to a recipient cell by genetic engineering techniques.

The terms "nucleic acid construct", "recombinant vector", "recombinant nucleic acid construct" or "recombinant expression vector" are used interchangeably herein and refer to DNA molecules comprising a vector and at least one insert, and also include functional RNA molecules produced by transcription of these constructs, or RNA equivalents synthesized chemically according to the sequence of the nucleic acid construct. Nucleic acid constructs are typically recombinant expression vectors generated for the purpose of expressing and/or propagating the insert or for the purpose of constructing other recombinant nucleic acid sequences. The inserted DNA molecule may or may not be operably linked to a promoter sequence and may or may not be operably linked to a DNA regulatory sequence.

The term "regulatory sequence" refers to a specific nucleic acid sequence present in the internal or collateral (5' or 3' end) sequence of a coding sequence that regulates transcription, RNA processing or, stability, or translation. Regulatory sequences include but are not limited to, promoters, enhancers, introns, transcriptional regulatory elements, polyadenylation signals, RNA processing signals, translation enhancers, etc.

The term "operably linked" means that the regulatory element, including but not limited to the promoter sequence and transcription termination sequence, is linked to a nucleic acid sequence (e.g., a coding sequence or open reading frame) such that the transcription and translation of the nucleotide sequence are controlled and regulated by said regulatory element.

As used in the present invention, a "promoter" is a DNA regulatory region capable of binding RNA polymerase and initiating transcription of a downstream (3' direction) coding or non-coding sequence, referring to a nucleic acid fragment capable of controlling the transcription of another nucleic acid fragment. In certain embodiments, the promoter is a promoter capable of controlling downstream gene transcription in a plant cell, whether or not it is of plant cell or viral origin. The promoter may be a constitutive promoter, a tissue-specific promoter, a developmentally regulated promoter, or aninducible promoter.

"Introduced" into a plant as used herein means to transform a nucleic acid molecule (e.g., plasmid, linear nucleic acid fragment, RNA, etc.) or protein into a plant cell by methods including, but not limited to, *Agrobacterium-mediated* transformation, biolistic transformation, electroporation, PEG transformation, etc., enabling said nucleic acid or protein to function in the plant cell.

"Agroinfiltration" refers to a method in plant biology for the induction of gene expression in plants by injecting a suspension of *Agrobacterium tumefaciens* into plant tissues, in which *Agrobacterium* present in the intercellular space transfer target genes located within the binary plasmid transfer DNA (T-DNA) to plant cells to obtain transient expression. *Agrobacterium* infiltration is applicable to a wide range of plants but is most effective in *Nicotiana benthamiana* and *Nicotiana tobacum.*

"Agroinfection" or "agroinfiltration" refers to a method of using *Agrobacterium tumefaciens* to inoculate infection factors (such as viruses or viroids) into plants. Viral DNA or cDNA sequences are cloned into the T-DNA region of *Agrobacterium tumefaciens* binary plasmids and transferred into plant cells through *Agrobacterium tumefaciens* infection. Replication of viruses or viroids within cells usually leads to systemic infection and the generation of symptoms. The term "transient expression" refers to the introduction of exogenous nucleic acid sequences into plant cells, tissues, organs, or individuals through agroinfiltration, biolistic transformation, viral vector infection, PEG-mediated protoplast transformation, etc. The exogenous nucleic acid sequences may or may not be integrated into plant chromosomes, and the exogenous nucleic acid fragments may be maintained at the introduced sites for expression for a period of time.

The term "virus-infectious clone" refers to a nucleic acid construct or plasmid containing a viral sequence that can rescue an active recombinant virus when introduced into a suitable host cell.

The term "viral rescue" refers to the process of introducing a viral infectious DNA clone or cDNA clone into a suitable host cell and producing an infectious virus.

For this document, "recombinant virus" refers to a virus generated using recombinant DNA technology. Unlike naturally occurring wild-type viruses, the sequence of a recombinant virus can be artificially modified through recombinant DNA technology.

The terms "viral vector", "recombinant viral vector", "recombinant viral expression vector", and "recombinant viral vector system" are used interchangeably herein and refer to viruses that have been genetically engineered in an experimental setting for the purpose of introducing a heterologous nucleic acid sequence into a host cell, tissue, organ or individual organism.

In some embodiments, the viral vector system may be a combination of one or more recombinant nucleic acid constructs or recombinant viral vectors, which can replicate, transcribe, and express heterologous sequences when introduced into host cells. In some embodiments, the viral vector system may be a combination of strains comprising different nucleic acid constructs or viral vectors, respectively, preferably said strains being *Agrobacterium;* in certain embodiments, said viral vector system may also be a genetically engineered viral particle.

The term "virion" refers to an infective viral structural form containing a genomic nucleic acid core and a protein capsid, with or without an outer envelope. For the purposes of this paper, "viral particle" includes a virus nucleocapsid particle without an envelope structure and with infective activity. The viral "positive-strand" RNA and "negative-strand" RNA are relative to the polarity of messenger RNA (mRNA), with the "positive-strand" RNA having the same polarity as mRNA and the "negative-strand" RNA being complementary to mRNA. For negative-strandedRNA viruses, as the name suggests, the genomic RNA wrapped in the viral particle is the negative-stranded RNA, also called virion RNA (vRNA); conversely, the complementary positive-stranded RNA is called complementary RNA (vRNA) or antigenomic RNA (agRNA).

In the present invention, "genetic modification", "gene modification", "nucleic acid modification" and "gene editing" refer to modification of the genome of an organism by means of genetic engineering to alter the structure or composition of the genomic sequence, such as altering the sequence of a gene, including single or multiple deoxyribonucleotide deletions, substitutions, insertions, or a combination of thereof, or to produce heritable epigenetic modifications, such as DNA methylation, histone modifications, etc.

"Genetically modified plant" means a plant that has been genetically engineered to include the introduction of single or multiple deoxyribonucleotide insertions, deletions, substitutions, or combinations thereof into its genome, and in which the modified genetic sequence is stably inherited.

"Nucleic acid modifying enzyme" refers herein to an enzyme that specifically targets and alters the structure or composition of intracellular nucleic acid sequences, such as altering the sequence of a gene, including single or multiple deoxyribonucleotide deletions, substitutions, insertions, or combinations thereof, or producing heritable epigenetic modifications such as DNA methylation, histone modifications, etc.

"Sequence-specific endonuclease", "endonuclease", "nuclease", and "nucleic acid modifying enzyme" are used interchangeably herein to refer to enzymes with activity for cleaving or modifying nucleic acids.

"Cas nuclease", "CRISPR nuclease", "CRISPR/Cas protein", "Gas protein", and "Gas effector protein" are used interchangeably herein and refer to a nuclease that includes a Cas protein or fragment thereof (e.g., a protein containing the active DNA cleavage domain of Cas and/or the guide RNAbinding domain of Cas) and is an RNA-directed nuclease. Cas proteins are protein components of the CRISPR/Cas (clustered regularly interspaced short palindromic repeats and their associated systems) genome editing system that can target and cleave DNA target sequences to form DNA double- or single-stranded breaks under the guidance of a guide RNA.

The terms "guide RNA," "gRNA," and "sgRNA" are used interchangeably herein to refer to an RNA molecule that binds and directs nucleases to specific sites in the genome. A guide RNA comprises a nucleotide sequence sufficiently complementary to a target sequence to hybridize to that target sequence and a nucleotide sequence that binds to a Cas protein to form a CRISPR/Cas complex. In the CRISPR/Cas9 nuclease system, the guide RNA is typically an RNA binary complex molecule formed by hybridization of a partially complementary CRISPR RNA (crRNA) and trans-acting CRISPR RNA (tracrRNA), or a gRNAmolecule formed by artificially ligating the two RNA molecules. In the CRISPR/Cas12a nuclease system, the guide RNA consists of crRNA only, without the tracrRNA molecule.

"Base editing" is a technique for editing specific bases of intracellular nucleic acid molecules. Sequence-specific nucleic acid modification enzymes deaminate single or multiple bases of intracellular target DNA or RNA molecules, and the cellular DNA replication and repair pathway converts the deaminated bases into other bases, such as cytosine to thymine or adenine to guanine.

A "base editor" is a reagent that specifically targets and modifies bases (e.g., A, C, G, T, or U) of a nucleotide (e.g., DNA or RNA) and causes the modified bases to be converted. Depending on its modified bases, the reagents can be classified as adenine base editor (ABE), cytosine base editor (CBE), etc. The "base editor" usually consists of Cas9 nickase (nCas9) or catalytically inactive dead Cas9 (dCas9) fused with base deaminase and other effector proteins, which can be directed by guide RNA to a target sequence. The deaminated bases are converted to other bases by the cellular DNA replication and repair pathway after "base editor" catalyzing target sequence deamination.

The term "Protospacer adjacent motif sequence (PAM) " refers to a nucleic acid sequence in the region of a DNA target sequence that is recognized by the corresponding Cas nuclease. PAM is essential for the cleavage of the target sequence by the guide RNA-directed Cas nuclease.

As used herein, "target DNA" is a DNA polynucleotide containing a "target site" or "target sequence". The terms "target site", "target sequence", and "protospacer DNA" are used interchangeably herein and, in the context of the present invention, refer to a segment of target DNA containing a nucleic acid sequence that binds to a guide RNA.

### Plant genome editing system

The present invention discloses a method for delivering sequence-specific nucleic acid modifying enzymes using a broad host spectrum plant RNA virus vector for plant gene editing, more specifically, including, but not limited to, a method for delivering genome editing elements and modifying plant genetic material through the viral vector system. An engineered infectious viral vector is used to carry a nucleic acid sequence that expresses the sequence-specific nucleic acid modifying enzyme intracellularly after infecting a plant. The nucleic acid modification enzymes target and cleave the target site to produce DNA double-strand breaks or modify specific sites to create base modifications in the plant genome. Then, cellular DNA repair machinery completes the modification of the plant's cellular genetic material, producing one or more types of base deletion, insertion, substitution, or a combination thereof.

Sequence-specific nucleases commonly used for genome editing include meganucleases, zinc finger nucleases, transcription-like activator effector nucleases, and CRISPR/Cas nucleases. The meganucleases, zinc finger nuclease, and transcription activator-like effector nuclease systems specifically target DNA sites through the DNA binding domain of the nuclease protein. The CRISPR/Cas nuclease system relies on a short guide RNA to guide the Cas protein to recognize the target sequence, thus eliminating the need to assemble multiple DNA binding units and requiring only the introduction of the Cas protein and a customized guide RNA into the cell. The CRISPR/Cas nuclease system is the most broadly used genome editing tool.

CRISPR/Cas systems have been classified into two major classes and six types (I~ VI) based on the number and function of Cas genes, among which the most facile gene editing tools are Class 2 type II and V systems that require only a single Cas effector protein. Type II CRISPR systems include CRISPR/Cas9 systems such as the *Streptococcus pyogenes* CRISPR/SpCas9 system and the *Staphylococcus aureus* CRISPR/SaCas9 system, which consists of the Cas9 protein, crRNA, and tracrRNA. The crRNAhybridizes with a portion of the tracrRNA complementary sequence to form a double-stranded RNA, and the structure is cleaved by endogenous double-stranded RNA enzymes to help the maturation of crRNA processing; meanwhile, crRNA hybridizes with tracrRNA to form a binary complex that binds to the Cas9 protein and directs the latter to target DNA sequences that are paired with the crRNA proto-spacer sequence (protospacer). The CRISPR/Cas9 system engineered for gene editing generally employs a single guide RNA (sgRNA or gRNA) formed by joining the crRNA and tracrRNA together, which contains a sequence structure that binds to Cas9 and a protospacer sequence that pairs with the target DNA. Type V CRISPR/Cas systems include CRISPR/Cas12a (also known as Cpf1), such as *Acidaminococcus* CRISPR/AsCas12a, *Lachnospiraceae bacterium* CRISPR/LbCas12a, and *Franisella novicida* CRISPR/FnCas12a, and other homologous proteins exhibit RNA-directed DNA endonuclease activity. The CRISPR/Cas12a nuclease system requires only a single guide RNA, crRNA, and does not involve an tracrRNA. The crRNA contains sequences that bind to Cas12a, as well as protospacer sequences that pair with the target DNA. In addition, Cas12a has its pre-crRNA processing activity. It can cleave transcripts containing an array of multiple crRNAs to generate multiple guide RNAs, making it easier to achieve precise processing of crRNAs and simultaneous expression of multiple crRNAs for multiplexed gene editing (Fonfara et al., Nature, 2016, 532: 517-521; Zetsche et al. Nat Biotechnol, 2017, 35: 31-34).

Methods for designing CRISPR/Cas9 or CRISPR/Cas12a nucleases for gene editing are publicly available (Jinek et al., Science, 337: 816-821; Cong et al., Science, 2013, 339: 819-823; Zetsche et al., Cell, 2015, 63: 759 -771), and SpCas9 and LbCas12a amino acid sequences are available in the SwissProt database search (accession numbers Q99ZW2 and 5ID6_A) . In short, the guide RNA sequence determines the targeting specificity, so only the targeting sequence (original spacer sequence) on the gRNA needs to be changed to alter the gene target of the Cas nuclease. Usually, Cas proteins are fused at one or both ends with one or more specific protein localization signals, such as the nuclear localization signal (NLS), which is sufficient to translocate the Cas protein and the guide RNA complex to a specific organelle (e.g., the nucleus) to perform its function. When Cas protein and engineered guide RNA are co-introduced or co-expressed within the cell, Cas protein and guide RNA form a complex and is directed by the guide RNA to bind to a genomic target site, while the Cas protein cleaves one or both DNA strands of the target sequence . Broken DNA strands can be repaired by the intracellular non-homologous end joining (NHEJ) repair machinery, which directly rejoins the double-stranded split ends, but this repair mechanism is prone to base deletions, insertions or substitutions at the break junction.

If an exogenous DNA recombination template is provided to the cell in which a double-stranded DNA break occurs for directing the repair of the broken DNA ends by the mechanism of homologous recombination (HR), desired sequence changes can be introduced into the genome through the repair template. The recombination template can be linear or circular single- or double-stranded DNA containing sequences homologous to both sides of the genomic target sequence being cleaved by the nuclease (homologous arms) . The length of the homologous arms can be 1, 5, 10, 15, 20, 25, 30, 35, 40, 45, 50, 60, 70, 80, 90, 100 nucleotides or longer. When the genomic target site DNA is broken due to nuclease cleavage, the free DNA ends can be used as a template for DNA synthesis mediated by homologous sequences from exogenously provided DNA, resulting in the incorporation of a predetermined exogenous sequence at the target site.

In some embodiments, the present invention employs the CRISPR/LbCas12a nuclease for plant gene editing; in other embodiments, the CRISPR/SpCas9 nuclease is employed for plant gene editing.

In addition, the Cas fusion protein may also contain one or more heterologous effector protein structural domains, ideally with the two structural domains connected by a linker peptide sequence. Protein structural domains that can be fused to Cas proteins include but are not limited to, antigenic epitope tags (e.g., HA, His, Flag, myc), reporter proteins (green fluorescent protein, β-glucosidase, luciferase), DNA methylation enzymes, transcriptional activation domains, transcriptional repression domains, histone modifying enzymes, DNA topoisomerases, DNA recombinases, cytosine deaminases, uracil DNA glycosylase inhibitor, adenine deaminase, reverse transcriptase, etc. The Cas endonuclease domains can be inactivated by mutations and still maintain the ability to form complexes with guide RNAs and target specific genomic DNA sequences . For example, when amino acidmutations are introduced into the active site of the RuvC or HNH endonuclease domain of the Cas9 protein, such as mutation of the tenth aspartate to alanine (D10A) or mutation of the 840^{th} histidine to alanine (H840A), or mutation of the 854^{th} asparagine to alanine (N854A), or mutation of the 863^{rd} asparagine to alanine (N863A), these mutations produce nCas9 mutant proteins that can only cut one strand and become a nickase. If both catalytical sites of the RuvC and HNH domains are mutated, the resulting dCas9 mutant will lose the ability to cut DNA. dCas9 or nCas9 protein can be fused to the specific effector protein domains mentioned above to target specific genomic sequences under the direction of a guide RNA for DNA modification (cytosine methylation, adenine base editing, cytosine base editing, etc.), transcriptional regulation (activation, repression, histone modification, etc.), DNA recombination, DNA synthesis, or other applications (Anzalone et al., Nat Biotechnol., 2020, 38: 824-844).

The base editor contains nuclease-inactivated Cas proteins (e.g., Cas9 and Cpf1, etc.) and DNA-dependent base deaminases such as cytosine deaminase and adenine deaminase. Cytosine deaminases are naturally occurring or engineered deaminases such as human APOBEC3A (hA3A). Naturally occurring adenine deaminases often use RNA as a substrate to convert adenosine on single-stranded RNA to inosine (I) by deamination. Recently, DNA-dependent adenine deaminases capable of converting deoxyguanosine on single-stranded DNA to inosine (I) using single-stranded DNA as a substrate have been obtained through directed evolutionary approaches based on *E. coli* tRNA adenine deaminase A (ecTadA) (Gaudelli et al., Nature, 2017, 551: 464-471) . The cytosine base editor (CBE) and adenine base editor (ABE) proteins can specifically target intracellular nucleic acid under the direction of a guide RNA and perform a deamination reaction on cytosine bases (C) or adenine bases (A) located within the target site without generating a double-stranded DNA break (DSB) . After DNA replication and repair, deaminated cytosine bases or adenine bases are replaced with thymine (T) or guanine (G), respectively, thus forming a targeted substitution from C to T (cytosine base editing) or A to G (adenine base editing).

Methods for designing guide RNAs in ABE and CBE systems for performing base modifications have been published in the literature (Komor et al., Nature, 2016, 533: 420-424; Gaudelli et al., Nature, 2017, 551: 464-471). Briefly, the guide RNA sequence determines the targeting specificity so that the gene target of the base editor can be altered by simply changing the targeting sequence (protospacer sequence) in the gRNA. When the base editor and the guide RNA are co-introduced into the cell, the base editor protein can produce a base conversion within the active window of the target sequence.

In some embodiments, the present invention employs CBE for plant genome base editing; in some embodiments, ABE is used for plant genome base editing.

### Viral vector-based nuclease delivery system

As described above, the present invention provides viral vector systems and methods for the delivery of CRISPR/Cas elements to plant cells.

Viral vectors are used to express one or more CRISPR elements within plant cells. For example, one Cas nuclease nucleic acid sequence, one or more gRNA nucleic acid sequences are inserted into different viral vectors and operably linked to viral transcriptional regulatory sequences; preferably, two or more CRISPR/Cas elements are inserted into the same viral vector, and operably linked to one or more transcriptional regulatory sequences. Cas nuclease and one or more gRNA nucleic acid sequences can be inserted at the same site on the viral genome and controlled by the same regulatory sequence; the Cas nuclease and one or more gRNA nucleic acid sequences can be inserted at different sites on the viral genome and controlled by different regulatory sequences.

More specifically, considering that most plant DNA viral vectors and positive-stranded RNA viral vectors can only package a limited length of exogenous fragments, making it difficult to express the larger size CRISPR/Cas nuclease genes (>4 kb), the present invention describes the engineering of a plant segmented, negative-stranded RNA virus, tomato spotted wilt virus (TSWV), as a preferred viral vector for delivery of CRISPR/Cas. In certain embodiments, the TSWV vector may comprise one or more (e.g., 2, 3, 4, 5, 6, 7, 8, 9, 10, or more) insertion sites, said insertion sites being upstream and/or downstream of the viral transcriptional regulatory elements . In a preferred embodiment, the Cas protein coding sequence contained within one genome segment of the recombinant TSWV vector is operably linked to the viral transcriptional regulatory sequence, and one or more gRNA coding sequences contained within the other genome segment are operably linked to the viral transcriptional regulatory sequence. Within TSWV-infected cells, viral transcriptional regulatory sequences drive the expression of Cas proteins and gRNAs; gRNAs direct Cas nucleases to target specific DNA sites in the genome, and Cas nucleases cleave one or both strands of the target sequence.

Tospoviruses refer to virus species taxonomically belonging to the genus *Orthotospovirus,* family *Tospoviridae,* order *Bunyavirales* in the phylum *Negarnaviricota.* Tospoviruses include Groundnut bud necrosis virus (GBNV), Groundnut ringspot virus (GRSV), Groundnut yellow spot virus (GYSV), Impatiens necrotic spot virus (INSV), tomato chlorotic spot virus (TCSV), Tomato spotted wilt virus (TSWV), Watermelon silver mottle virus (WSMoV), Zucchini lethal chlorosis virus (ZLCV), Groundnut chlorotic fan-spot virus (GCFSV), Watermelon bud necrosis virus, Melon yellow spot virus (MYSV), Iris yellow spot virus (IYSV), Chrysanthemum stem necrosis virus (CSNV), etc.

Tospoviruses have conserved morphological, biological, and molecular characteristics. The particles of tospoviruses are quasi-spherical in shape with a diameter of about 80-110 nanometers (nm) and are wrapped by a membrane whose outer layer consists of approximately 5 nm of glycoprotein spikes. Three single-stranded, negative-sense genomic RNAs of different lengths, i.e. , L, M and S genomic fragments, with a total genome length of about 16,600 bases (nt), are wrapped inside the viral particle . Each genomic segment has a highly conserved nine bases at the 3' end and is complementary to the 5' end to form a panhandle structure, giving each genomic segment a pseudo-loop shape, and this structure plays an important role in viral transcription and replication (FIG. 2) . Both the M and S genome segments use an ambisense coding strategy, with the positive- and negative-sense strands each containing an open reading frame separated by an intergenic region (IGR) that contains the cis-element responsible for transcriptional termination. The M genome RNA encodes the nonstructural protein M (NSm), which plays an important role in viral intercellular movement and long-distance movement, and the antigenomic RNA encodes the glycoprotein precursor (GP), which is processed and matured into G_{N} and G_{C} glycoproteins. Both glycoproteins are spikes anchored to viral particles and required for virus particle maturation and insect transmission. The S genomic RNA encodes the nonstructural protein NSs as an RNA silencing suppressor, while the antigenomic RNA encodes the nucleocapsid protein (N) . The N protein is the viral structural protein that encapsidates the viral genome to form the nucleocapsid. The minimal infection unit of the negative-stranded virus is the nucleocapsid, which is the ribonucleoprotein complex formed by the viral genomic RNA packaged by the N protein in association with the RdRp protein.

Tospoviruses are transmitted by the insect vector thrips under natural conditions and affect a variety of food crops, vegetables, flowers, and other plants. Tospoviruses can readily infect their natural or experimental host plants by mechanical rubbing and grafting under laboratory conditions. Tomato spot wilt virus (TSWV) has a vast host range of more than 1090 monocotyledonous and dicotyledonous plants from more than 85 families, including Solanaceae, Cucurbitaceae, Asteraceae, Leguminosae, and Cruciferae (Parrella et al., J Plant Pathol, 2003, 85: 227-264), such as *Abelmoschus esculentus, Ageratum houstonianum, Allium cepa, Amaranthus caudatus, Amaranthus hybridus, Amaranthus retroflexus, Amaranthus inosus, Amaranthus viridis, Amaryllis sp., Ananas comosus, Anemone, Apium graveolens* var. dulce , *Aquilegia vulgaris , Arabidopsis thaliana, Arachis hypogaea, Arctium lappa, Arum palaestinum, Begonia sp. , Belamcanda chinensis, Beta vulgaris, Bidens pilosa, Brassica campestris, Brassica oleracea* var. *botrytis, Brassica pekinensis, Brassica rapa* ssp. *Chinensis, Calceolaria herbeohybrida, Calendula officinalis, Callistephus chinensis, Campanula glomerata, Campanula isophylla, Campanula latiloba, Campan ula pyramidalis, Canavalia gladiata, Canavalia obtusifolia, Canavalia occidentalis, Capsella bursa-pastoris, Capsicum annuum, Capsicum frutescens, Capsicum Chinense, Capsicum Baccatum, Capsicum Pubescens, Carica papaya, Cassia occidentalis, Cassia tora, Catharanthus roseus, Centaurea cyanus, Cheiranthus cheiri, Chenopodium album, Chenopodium ambrosioides, Chenopodium amaranticolor, Chenopodium murale, Chrysanthemun coronarium, Chrysanthemun indicum, Chrysanthemun leucanthemum, Chrysanthemun morifolium, Cicer arietinum, Cichorium endiva, Cichorium intybus, Cineraria, Citrulluslanatus, Coffeaarabica, Convolvulusarvensis, Conyza bonariensis, Cordyline terminalis, Coriandrum sativum, Coreopsis drummondii, Coronopus didymus* (synonym *Youngia japonica), Crotalaria incana, Crotalaria juncea, Crotalaria pallida* (synonym *Crotalaria mucronata*)*, Cucumis melo, Cucumis sativus, Cucurbita maxima, Cucurbita moschata, Cucurbita pepo, Cyclamen persicum, Cynara scolymus, Dahlia pinnata, Dahlia variabilis, Datura stramonium, Delphinium sp., Desmodium triflorum, Desmodium unicinatum, Duboisia leichhardtii, Emilia sonchifolia, Foeniculum vulgare, Fragaria vesca, Galinsoga parviflora, Galinsoga quadriradiata, Gladiolus sp., Glycine max, Glycine soja, Gomphrena globosa, Gossypium hirsutum, Gossypium barbadense, Helianthus annuus, Hibiscus tiliaceus , Hippeastrum hybridum, Hippeastrum reginae, Hippeastrum rutilum, Hoya carnosa, Hydrangea macrophylla, Hyoscyamus niger, Impatiens holsii, Impatiens sultanii, Impatiens wallerana, Ipomea batatas, Lactuca sativa, Lathyrus odoratus, Lathyrus odoratus, Leonotis nepetaefolia, Limonium latifolium, Lupinus leucophyllus, Lupinus mutabilis, Lupinus polyphyllus, Lycium procissimum, Lycopersicon esculentum, Lycopersicon hirsutum, Lycopersicon pimpinellifolium, Malva parviflora, Matthiola incana, Medicago polymorpha, Melilotus officinalis, Nerium oleander, Nicandra physaloides, Nicotiana acuminata, Nicotiana bigelovii, Nicotiana clevelandii, Nicotiana glutinosa, Nicotiana rustica, Nicotiana sylvestris, Nicotiana tabacum, Papaver nudicaule, Pelargonium hortorum, Petunia hybrida, Phaseolus angularis, Phaseolus vulgaris, Phlox drummondii, Physalis angulate* (synonym *Physalis minima*)*, Physalis spp., Pisum arvense, Pisum sativum, Plantagomajor, Plantago rugelii, Polygonum convolvulus, Portulaca oleracea, Primula sp. , Primula malacoides, Primula sinensis, Ranunculus sp., Rubus idaeus, Rudbeckia amplexicaulis, Saintpaulia ionantha, Salpiglossis, Scabiosa, Schizanthus, Sechium edule, Senecio cruentus, Senecio jacobea, Sesamum indicum, Sinningia eciosa, Solanum capsicastrum, Solanum carolinense, Solanum laciniatum, Solanum mammosum Solanum melongena, Solanum nigrum, Solanumnodiflorum, Solanum seaforthianum, Solanum triflorum, Solanum tuberosum, Sonchus oleraceus, Spinacia oleracea, Stachys arvensis, Stapellia, Stellaria media, Streptosolen jamesonii, Tagetes patula, Taraxacum, Tephrosia purpurea, Tribulus terrestris, Trifolium repens, Trifolium subterraneum, Tropaeolum majus, Vallota, Verbena brasilliensis, Verbena litoralis, Verbesina enceloides, Vicia faba, Vigna mungo, Vigna radiata, Vigna unguiculata, Xanthium saccharatum, Zantedeschia aethopica, Zantedeschia albo-maculata, Zantedeschia elliottiana, Zantedeschia melanoleuca, Zantedeschia rehmannii, Zinnia elegans, etc.*

As described above, one aspect of the present invention provides a TSWV expression vector system. As can be understood by one skilled in the art, genetic manipulation of viral genomes and viral vector development entail the availability of a virus infectious clone, i.e., viral (c)DNA clones that can produce recombinant viruses upon introduction into a suitable host cell. The process, also known as virus rescue, allows for the expression of viral or non-viral sequences carried by the virus in the host cell.

Methods for construction and applications of plant negative-stranded RNA virus infectious cDNA clones and viral expression vectors have been published in related articles or patents, such as those related to Sonchus yellow net virus (SYNV) (Wang et al., PLoS Pathog, 2015, 11: e 1005223; patent CN 105039388 B), Barley yellow striate mosaic virus (BYSMV) (Gao et al., New Phytol, 2019, 223: 2120-2133, patent CN 110511955 A), Tomato spotted wilt virus infectious clone system (Feng et al., PNAS, 2020, 117: 1181-1190). The process of rescuing a recombinant negative-stranded RNA virus from cloned DNA involves: 1) construction of a nucleic acid construct that transcriptionally generates viral genomic or antigenomic RNA and at least one nucleic acid construct that expresses the viral core protein; 2) co-introduction of the above nucleic acid constructs into a suitable host cell in the form of a circular plasmid, or a linear DNA fragment, or a nucleic acid fragment generated by in vitro transcription; 3) intracellular expression of viral RNA and protein that assemble to form an infectious unit to produce a recombinant virus. The above introduction methods are known in the art, such as *Agrobacterium* transformation, biolistic bombardment, mechanical inoculation with nucleic acid (plasmid DNA or *in vitro* transcribed RNA), PEG-mediated transformation, electroporation, microinjection, liposome transformation, or nanoparticle-mediated transformation.

After introducing the above nucleic acid constructs into suitable plant host cells, the plant RNA silencing response is an important limiting factor preventing their efficient expression and subsequent generation of recombinant viruses. As an important mechanism to defend against exogenous nucleic acid invasion, the plant RNA silencing response inhibits transient expression of the introduced nucleic acid (Johansen et al., Plant Physiol 2001, 126: 930-938) and also limits replication and infection of nascent recombinant viruses (Kasschau et al., Virology 2001, 285: 71-81). To improve transient expression efficiency and recombinant plant negative-strand RNA virus recovery, the strategy known in the art is to co-express viral suppressors of RNA silencing (Wang et al., PLoS Pathog, 2015, 11: e 1005223; Ma & Li, Viruses, 2020, 12: 1459). A large number of viral RNA silencing suppressors are known, including tomato bushy stunt virus p19, tobacco etch virus P1/Hc-Pro, barley stripe mosaic virus γb, TSWV NSs, turnip crinkle virus p38, flock house virus B2, influenza virus NS1, and Ebola virus VP35, etc. When co-expressed, these silencing suppressors have different modes of action and have a synergistic effect on promoting recombinant negative-stranded virus recovery (Ganesan et al., J Virol 2013, 87:10598-10611; Ma & Li, Viruses 2020, 12: 1459).

Methods for constructing TSWV vector systems and recombinant viral rescue technology are disclosed in relevant embodiments of the present invention. The TSWV genome sequences, i.e., its S, M, and L segment sequences, are disclosed in SEQ ID NO: 1, 2, and 3, respectively. It should be understood in the art that the TSWV genome sequences need not be 100% identical to the disclosed sequences as set forth in SEQ ID NO: 1, 2, 3. The TSWV genome sequences have at least 70%, at least 80%, at least 90%, at least 95%, at least 99% identity with the whole genome sequence disclosed herein; or the amino acid sequence of the RdRp protein encoded therein has at least 70%, at least 80%, at least 90%, at least 95%, at least 99% identity.

In order to generate nucleic acid constructs that can be transcribed to produce viral genomic RNA, in certain embodiments, TSWV S, M, and L genomic nucleic acid sequences are inserted into plant expression vectors and are operably ligated downstream of a promoter sequence. Numerous suitable plant expression vectors are known to those with ordinary skills in the art and are commercially available, such as pCambia, pGreen, pCASS, PBin19, pGD, pCB301, and others. Suitable promoters include constitutive or inducible promoters derived from plant endogenous genes or plant viruses, such as the actin promoter, the ubiquitin promoter, or the cauliflower mosaic virus (CaMV) 35S promoter, etc. A version of the CaMV 35S promoter (SEQ ID NO: 9), which produces viral RNAs with precise 5' end, is used in certain embodiments.

In the TSWV nucleic acid constructs described above, the 3' end of the viral sequence is operably attached to a self-cleaving hepatitis D virus ribozyme sequence so that the transcribed viral RNAs can be cleaved by the ribozyme to produce a faithful viral 3' end sequence. The design of the hepatitis D virus ribozyme is described in papers (Sharmeen et al., 1998, J. Virol. 62, 2674-2679; Feng et al., Proc Natl Acad Sci U S A, 2020, 2: 1181-1190) and a U.S. Patent 522,533,7 (Ribozyme compositions and methods for use) and is within the knowledge of those skilled in the art.

In some embodiments, the TSWVL segment nucleic acid construct comprises an L antigenomic cDNA sequence being operably linked to a 35S promoter to transcribe the L antigenomic (positive-sense) RNA; the M segment nucleic acid construct comprises an M genomic or antigenomic cDNA sequence being operably linked to a 35S promoter to transcribe the M genomic (negative-sense) or antigenomic (positive-sense) RNA; the S segment nucleic acid construct contains the S antigenomic cDNA sequence being operably linked to the 35S promoter to transcribe the S antigenomic (positive-sense) RNA.

Rescuing negative-stranded viruses requires intracellular co-expression of viral core protein and viral RNA to assemble viral infectious ribonucleoprotein complexes. In some embodiments, the L and S antigenomic RNA transcripts (positive) can be used as translation templates to express the viral core proteins RdRp (or termed L) and N, respectively, which in turn package viral (anti) genomic RNAs to form a viral nucleocapsid that serves as a template for viral replication to produce viral progeny genome.

Those skilled in the art should understand that the L and S segment constructs can also be made to contain the L and S genomic cDNA sequences being operably ligated to the 35S promoter, but in this case, the L and S genomic RNA transcripts (negative sense) are unable to express the viral core protein and therefore require the RdRp and N provided from helper expression vectors to translate the viral core proteins that function to encapsidate the viral genomic RNA and initiate recombinant viral replication and infection.

The TSWV L genome nucleic acid construct is further modified in embodiments of the present invention by replacing the sequence encoding RdRp in the L genome (SEQIDNO: 4) with a codon-optimized coding sequence. Preferably, the optimized RdRp sequence is shown in SEQ ID NO: 5. It is understood that gene sequence optimization is a routine operation in the art and different versions of optimized sequences can be obtained for the same gene depending on the optimization method and that the codon-optimized sequences provided in the present invention are only examples. It is conceivable that other optimized sequence versions may also be suitable to improve the expression of TSWV RdRp in plant cells and assist recombinant virus production.

In certain embodiments, the TSWV M genomic segment may be manipulated by recombinant DNA techniques, e.g., insertion of heterologous nucleic acid sequences or partial or complete deletion or replacement of the GP sequence (SEQ ID NO: 6) . In some preferred embodiments, all or part of the GP coding sequence is replaced with a heterologous reporter gene nucleic acid sequence.

In certain embodiments, the TSWV S genomic segment may be manipulated by recombinant DNA technology, e.g., by insertion of a heterologous nucleic acid sequence upstream or downstream of the coding sequence of the *NSs* gene (SEQ ID NO: 7), or by partial or complete deletion or replacement of the *NSs* gene. In some preferred embodiments, all or part of the sequence of the *NSs* gene is replaced with a heterologous reporter gene nucleic acid sequence.

In certain embodiments, heterologous nucleic acid sequences contained in the TSWV M and S genome segments are operably linked to viral transcriptional regulatory cis-elements, for instance, the promoter sequences responsible for mRNA transcription within the non-coding regions of the M and S genomes and the cis-acting element sequence responsible for transcription termination within the viral intergenic region.

Further, the above recombinant TSWV S, M, and L genomic nucleic acid constructs are introduced into suitable plant cells under conditions sufficient to achieve recombinant virus rescue. To facilitate recombinant virus production, in certain embodiments, the nucleic acid constructs co-introduced into the cell further comprise helper expression vectors encoding one or more RNA silencing suppressors being operably linked to the promoter, such as the 35S promoter. Preferably, in some embodiments, the RNA silencing suppressor is a tomato bushy stunt virus p19, tobacco etch virus Hc-Pro, barley stripe mosaic virus γb, and TSWV NSs, or a combination of two or more of these suppressors. If desired, the nucleic acid constructs co-introduced into the cells may include TSWV N and RdRp expression vectors.

In some embodiments, the nucleic acid construct compositions introduced into plant cells comprise: a) nucleic acid constructs comprising the recombinant TSWV S, M, and L segment sequences; b) helper expression vectors encoding one or more viral suppressors of RNA silencing; and, if desired, c) one or more helper expression vectors encoding TSWV N and L proteins.

The method of "introduction into plant cells" described in this aspect is a conventional method in the art, such as *Agrobacterium-mediated* transformation, biolistic bombardment, pollen tube introduction, PEG-mediated fusion, electroporation, microinjection, ultrasonic transformation, liposome transformation, or nanoparticle-mediated transformation, etc., through which viral nucleic acid sequence-containing circular plasmids or linear DNA fragments, or *in vitro* transcribed viral RNA, etc. are introduced into suitable eukaryotic cells.

In certain embodiments, the method of introduction into plant cells is the *Agrobacterium-mediated* transformation method, wherein one or more nucleic acid constructs are transformed into suitable plant cells through agroinfiltration. The nucleic acid construct is a plasmid vector suitable for *Agrobacterium-*-mediated transformation, i.e., a binary plasmid vector comprising a transfer DNA (T-DNA) region that is transferred to the plant cell during *Agrobacterium* infection of the plant. Nucleic acid constructs can also be introduced into cells by other means, such as biolistic transformation, leaf rubbing, PEG fusion, etc. In this case, the nucleic acid constructs can be introduced into the cell in the form of DNA, RNA, or ribonucleoprotein complexes.

In the preferred example of the present invention, the transferred T-DNA fragment within the plasmid is introduced into N. *benthamiana* cells by agroinfiltration of the leaf tissues to initiate viral transcription, expression, and generation of recombinant virus. *N. benthamiana* is one of the most suitable plants for *Agrobacterium*-mediated trans formation, but a variety of other plants also have varying degrees of sensitivity to this technique.

In certain embodiments, the recombinant TSWV is devoid of functional GP and can infect plants but cannot be acquired by the insect vector thrips and be transmitted to other plants via the insects. Thus, the recombinant virus and method provided by the present invention is biologically containable.

In certain embodiments of the present invention, the recombinant TSWV lacks functional NSs, is still infective to plants, and is less virulent than the wild-type virus.

The TSWV vector may comprise one or more (e.g., 2, 3, 4, 5, 6, 7, 8, 9, 10, or more) insertion sites upstream and/or downstream of viral transcriptional regulatory elements. In some embodiments, the recombinant TSWV M and S segments each carry a reporter gene that is operably linked to a viral transcriptional regulatory sequence. Preferably, the reporter genes are the green fluorescent protein gene (*GFP*) and the red fluorescent protein gene (*RFP*). After introducing the recombinant nucleic acid constructs into plant cells, replication and systemic infection of the recombinant virus can be tracked by the expressed reporters.

In another aspect, the present invention discloses a TSWV expression vector system for delivering sequence-specific nucleic acid modifying enzymes to plants and a method of constructing the vector system.

In certain embodiments, the recombinant TSWV M segment contains the coding sequence of one component of the nucleic acid modifying enzyme operably linked to the viral transcriptional regulatory sequence, while the S segment contains another component of the nucleic acid modifying enzyme operably linked to the viral transcriptional regulatory sequence. In TSWV-infected cells, viral transcriptional regulatory sequences drive the co-expression of the nucleic acid modifying enzyme components, forming a complex that targets specific DNA sites in the genome and produces modifications.

As described above, the TSWV vectors containing multiple insertion sites and recombinant viruses containing exogenous nucleic acid sequences in the M and/or S segment, respectively, or simultaneously, can systematically infect plants and express one or more exogenous sequences. Thus, the present invention provides viral expression vector systems that can express sequence-specific nucleic acid modifying enzymes. More specifically, at least two polynucleotide sequences encoding the components of CRISPR/Cas nucleic acid modifying enzymes are inserted into the recombinant TSWV genome and operably linked to viral transcriptional cis-elements. When the recombinant TSWV vector is introduced into plant cells, the nucleic acid modifying enzyme is expressed.

In certain embodiments, the CRISPR/Cas nucleic acid modifying enzyme is the *Lachnospiraceae bacterium* CRISPR/LbCas12a nuclease.

In certain embodiments, the CRISPR/Cas nucleic acid modifying enzyme is the *Streptococcus pyogenes* CRISPR/SpCas9 nuclease.

In certain embodiments, the CRISPR/Cas nucleic acid modifying enzyme is an ABE based on the CRISPR/SpCas9 system.

In certain embodiments, said CRISPR/Cas nucleic acid modifying enzyme is a CBE based on the CRISPR/SpCas9 system.

To improve the efficiency of Cas effector protein expression in plant cells, provided in certain embodiments are plant codon-optimized coding sequences, such as the rice codon-optimized LbCas12a (as shown in SEQ ID NO: 10), the N. *benthamiana* codon-optimized SpCas9 (as shown in SEQ ID NO: 11), the N. *benthamiana* codon-optimized ABE (as shown in SEQ ID NO: 84), and the *N*. *benthamiana* codon-optimized CBE (as shown in SEQ ID NO: 85) . It will be appreciated by those with ordinary skill in the art that methods of codon-optimization for specific host species are feasible and known.

As shown by the sequences above, the Cas effector proteins of the present invention are fusion proteins containing nuclear localization sequences at their N-terminal and/or C-terminal ends to achieve sufficient accumulation of the CRISPR/Cas complex in the plant cell nucleus. In addition, depending on the DNA location to be edited, the Cas fusion protein may also include other localization sequences, such as cytoplasmic localization sequences, chloroplast localization sequences, mitochondrial localization sequences, etc. In some embodiments, the sequence encoding Cas12a protein replaces the GP coding sequence in the TSWV M genomic segment, and the expression of Cas12a protein is controlled by the viral transcriptional cis-elements. A heterologous nucleic acid sequence encoding a guide RNA (crRNA) for Cas12a is inserted upstream or downstream of the NSs open reading frame in the S genomic segment and controlled by the viral transcriptional cis-element. Preferably, the NSs coding sequence in the S genome is replaced by a reporter gene so that the recombinant virus infection can be tracked by the reporter. In certain embodiments of this aspect, the guide RNA transcript is processed by Cas12a nuclease to trim the terminal polynucleotide sequences derived from the virus.

In certain embodiments, the GP coding sequence in the M genomic segment is replaced by the Cas9 coding sequence, and the expression of the Cas9 protein is controlled by the M transcriptional cis-elements. The heterologous nucleic acid molecule encoding the Cas9 guide RNA is inserted upstream or downstream of the NSs coding sequence in the S genome, and the expression of the guide RNA is controlled by the S genome transcriptional cis-elements. Preferably, the NSs coding sequence is replaced by a reporter gene. In certain embodiments, the guide RNA transcript is trimmed by the Cas9 nuclease and endogenous RNA processing machinery to remove the terminal polynucleotide sequences derived from the virus.

In certain embodiments, the GP coding sequence in the M genomic segment is replaced by the ABE coding sequence, and the expression of the ABE protein is controlled by the M transcriptional cis-elements. The heterologous nucleic acid molecule encoding the guide RNA is inserted upstream or downstream of the NSs coding sequence in the S genome, and the expression of the guide RNA is controlled by the S genome transcriptional cis-elements. Preferably, the NSs coding sequence is replaced by a reporter gene.

In certain embodiments, the GP coding sequence in the M genomic segment is replaced by the CBE coding sequence, and the expression of the CBE protein is controlled by the M transcriptional cis-elements. The heterologous nucleic acid molecule encoding the guide RNA is inserted upstream or downstream of the NSs coding sequence in the S genome, and the expression of the guide RNA is controlled by the S genome transcriptional cis-elements. Preferably, the NSs coding sequence is replaced by a reporter gene.

In certain embodiments, the recombinant TSWV vector contains two heterologous nucleic acid sequences encoding a Cas effector protein and a guide RNA (crRNA or gRNA) inserted in the M and S segments, respectively. Prior solutions in the art have shown that nucleic acid sequences encoding Cas effector proteins and gRNA (or crRNA) elements can be expressed in tandem with a single RNA transcript (Campa et al., Nat Methods, 2019, 16: 887-893; Tang et al., Plant Biotechnol J, 2019, 17: 1431- 1445) . It is, therefore, conceivable that the two nucleic acid sequences encoding the Cas protein and the gRNA element can be joined to be expressed as a single nucleic acid sequence from a single genome segment of TSWV, such as the M, S, or L segment.

Further, in certain embodiments, the recombinant TSWV vector described above expresses the CRISPR/Cas nucleic acid modifying enzyme in plant cells. The aforementioned nucleic acid constructs containing the TSWV recombinant genomic segments and the helper expression vectors are introduced into suitable plant cells under conditions sufficient to rescue recombinant viruses.

In certain embodiments, the nucleic acid construct composition introduced into a plant cell comprises: a) nucleic acid constructs comprising recombinant TSWV S, M and L genomic sequences, wherein the S segment comprises one or more heterologous nucleic acid sequences encoding a CRISPR guide RNA, the M segment comprises one or more heterologous nucleic acid sequences encoding a Cas nuclease component, and the L genome comprises a codon-optimized viral RdRp sequence (SEQ ID NO: 5); b) helper expression vectors encoding one or more viral suppressors of RNA silencing; and, if desired, c) helper expression vector encoding the TSWV N and L proteins.

The method of "introduction into plant cells" described in the present invention is a conventional method in the field, e.g., by transforming a circular plasmid, linear DNA fragment, or *in vitro* transcribed RNA containing a viral nucleic acid sequence through *Agrobacterium*-mediated transformation, particle bombardment, pollen tube introduction, PEG-mediated fusion, electroporation, microinjection, laser transformation, ultrasonic transformation, liposome transformation, or nanoparticle-mediated transformation, and other methods to introduce into suitable eukaryotic cells. The method of introduction into plant cells described in certain embodiments of the present invention is the agroinfiltration method, whereby *Agrobacterium tumefaciens* carrying the above-mentioned nucleic acid constructs (binary vectors) were infiltrated into the leaf tissues of N. *benthamiana.* This introduces the DNA fragment within the binary plasmids into N. *benthamiana* cells to initiate transcription, expression, and generation of recombinant virus.

TSWV is a tri-segmented, negative-stranded RNA virus. When recovering recombinant TSWV from cloned DNAs, the S, M, and L segments need to be present simultaneously in the same cells in order to assemble active infection units and initiate recombinant virus infection. Due to the large differences in the length of the three genomic segments, their expression and assembly efficiencies may differ from one to the others. In addition, the insertion of large heterologous sequences, such as the 3.9 kb LbCas12a coding sequence, the 4.2 kb SpCas9 coding sequence, the 4.9 kb ABE coding sequence, or the 5.2 kb CBE coding sequence, can further affect the rescue efficiency of the recombinant genomic segments. Some embodiments in the present invention show that efficient generation of recombinant viruses requires optimal adjustment of the polarity (positive or negative sense) of the TSWV cDNA in the nucleic acid construct and the ratio of the three genomic nucleic acid constructs.

In certain embodiments, the recombinant L and S segment sequences are operably linked to the 35S promoter in the antigenomic cDNA orientation to produce positive sense viral RNAs that can serve as a template for translation of the N and L proteins and for genomic replication.

In certain embodiments, the recombinant M segment sequence comprising the LbCas12a coding sequence is operably linked to the 35S promoter in an antigenomic cDNAorientation to transcribe a positive sense RNA.

In certain embodiments, the recombinant M segment sequence comprising the SpCas9 coding sequence is operably linked to the 35S promoter in an antigenomic or genomic cDNA orientation, preferably in a genomic cDNA orientation, to transcribe a positive sense RNA.

In certain embodiments, the recombinant M genomic segment comprising the ABE or CBE heterologous coding sequence is operably linked to the 35S promoter in the antigenomic cDNA orientation to transcribe a positive-sense RNA.

When Agrobacterium-infiltration is used to introduce viral expression vectors, the ratio of each recombinant viral construct introduced into the plant can be altered by adjusting the concentration of the *Agrobacterium* cultures (showed as OD₆₀₀) to achieve coordinated expression and efficient assembly of the three recombinant TSWV genome segments. It is understood that it is possible to rescue recombinant viruses from cloned viral cDNAs within a certain range of the ratios of viral constructs.

In certain embodiments, recovery of recombinant TSWV is achieved when agrobacterial cultures carrying the recombinant S genomic construct containing crRNA, the recombinant M genomic construct containing LbCas12a, and the recombinant L genomic construct are adjusted at the ratios of 2:2:1, or 1:2:1, or 1:10:2. Preferably, the most efficient virus recovery is achieved when the S:M:L ratio is set to 1:10:2.

In certain embodiments, efficient recovery of recombinant TSWV is achieved when agrobacterial cultures carrying the recombinant S genomic construct containing crRNA, the recombinant M genomic construct containing SpCas9, and the recombinant L genomic construct are adjusted at the ratio of 1:10:2.

In certain embodiments, efficient recovery of recombinant TSWV is achieved when agrobacterial cultures carrying the recombinant S genomic construct containing crRNA, the recombinant M genomic construct containing ABE or CEB, and the recombinant L genomic construct are adjusted at the ratio of 1:10:2.

Limited packaging capacity is the most significant impediment to the use of recombinant viral vectors to deliver sequence-specific nucleases. In some embodiments, the developed TSWV vector has multiple insertion sites and unusual carrying capacity. The TSWV vectors carrying CRISPR/Cas nuclease fragments longer than 5 kb can systematically infect plants and express the exogenous sequences. It is understood that TSWV can also express other CRISPR/Cas nucleases of similar or smaller sizes, including, but not limited to, SaCas9, STl1Cas9, STl2Cas9, St3Cas9, NmCas9, AsCas12a, FnCas12a, CasX, CasY, CasΦ nucleases, their mutants or derivatives, such as the high-fidelity Cas nucleases espCas9, HFCas9, Cas9 nickase (nCas9), dead Cas9 (dCas9), prime editor, etc. TSWV delivery of other small-sized nuclease, such as meganuclease (~1 kb), zinc finger nucleases (~2 kb), and transcription activator-like effector nucleases (~3 kb), is also anticipated.

In some embodiments, The TSWV vector is used as a typical example of the genus *Othortospovirus.* It is understood that this methodology can be applied to other virus members in this genus or other phylogenetically related segmented plant negative-stranded RNA viruses having similar genomic structure and biological properties, including, but not limited to, Groundnut bud necrosis virus (GBNV), Groundnut ringspot virus (GRSV), Groundnut yellow spot virus (GYSV), Impatiens necrotic spot virus (INSV), Tomato chlorotic spot virus (TCSV), Watermelon silver mottle virus (WSMoV), Zucchini lethal chlorosis virus, ZLCV), Groundnut chlorotic fan-spot virus (GCFSV), Watermelon bud necrosis virus (WBNV), Melon yellow spot virus (MYSV), and Iris yellow spot virus (IYSV), and Chrysanthemum stem necrosis virus (CSNV).

### Plant genome editing based on viral delivery system

In another aspect, the present invention provides a method for modifying plant genetic material using the TSWV vector to deliver a CRISPR/Cas nucleic acid modifying enzyme complex to plant cells, wherein the Cas effector protein directed by a guide RNA targets cleave or modify specific sequences of the plant genome, and the cellular DNA repair machinery completes the modification of the plant genetic material by producing one or more types of base deletion, insertion, substitution or a combination thereof.

In certain embodiments, the nuclease delivered by the TSWV vector is a CRISPR/LbCas12a nuclease; In certain embodiments, the nuclease is a CRISPR/SpCas9 nuclease.

In certain embodiments, the base editor delivered by the TSWV vector is an ABE; In certain embodiments, the base editor is a CBE.

Methods for designing and constructing a suitable CRISPR guide RNA based on a given target sequence are known to those with ordinary skills in the art. For example, the design of LbCas12a crRNA can be found in Fonfara et al. (Nature, 2016, 532: 517-521) . In general, LbCas12a crRNA contains a protospacer sequence of about 23 nucleotides complementary to the target sequence and a direct repeat (DR) sequence that binds to the LbCas12a protein to form a complex, wherein the DR sequence is responsible for binding Cas12a to form the complex, and the spacer sequence targets the complementary target DNA. The target sequence has the following structure: 5'-TTTN-N_{X}-3', where N is any one base of A, G, C or T, N_{X} denotes X consecutive nucleotides, X is an integer of 15 ≤ X ≤ 30, and TTTN is the protospacer adjacent motif (PAM) sequence of LbCas12a. In some specific embodiments of the present invention, X is 23.

In addition, Cas12a has its crRNA processing activity and can cleave transcripts containing multiple crRNA alignments to generate multiple guide RNAs, making it easier to achieve precise processing of crRNAs and simultaneous expression of multiple crRNAs for multiplexed gene editing. (Fonfara et al, Nature, 2016, 532: 517-521; Zetsche et al, Nat Biotechnol, 2017, 35: 31-34).

In certain embodiments, the guide RNA for Cas12a is a single crRNA, with a structure of "direct repeat (DR) - Spacer - direct repeat (DR)". In these embodiments, the TSWV vectors express a primary transcript containing the "DR-Spacer-DR" guide RNA with virus-derived sequences at both ends, and the Cas12a protein binds to the DR sequence to process the virus-derived terminal polynucleotide through its nuclease activity to form a mature crRNA containing the "DR-Spacer" structure.

In some embodiments, the guide RNA for Cas12a is a tandem of multiple crRNAs, e.g., 2, 3, 4, 5, 6, 7, 8, 9, 10 or more crRNA sequences. Specifically, the tandem array of crRNA is expressed in the form of "DR-Spacer 1-DR-Spacer 2-DR...". In these embodiments, the TSWV vectors express a primary transcript containing a tandem repeat of crRNA with virus-derived sequences at both ends, and the Cas12a protein binds to the DR sequence to release two or more mature crRNAmolecules through its nuclease activity. The resulting target gene editing efficiency is similar to that of the corresponding TSWV vector delivering a single crRNA.

In certain embodiments, the LbCas12a and crRNA complex delivered by TSWV vectors target the crPDS1 target sequence 5'- **TTT**CTGCTGTTAACTTGAGAGTCCAAG, which is conserved in the *phytoene desaturase (PDS)* gene in N. *benthamiana* and tobacco (SEQ ID NO: 12) (bold letters are PAM sequences, hereinafter), or the crPDS2 target sequence 5'-**TTT**CTGCTGTTAACTTGAGAGTCCAAG-3' of the tomato SLPDS gene (SEQ ID NO: 13), or the crPDS3 target sequence 5'-**TTT**CTGCTGTCAACTTGAGAGTCCAAG-3' (SEQIDNO: 14) in the pepper *PDS* gene, or the crPDS4 target sequence 5'-**TTT**GACAGAAAACTGAAGAACACATAT-3' (SEQ ID NO: 15) conserved in peanut and soybean *PDS* genes.

In certain embodiments, the LbCas12a and crRNA complex delivered by the TSWV vector targets to the crFucT target sequence 5'- **TTT**GGAACAGATTCCAATAAGAAGCCT-3' ( SEQ ID NO: 86), which is conserved in the N. *benthamiana FucT* genes, or the crDCL2 target sequence 5' - **TTTG**AGAAGCTATGCTTATCTTCTTCG -3' (, SEQ ID NO: 87) in the N. *benthamiana DCL2,* or the crER target sequence 5'-**TTTA**ATGTTAAACCTTTAGTGTCCTTT -3' (SEQ ID NO: 88) in the ground cherry ER gene, or the crPDS5 target sequence 5'-AATGCAATGTATATTGATTGCCTT**AAA** -3' (SEQ ID NO: 98) conserved in the peanut *PDS* genes, or the crPDS6 target sequence 5'-**TTT**CTGCTGTCAACTTGAGAGTCCAAG -3' (SEQ ID NO: 99) in the pepper *PDS* gene.

In certain embodiments, the TSWV vector delivers LbCas12a and multiple crRNAs to form multiple LbCas12a/crRNA complexes that can simultaneously target multiple genes for multiplexed gene editing, such as tandem expression of two crRNAs (crPDS1 & crFucT; SEQ ID NO: 135) targeting the N. *benthamiana* crPDS1 and crFucT loci, or tandem expression of three crRNAs (crPDS1&FucT&crDCL2; SEQ ID NO: 136) targeting the N. *benthamiana* crPDS, crFucT and crDCL2 loci. The editing efficiency generated by multi-gRNA TSWV vectors is similar to that of the corresponding single-gRNA vector.

The design of SpCas9 gRNA is described by Ran et al. (Nat Protoc, 2013, 11: 2281-2308) . SpCas9 gRNA contains aprotospacer sequence of approximately 20 nucleotides complementary to the target sequence, as well as a sequence that binds to the SpCas9 protein to form a complex. The target sequence has the following structure: 5'-N_{X}-NGG-3', where N is any one base of A, G, C or T, N_{X} denotes X consecutive nucleotides, X is an integer of 14 ≤ X ≤ 30, and NGG is the PAM sequence of SpCas9. In certain embodiments of the present invention, X is 20.

When a gRNA is expressed by an RNA viral vector, the primary gRNA transcript typically contains a gRNA sequence embedded in virus-derived sequences at both ends. In the presence of co-expressed Cas9 that binds the gRNA sequence, the unbound virus-derived sequences can be trimmed off by cellular RNA processing enzymes to release one or more mature gRNA molecules (Mikami et al., Plant Cell Physiol, 2017, 58: 1857-1867; Ellison et al., Nat Plants, 2020, 6: 620-624).

In certain embodiments, the SpCa9 and gRNA complex delivered by the TSWV vector targets the gGFP target sequence 5'-GATACCCAGATCATATGAAG**CGG**-3' (SEQ ID NO: 16) in the N. *benthamiana GFP* transgene, or the conserved gPDS1 target sequence 5' - GGACTCTTGCCAGCAATGCT**TGG**-3' (SEQ ID NO: 17) in the *N. benthamiana* and common tobacco *PDS* genes.

In certain embodiments, the SpCas9 and gRNA complex delivered by the TSWV vector target the gPDS2 target sequence 5'-TTGGTAGTAGCGACTCCATG**GGG**-3' (SEQ ID NO: 89), which is conserved in the N. *benthamiana PDS* gene, or the gRDR6arget sequence 5'-**CCC**CTCCCCTGACTCTTACCCAACT -3' (SEQ ID NO: 90) in the *RDR6* gene, or the gSGS3 target sequence 5'- ACAAGAGTGGAAGCAGTGCT**GGG**-3' (SEQ ID NO: 91) in the *SGS3* gene.

In certain embodiments, the TSWV vector delivers SpCas9 and multiple tandem gRNAs to form multiple SpCas9/gRNA complexes that can simultaneously target multiple genes for multiplex gene editing, such as tandem expression of two gRNAs (gPDS2 & gRDR6; SEQ ID NO: 137) targeting the gPDS2 and gRDR6 loci, or tandem expression of three gRNAs (gPDS2&gRDR6&gSGS3; SEQ ID NO: 138) targeting the gPDS2, gRDR6 and gSGS3 sites. The editing efficiency generated by multi-gRNA TSWV vectors is similar to that of the corresponding single-gRNA vector.

Designing gRNAs for the base editors (ABE and CBE, etc.) is similar to the design for the SpCas9 nuclease. In certain embodiments, the ABE and gRNA complexes delivered by the TSWV vector are designed to target the gPDSa target sequence 5'-TGCAAATTGAGTTGGGAGTG**AGG**-3' (SEQIDNO: 92) in the *N*. *benthamiana PDSa* gene, target sequence 5'- CCTCTTGAGAATGTTGCTAT**TGG** -3' (gFucTa1, SEQ ID NO: 93) from the *FucTa* gene of N. *benthamiana,* or selected from the target sequence 5'-GAAATCAGAGTAAGGTGCGG**AGG** -3' (gBBLd, SEQ ID NO: 97) of the N. *benthamiana berberine bridging enzyme-like nucleic acid (BBL)* d gene.

In certain embodiments, the targets of the CBE and gRNA complexes delivered by the TSWV vectors are selected from the target sequence 5'- CACACCTTCCTCCAGGAGAT**AGG**-3' of the N. *benthamiana alpha-1,3-fucosyltransferase a* gene (*FucTa*) (gFucTa2. SEQ ID NO: 94), the target1 sequence 5'-**CCA**ATATCTAGGCGTGAGGGATT-3' (gRDR6a1, SEQ ID NO: 95) of the *RNA-dependent RNA polymerase 6a* gene (*RDR6a*) and the target2 sequence 5 '- **CCC**GACTTCATGGGGAAGGAGGA-3' (gRDR6a2, SEQ ID NO: 96) .

The guide RNAs described above may target a single gene or multiple genes, such as 2, 3, 4, 5, 6, 7, 8, 9 or 10 and more. In certain embodiments, the guide RNA targets a single gene, such as Cas12a crRNA-targeted the tomato *PDS* gene, pepper *PDS* gene, soybean *PDS* gene, and Cas9 gRNA-targeted the *GFP* transgene . In certain embodiments, the guide RNA targets two genes, such as the N. *benthamiana* and tobacco *PDSa* and *PDSb* genes, the peanut *PDSa* and *PDSb* genes.

For modifing plant genetic material, the above-mentioned TSWV vectors containing Cas effector proteins and guide RNAs targeting different genomic loci can be introduced into plant cells, tissues, or organs by any method known in the art. For example, circular plasmids, linear DNA fragments, or *in vitro* transcribed RNA containing the nucleic acid sequences of recombinant viral vectors are introduced into suitable plant cells by agroinfection, biolistic bombardment, pollen tube introduction, PEG-mediated fusion, electroporation, microinjection, ultrasonic transformation, liposome transformation, or nanoparticle-mediated transformation. In certain embodiments, the method described for introducing viral vectors into plant cells is the *Agrobacterium* infection method, whereby *Agrobacterium* cultures carrying the above-mentioned nucleic acid construct (binary vectors) are infiltrated into *benthamiana* leaf tissues. The viral nucleic acid sequence introduced into the N. *benthamiana* cells directs the production of a recombinant virus that expresses the CRISPR nuclease complex in the inoculated leaves and upper uninoculated leaves, which targets and cleaves or modifies the target sequence and induces modification of the genetic material.

TSWV viral vectors can also be introduced into plant cells by any means of plant inoculation with virus particles. For example, plant saps containing recombinant virus particles are used to infect plants through mechanical rubbing, pressurized spraying, needle-pricking, syringe injection, vacuum infiltration, biolistic bombardment, or by grafting of an infected plant with a plant to be infected. In certain embodiments, the method of virus infection described is to inoculate N. *benthamiana,* tobacco, tomato, sweet pepper, chili pepper, habanero pepper, potato, ground cherry, lettuce, peanut, soybean, and cotton with N. *benthamiana* saps containing recombinant virus particles through mechanical inoculation and pressurized spraying. Recombinant virus particles introduced into plant cells initiate virus replication and infection and express CRISPR nuclease complexes in the inoculated leaves and/or systemic leaves, which cleave or modify target sequences and induce modifications of genetic material.

After a plant is infected with a viral vector, any method known in the art can be used to identify whether a modification of the target sequence has occurred. Examples include functional assays such as inactivation or activation of reporter gene expression or amplification of the target DNA sequence using polymerase chain reaction for Surveyor and T7EI nuclease assays, restriction endonuclease protection analysis, Sanger sequencing analysis, or high-throughput sequencing analysis.

In certain embodiments, methods for identifying genetic modifications are restriction endonuclease protection analysis and Sanger sequencing analysis . Depending on the target sequence, the frequencies of target gene editing generated by the nuclease expressed in the viral vector in the present invention can range from 45% to 98%.

In certain embodiments, the method for identifying genetic modifications is high-throughput sequencing analysis. High-throughput sequencing is performed by amplifying a sequence, including the target with specific primers with adaptors using total plant DNA as the template, barcoding the amplicons in a secondary round of amplification, and then performing high-throughput sequencing. The amplicon sequencing results are compared with the wild-type sequence, and the percentage of reads with expected mutations at the target to the total reads is calculated to represent the frequency of target gene modification (editing).

Using the method disclosed in the present invention, the CRISPR nucleic acid modification enzyme is transiently expressed locally or systematically in plant tissues by the introduced recombinant TSWV viral vector, achieving modification of specific genomic sequences without the need for transformation and integration of the nuclease-encoding gene in the plant genome.

In the present invention, the target sequence to be modified can be located at any position in the genome, for example, at a functional gene body such as a protein-coding gene exon or intron, or can be located in a gene expression regulatory region such as a promoter region or an enhancer region, thereby achieving functional modification of said gene or modification of gene expression. In certain embodiments, the target sequence modification comprises deletion, insertion, substitution of one or more bases, or a combination thereof, which may result in frameshift and premature termination, amino acid substitution, or deletion.

If an exogenous DNA recombinant template is provided to direct DNA homologous recombination in plant cells infected with a recombinant TSWV virus vector, it is conceivable that the expressed nuclease cleaves the genomic target gene to produce DNA breaks, the free DNA ends can be used for DNA synthesis mediated by homologous sequences using the exogenously provided DNA as a template, thereby integrating the predetermined exogenous sequence at the target gene locus.

Plants that can be genetically modified by the methods provided by the present invention include natural or experimental hosts that can be infected with the viral vectors, such as *Abelmoschus esculentus, Ageratum houstonianum, Allium cepa, Amaranthus caudatus, Amaranthus hybridus, Amaranthus retroflexus, Amaranthus inosus, Amaranthus viridis, Amaryllis sp., Ananas comosus, Anemone, Apium graveolens* var. dulce, *Aquilegia vulgaris , Arabidopsis thaliana, Arachis hypogaea, Arctium lappa, Arum palaestinum, Begonia sp. , Belamcanda chinensis, Beta vulgaris, Bidens pilosa, Brassica campestris, Brassica oleracea* var. *botrytis, Brassica pekinensis, Brassica rapa ssp. Chinensis, Calceolaria herbeohybrida, Calendula officinalis, Callistephus chinensis, Campanula glomerata, Campanula isophylla, Campanula latiloba, Campan ulapyramidalis, Canavalia gladiata, Canavalia obtusifolia, Canavalia occidentalis, Capsella bursa-pastoris, Capsicum annuum, Capsicum frutescens, Capsicum Chinense, Capsicum Baccatum, Capsicum Pubescens, Carica papaya, Cassia occidentalis, Cassia tora, Catharanthus roseus, Centaurea cyanus, Cheiranthus cheiri, Chenopodium album, Chenopodium ambrosioides, Chenopodium amaranticolor Chenopodium murale, Chrysanthemun coronarium, Chrysanthemun indicum, Chrysanthemun leucanthemum, Chrysanthemun morifolium, Cicer arietinum, Cichorium endiva, Cichoriumintybus, Cineraria, Citrulluslanatus, Coffeaarabica, Convolvulusarvensis, Conyzabonariensis, Cordyline terminalis, Coriandrum sativum, Coreopsis drummondii, Coronopus didymus* (synonym *Youngia japonica* ), *Crotalaria incana, Crotalaria juncea, Crotalaria pallida* (synonym *Crotalaria mucronata),* , *Cucumis melo, Cucumis sativus, Cucurbita maxima, Cucurbita moschata, Cucurbita pepo, Cyclamen indicum* (synonym *Cyclamen persicum*), *Cynara scolymus, Dahlia pinnata, Dahlia variabilis, Datura stramonium, Delphinium sp., Desmodium triflorum, Desmodium unicinatum, Duboisia leichhardtii, Emilia sonchifolia, Foeniculum vulgare, Fragaria vesca, Galinsoga parviflora, Galinsoga quadriradiata, Gladiolus sp., Glycine max, Glycine soja, Gomphrena globosa, Gossypium hirsutum, Gossypium barbadense, Helianthus annuus, Hibiscus tiliaceus , Hippeastrum hybridum, Hippeastrum reginae, Hippeastrum rutilum, Hoya carnosa, Hydrangea macrophylla, Hyoscyamus niger, Impatiens holsii, Impatiens sultanii, Impatiens wallerana, Ipomea batatas, Lactuca sativa, Lathyrus odoratus, Leonotis nepetaefolia, Limonium latifolium, Lupinus leucophyllus, Lupinus mutabilis, Lupinus polyphyllus, Lycium procissimum, Lycopersicon esculentum, Lycopersicon hirsutum, Lycopersicon pimpinellifolium, Malva parviflora, Matthiola incana, Medicago polymorpha, Melilotus officinalis, Nerium oleander, Nicandra physaloides, Nicotiana acuminata, Nicotiana bigelovii, Nicotiana clevelandii, Nicotiana glutinosa, Nicotiana rustica, Nicotiana sylvestris, Nicotiana tabacum, Papaver nudicaule, Pelargonium hortorum, Petunia hybrida, Phaseolus angularis, Phaseolus vulgaris, Phlox drummondii, Physalis angulate* (synonym *Physalis minima*), *Physalis spp. , Pisum arvense, Pisum sativum, Plantago major, Plantago rugelii, Polygonum convolvulus, Portulaca oleracea, Primula sp., Primula malacoides, Primula sinensis, Ranunculus sp., Rubus idaeus, Rudbeckia amplexicaulis, Saintpaulia ionantha, Salpiglossis, Scabiosa, Schizanthus, Sechiumedule, Senecio cruentus, Senecio jacobea, Sesamum indicum, Sinningia eciosa, Solanum capsicastrum, Solanum carolinense, Solanumlaciniatum, Solanum mammosum Solanummelongena, Solanum nigrum, Solanumnodiflorum, Solanum seaforthianum, Solanum triflorum, Solanum tuberosum, Sonchus oleraceus, Spinacia oleracea, Stachys arvensis, Stapellia, Stellaria media, Streptosolen jamesonii, Tagetes patula, Tarax patula, Taraxacum, Tephrosia purpurea, Tribulus terrestris, Trifolium repens, Trifolium subterraneum, Tropaeolum majus, Vallota, Verbena brasilliensis, Verbena litoralis , Verbesina enceloides, Vicia faba, Vigna mungo, Vigna radiata, Vigna unguiculata, Xanthium saccharatum, Zantedeschia aethopica, Zantedeschia albo-maculata, Zantedeschia elliottiana, Zantedeschia melanoleuca, Zantedeschia rehmannii, Zinnia elegans, etc.*

In certain embodiments, the plants include N. *benthamiana,* tobacco, tomato, sweet pepper, chili pepper, habanero pepper, ground cherry, potato, lettuce, peanut, soybean, and cotton.

It is well known that plants, especially cultivated crops, contain different varieties, lines, cultivars, germplasms, genotypes, etc. In certain embodiments of the present invention, the TSWV vectors effectively infect different varieties within the same species and express nucleic acid modification enzymes, producing targeted modification efficiencies in target genes at similar frequencies.

In certain embodiments, ten peanut varieties, including the varieties Huayu 25, Huayu 32, Huayu 39, Huayu 48, Huayu 9616, Fengyou 68, Honghua 1, Dabaisha, Sili Hong, and Shuofeng 518, are infected with the TSWV vectors to produce targeted modifications in the target sequences at similar frequencies.

In certain embodiments, ten sweet pepper varieties, including Zhoula 2, Zhoula 3, Zhoula 4, Fuxiang Xiuli, Bola 5, Bola 7, Bola 15, Xingshu 201, Xingshu 301, and Xingshu Guiyan varieties, are infected with the TSWV vectors to produce targeted modifications in the target sequences at similar frequencies.

It is generally believed that virus infection of host plants is less dependent on the plant genotypes. It is conceivable that TSWV vectors could effectively infect different varieties, lines, cultivars, germplasms, or genotypes of other host plants to modify their genetic materials.

### Methods for generating genetically modified plants

Further, the present invention discloses methods for obtaining plants from the plant cells with modified genetic material and selecting plants with modified genetic material.

The plant cells in the present invention can be cells in intact plants infected with viruses or isolated plant cells, such as callus tissues, suspension cells, or protoplasts.

Methods for obtaining plant organs or individual plants from plant cells include propagation by seed, such as plant cells being naturally or artificially induced to develop into adult embryonic stem cells, which in turn develop into germ cells that produce seeds after sexual reproduction. Plants can also be obtained from plant cells through micropropagation, i.e., asexual propagation of plant cells *in vitro* using plant tissue culture techniques.

In certain embodiments, the method for obtaining plants with modified genetic material is micropropagation. The method comprises using virus-infected plant cells or tissues as explants for tissue culture and plant regeneration on a selection marker-free medium, i.e., no selection agents (e.g., antibiotics, herbicides, etc.) are used in the tissue culture process. Further, the method comprises selecting plants with modified genetic material from the regenerated plants, wherein the selection process does not require selection by any selection marker. Instead, the selection is conducted by screening the plants using restriction endonuclease analysis or DNA sequencing to analyze target gene sequences.

The present invention further compares the effects of TSWV viral vectors with or without the *NSs* gene on plant cell differentiation and regeneration and finds that *NSs* inhibits plant regeneration. Thus, in some preferred embodiments of the present invention, plant cells infected with TSWV vectors deficient in functional NSs are used to modify the plants ' genetic material.

Plant tissue culture and regeneration methods are known to those skilled in the art, and the requirements for medium composition and growth hormone compositions vary among plant cells.

In certain embodiments, the TSWV vector-infected N. *benthamiana* leaf cells were used for tissue culture to obtain plants with modified genetic material and without the insertion of exogenous nucleic acid.

In certain embodiments, the TSWV vector-infected tobacco leaf cells were used for tissue culture to obtain plants with modified genetic material and without the insertion of exogenous nucleic acid.

In certain embodiments, the TSWV vector-infected tomato leaf cells were used for tissue culture to obtain plants with modified genetic material and without the insertion of exogenous nucleic acid.

When virus-infected somatic cell tissues were used for tissue culture, the virus often continued to be present in the regenerated plants. Persistently viral infections may result in continuous expression of nucleic acid modifying enzymes in the regenerated plants, which leads to non-heritable editing in the somatic cells of the regenerated plants or increased off-target modifications. In addition, persistent virus infection can interfere with plant physiology and phenotype or even adversely affect plant fertility and seed setting. Therefore, removing viral vectors during plant regeneration can avoid these adverse effects.

Certain compounds can effectively inhibit human or animal RNA virus replication *in vivo* and *in vitro.* These compounds include various nucleoside or nucleotide analogs, such as ribavirin, favipiravir, remdesivir, 6-azauridine, oxypurinol, gemcitabine, galidesivir (BCV4430), mizoribine, monupiravir, sofosbuvir, tenofovir, etc. (Geraghty et al., Viruses, 2021, 13: 667; Borbone et al, Molecules, 2021, 26: 986). These nucleoside or nucleotide analogs target the viral RdRP and are mis-incorporated by RdRp into the nascent viral RNA during viral replication, leading to the termination of viral RNA replication or causing the accumulation of numerous deleterious mutations in the viral genomes. Different compounds have significantly different affinities for RdRP of different animal RNA viruses, and their antiviral effects also vary. The present inventors unexpectedly found that when using TSWV-infected somatic cell tissues for tissue culture, the addition of certain nucleoside or nucleotide analogs within the medium greatly facilitated virus clearance from the regenerated plants and significantly increased the proportion of the regenerated plants with genetic modifications.

In certain embodiments, when TSWV CRISPR/Cas12a vector-infected leaf tissues are cultured in a medium containing 40 mg/L ribavirin, 100% of the regenerated plants were free of virus. In contrast, 28.9% of plants have tetra-allelic mutations at the crPDS1 target site. In comparison, only 4.4% of the regenerated plants in the mock were virus-free, and 15.6% contained bi-allelic mutations.

In certain embodiments, when TSWV CRISPR/Cas12a vector-infected leaf tissues are cultured in a medium containing 40 mg/L of favipiravir, 66.7% of the regenerated plants were free of virus.

In certain embodiments, when TSWV CRISPR/Cas12a vector-infected leaf tissues were cultured in a medium containing 1.2 mg/L of remdesivir, 15.6% of the regenerated plants were free of the virus.

In another aspect, the present invention also provides recombinant TSWV particles, as described above, wherein the virus particles comprise chimeric genomic segments containing a heterologous sequence encoding a nucleic acidmodifying enzyme . The recombinant TSWV virus particles can be introduced into isolated plant cells or specific organs or tissues of intact plants, such as leaves, leaf veins, roots, etc., through conventional methods in the art, such as mechanical inoculation, pressurized spraying, etc. Virus particles infect plant cells and express the nucleic acid modifying enzymes that cleave or modify plant genomic target sequences and induce the modifications of genetic material.

In another aspect, the present invention further provides kits that contain the viral vector elements mentioned in the above methods and compositions. The kit may comprise a user manual and viral vector system comprising: a) a nucleic acid construct comprising recombinant TSWV S, M, and L chimeric genomic sequences, wherein the S segment comprises one or more heterologous nucleic acid sequences encoding a CRISPR guide RNA component, the M segment comprises one or more heterologous nucleic acid sequences encoding a Cas nucleic acid modifying enzyme component, and the L segment comprises codon-optimized viral RdRp sequence (SEQ ID NO: 5) ; b) helper expression vectors encoding one or more viral suppressors of RNA silencing. The viral vector is introduced into a plant cell to generate a recombinant virus that transiently expresses CRISPR/Cas nucleic acid modifying enzymes that modify the genetic material in the cell. The viral vector reagents may be provided individually or in combination and may be placed in a suitable container, such as a tube, bottle, or pill bottle.

Compared to the prior art, the benefit offered by the viral delivery system-based plant genome editing technology disclosed in the present invention include:
1) the tospovirus vector has a large cargo capacity, is genetically stable, and can be used to deliver the large Cas nuclease without the need for transgenic expression of the nuclease component in the plant cells to be modified;
2) The viral vectors are fully infectious in plants and can deliver and express nucleases in multiple organs and tissues in intact plants (*in planta);*
3) The virus has a wide host range, and a variety of important crop plants can be infected through mechanical inoculation using recombinant virus particles;
4) The viral vector is a mutant form lacking the envelope, which is non-insect-transmissible and biocontainable;
5) Abundant expression of the sequence-specific nucleases driven by viral replication boosts gene editing efficiency;
6) The virus replication process does not involve a DNA phase and minimizes the risk of DNA integration, and the obtained gene-edited plants do not contain exogenous genetic material;
7) Viral vector-mediated transient expression is less dependent on the host genotypes and can be applied to different crop varieties. In contrast, traditional *Agrobacterium-mediated* stable transformation is often only effective in specific plant species or varieties;
8) Viral vectors can be efficiently eliminated by antiviral compounds during plant regeneration to obtain virus-free plant mutants.

### Embodiments

The present invention is described in further detail hereinafter in connection with the embodiments, which are used only to illustrate the present invention and should not be regarded as limiting the scope of the present invention. Any modifications or improvements made on the basis of the present invention without deviating from the present invention belong to the scope of protection of the present invention.

The experimental methods used in the following embodiments are conventional methods, and the reagents and materials used can be obtained from commercial sources unless otherwise specified.

The recombinant TSWV vectors are described using the TSWV-X-Y nomenclature (or V-X-Y for short) in the following embodiments, where X refers to the heterologous nucleic acid sequence carried by the M genome segment, and Y refers to the heterologous nucleic acid sequence inserted in the S genome. When a genome segment contains two tandem heterologous nucleic acid elements, these two elements are joined by a semicolon, e.g., TSWV-X-Y: Z. For example, TSWV-Cas12a-crPDS1:GFP is a recombinant TSWV vector in which the M segment contains the heterologous Cas12a sequence and the S segment contains the heterologous crRNA and GFP sequences. The pS/M/L_{(-/+)X} is used to describe the constructs containing the TSWV S, M, or L genomic cDNA sequences, in which the (-) indicates genomic cDNA inserted downstream of the promoter in the construct and to produce the negative-sense viral RNA, the (+) indicates antigenomic cDNA inserted downstream of the promoter in the construct to produce transcription to produce the positive-sense viral RNA, and the X indicates the heterologous nucleic acid sequence carried in the genome . For example, pM_{(+) cas12a} is a TSWVM construct containing the antigenomic cDNA sequence and a heterologous Cas12a sequence.

The *Agrobacterium* binary vector pCB301-2µ-HDV (FIG. 3A) is disclosed in the paper "Sun K, Zhao D, Liu Y, Huang C, Zhang W, LiZ. Rapid construction of complex plant RNA virus infectious cDNA clones for agroinfection using a yeast-E. coli-Agrobacterium shuttle vector. Viruses, 2017, 9 (11) . pii: E332".

The *Agrobacterium* binary vector pGD (FIG. 3B), as well as the pGD-GFP and pGD-RFP expression vectors, are described in "Goodin MM, Dietzgen RG, Schichnes D, et al. pGD vectors: versatile tools for the expression of green and red fluorescent protein fusions in agroinfiltrated plant leaves. Plant J, 2002, 31: 375-83".

The viral suppressor of RNA silencing (VSR) expression vectors, pGD-HcPro, pGD-p19, and pGD-γb, are described in "Ganesan U, Bragg JN, Deng M, et al. minireplicon: a step toward reverse genetic analysis of plant negative-strand RNA viruses. J Virol. 2013, 87: 10598-10611".

The N. *benthamiana* codon-optimized SpCas9 sequence is described in "Yin K, Han T, Liu G, et al., Geminivirus-based guide RNA delivery system for CRISPR/Cas9 mediated plant genome editing. Scientific Reports, 2015, 5: 14926".

The plasmid pYQ230 containing the rice codon-optimized LbCas12a sequence is described in "Tang, X., Lowder, L.G., Zhang, T., et al. A CRISPR-Cpf1 system for efficient genome editing and transcriptional repression in plants. Nature Plants, 2017, 3:17018".

*Nicotiana benthamiana* LAB accession and the 16C transgenic line are described in "Ruiz MT, Voinnet O, Baulcombe DC. Initiation and maintenance of virus-induced gene silencing. Plant Cell, 1998, 10:937-46". Tobacco (*N. tabacum* cv. K326), tomato (*Solanum lycopersicum* cv. Hongbaoshi), potato (S. *tuberosum),* sweet pepper (*Capsicum annuum*)*,* chili pepper (C. *frutescens),* habanero pepper (C. *chinense),* ground cherry (*Physalis grisea),* lettuce (*Lactuca sativa*)*,* land cotton (*Gossypium hirsutum* cv. Junmian No. 1), peanut (*Arachis hypogaea*), and soybean (*Glycine max* cv. William 82) were the regular plant materials available to the public from the Institute of Biotechnology, Zhejiang University.

The target gene sequences of Solanaceae species (N. *benthamiana,* tobacco, tomato, potato, and pepper) were retrieved from the Sol Genomics Network (SGN)e (https://solgenomics.net/), while the target gene sequences of legumes (peanut and soybean) were available from the Legume Genome Information System LIS database (https://legumeinfo.org/). The Cas12a target sequences were designed using the CRISPR RGEN Tools (http://www.rgenome.net/) online software, and the target sites for Cas9 and Cas9-derived base editors were designed by using the CRISPR-P (http://cbi.hzau.edu.cn/cgi-bin/CRISPR) online software.

### Example 1: Construction of TSWV dual reporter vector

### Total RNA extraction from TSWV-infected plant samples.

0.1 g of TSWV-infected tomato plants grown in the laboratory is collected and quickly ground into powder in liquid nitrogen. Total RNA is extracted from the leaf samples using Ttizol reagent (Invitrogen), dissolved in 50 µL of DEPC-treated pure water after precipitation with isopropanol. The extracted RNA is used immediately for subsequent experiments or stored in -80°C refrigerator in aliquots.

### Reverse transcription to obtain viral genomic cDNA

Procedure according to TaKaRa AMV reverse transcriptase instructions as follows:
1) Mix the following reagent in the reaction:

| | |
|---|---|
| Total plant RNA | 1 µg |
| 5×AMV buffer | 4 µL |
| 0.01 mM specific primers | 1 µL |
| 10 mM dNTPs | 1 µL |
| RNase Inhibitor | 0.5 µL |
| AMV reverse transcriptase 0.5 U | 0.5 µL |
| DEPC-dd H₂O | To a total volume of 20 µL |

The primers used for reverse transcription were as follows:

| Primer | Sequence (5'-to-3') | Describe |
|---|---|---|
| L | AGAGCAATCAGGTAACAACGATTTTAAG (SEQ ID NO: 28) | Reverse transcription to obtain L genome cDNA |
| 3'UTR/F | | |
| M | AGAGCAATCAGTGCAAACAAAAACC (SEQ ID NO: 29) | Reverse transcription to obtainM genome cDNA |
| 3'UTR/F | | |
| S | AGAGCAATTGTGTCAATTTTATTCAAACC (SEQ ID NO: 30) | Reverse transcription to obtain S genome cDNA |
| 3'UTR/F | | |

2) The following reaction steps are performed in the PCR instrument: 42°C for 1 h; 50°C for 30 min; 95°C for 5 min; 4°C for 10 min; the products can be directly diluted and used as a PCR template or stored at -20°C.

### Construction of recombinant TSWV vector and binary vectors for transient expression viral proteins

The tri-segmented, negative-stranded TSWV RNA genome consists of the large (L), medium (M), and small (S) RNAs (FIG. 2), and the sequences are shown in SEQ ID NO: 1, 2 and 3. Binary constructs based on the pCB301-2µ-HDV backbone were used to recover recombinant TSWV vectors. As shown in FIG. 3A, the pCB301-2µ-HDV was digested with the StuI/SmaI restriction enzymes to obtain a blunt-ended linearized vector, and after the insertion of the exogenous viral sequence, the 35S promoter (SEQ IDNO: 9) directs the transcription of the inserted sequence with a precise 5' end, while a hepatitis delta virus ribozyme placed immediately downstream of viral cDNA generates an exact viral 3' terminal. The binary vector pGD was used to construct the transient expression vector of the viral proteins (FIG. 3B) . The primer sequences involved in the vector construction of the present invention are listed in Table 1. Primers and gene fragments were synthesized at Genscript (Nanjing, China) .

### 1) pL₍₊₎ and pL_{(+) opt} vector construction

pL₍₊₎ is a construct containing the TSWV L antigenomic cDNA (FIG. 4A). The L cDNA obtained from the above reverse transcription was used as a template to amplify the full-length L antigenomic cDNA (SEQ ID NO: 3) using the specific primers L(+) /F and L(+) /R (Table 1) . Linearized plasmid DNA fragments were obtained by digestion of pCB301-2µ-HDV vector using restriction endonucleases StuI and *Sma*I*,* and co-transformed with L cDNA segments to obtain circular plasmids through cloning method, using on yeast homologous recombination-based cloning as described by Sun et al., (Viruses, 2017, 9. pii: E332.). The plasmids were extracted from yeast colonies grown in tryptophan-deficient yeast media, and after the transformation of *E. coli,* the purified plasmids were verified by enzymatic cleavage and further verified by Sanger sequencing.

pL (+) opt is a construct derived from pL₍₊₎, in which the RdRp coding sequence (SEQ ID NO: 4) is replaced by a codon-optimized sequence (SEQ ID NO: 5) (FIG. 4A) . We optimized the RdRp sequence based on the preference of N. *benthamiana* codons. We used the Alternative Slice Site Predictor (ASSP) (http://wangcomputing.com/assp/) to predict potential intron-exon splicing sites and disrupted the predicted splicing sites through codon optimization. The codon-optimized RdRp sequence (SEQ ID NO: 5) was chemically synthesized. The pL₍₊₎ plasmid was used as a template in PCR to obtain a vector backbone fragment excluding the RdRp coding sequence using the primers Lopt 5' UTR/F and Lopt 3' UTR/R. The recombinant clone pL₍₊₎ₒₚₜ was assembled through transforming yeast cells with the codon-optimized RdRp fragment and the amplified vector backbone fragment.

### 2) Construction of pM(_), pM_{(-) RFP}, and pM_{(+) RFP}

pM₍₋₎ is a construct containing the TSWVM genomic cDNA (FIG. 4B) . The M genomic cDNA (SEQ ID NO: 2) was amplified from viral cDNAs mentioned above using the primer pairs M 5'UTR/F and M 3'UTR/R. The primers contain a 15 bp sequence homologous to each end of the StuI and SmaI linearized pCB301-2µ-HDV vector. The pM₍₋₎ plasmid was obtained by inserting the M full-length genomic cDNA fragment into the linearized pCB301-2µ-HDV using the ClonExpress MultiS One Step Cloning reagents.

pM_{(-)RFP} is a construct derived from pM₍₋₎ in which the GP gene is replaced by the RFP (FIG. 4B). To this end, we amplified from pM₍₋₎ the ScaI-IGR fragment containing the complete M gene intergenic region (IGR), the 3'UTR-PmeI fragment spanning from the 3'UTR to the PmeI restriction site, using primer pairs M IGR/ScaI/F and M IGR/R, RFP/M 3'UTR/F and pCB-PmeI/R, respectively. In addition, the RFP fragment was amplified from the pGD-RFP plasmid using primers RFP/F and RFP/R, and the vector fragment was recovered after digestion of the pM(_) vector with ScaI and PmeI. The above four fragments were ligated by an In-Fusion cloning reagent to obtain pM_{(-)RFP}.

The pM_{(+) RFP} has the same insertion sequence as the pM_{(-) RFP}, but the sequence is inserted into the vector in the opposite direction to produce the M antigenomic RNA (FIG. 4B). Viral M genomic cDNA containing RFP was amplified using the primers 35S/M 3'UTR/F and HDV/M 5'UTR/R and the pM_{(-)RFP} plamid as a template and ligated to SmaI/StuI digested pCB301-2µ-HDV via In-Fusion cloning.

### 3) pS₍₊₎ and pS_{(+) GFP} vector construction

pS₍₊₎ is a construct containing the TSWV S antigenomic cDNA (FIG. 4C). The full-length antigenomic S cDNA (SEQ ID NO: 1) was amplified from viral cDNAs using the primer pairs 35S/S 3'UTR/F and HDV/S 5'UTR/R and then assembled with the StuI/SmaI-double digested pCB301 vector fragment through In-Fusion cloning.

The pS_{(+)GFP} is a construct derived from pS₍₊₎ in which the *NSs* gene is replaced by the *GFP* (FIG. 4C). The DNA fragment excluding the NSs sequence was amplified from pS₍₊₎ using the primers S 5'UTR/F and S IGR/R, the GFP fragment was amplified using primers GFP/5'UTR/F and GFP/IGR/R from the pGD-GFP plasmid, and the above two fragments were seamlessly assembled by In-Fusion cloning reagents to obtain pS_{(+)GFP}.

### 4) Construction of the expression vectors pGD-NSs andpGD-NSm

Using the S and M cDNAs as templates, the coding regions of NSs (SEQ ID NO: 7) and NSm (SEQ ID NO: 8) were amplified using primer pairs BamHI/NSs/F, and SalI/NSs/R, BamHI/NSm/F and SalI/NSm/, respectively. The above PCR products were inserted into the pGD vectors through BamHI and SalI digestion and ligation to obtain the protein expression vectors pGD-NSs and pGD-NSm, respectively.

### Example 2: TSWV vector expressing two fluorescent reporter proteins

### Agroinoculation of N. benthamiana

Recombinant binary plasmids were introduced into the *Agrobacterium tumefaciens* EHA105 strain by electroporation. Single A. *tumefaciens* colony was grown overnight at 28°C in Yeast Extract and Peptone (YEP) culture medium containing 50mg l⁻¹ kanamycin and 50 mg l⁻¹ rifampicin. The overnight cultures were transferred to a fresh YEP culture medium and allowed to grow to about 0.8-1.0 optical density (OD₆₀₀). Cultures were collected by centrifugation and resuspended in infiltration buffer (10 mM MES, 10 mM MgCl₂, and 100 µM Acetosyringone, pH 5.6) at 1.0 OD₆₀₀, followed by incubation for 2-4 h at room temperature.

For analysing the effect of M genome polarity (positive or negative sense) and co-expression of NSm proteins on recombinant TSWV rescue, *N. benthamiana* leaf tissues were infiltrated with *Agrobacterium* mixtures harboring various combinations of recombinant viral constructs containing two reporter genes as listed below.
Group 1: pL₍₊₎ₒₚₜ, pM_{(-)RFP}, pS_{(+)GFP}, pGD-p19, pGD-HcPro, pGD-γb, pGD-NSs;
Group 2: pL₍₊₎ₒₚₜ, pM_{(+)RFP}, pS_{(+)GFP}, pGD-p19, pGD-HcPro, pGD-γb, pGD-NSs;
Group 3: pL₍₊₎ₒₚₜ, pM_{(+)RFP}, pS_{(+)GFP}, pGD-NSm, pGD-p19, pGD-HcPro, pGD-γb, pGD-NSs.

In the mixtures, the final concentration of the bacterial solution containing pL, pM, and pS was adjusted to OD₆₀₀ =0.1. *Agrobacterium* cultures harboring the pGD plasmids for expressing three VSR (barley stripe mosaic virus γb, tomato bushy stunt virus p19, tobacco etch virus P1/HC-Pro) were mixed in equal proportions and added to the mixtures at a final OD₆₀₀ of 0.05 to minimize RNA silencing. *Agrobacterium* culture harboring the pGD-NSm expression vector was adjusted to a final concentration of OD₆₀₀=0.1. The mixed cultures were infiltrated into the abaxial surfaces of fully expanded N. *benthamiana* leaves at approximately the 6-leaf stage with a 1-ml needleless syringe, as shown in FIG. 5A. The infiltrated plants were grown in a greenhouse and monitored for symptom appearance.

### Reporter gene expression analysis

Expression of GFP and RFP was visible five days after agroinoculation in all three groups of inoculated leaves of *N. benthamiana.* Compared with RFP, the area of leaf tissue with GFP expression is smaller, especially in group 3 (FIG. 5B).

Green and red fluorescence began to appear in the upper non-inoculated leaves 7 days after agroinoculation in both Group 1 and Group 2, and there was widespread fluorescence spread in the systemic leaves at day 10. In contrast, fluorescence in the upper non-inoculated leaves in Group 3 was delayed, and scattered red and green fluorescence cell clusters were seen at day 10 (FIG. 5B).

By 6-8 days after agroinoculation, all inoculated plants in Groups 1 and 2 successively developed systemic symptoms, such as young leaves curling and chlorosis, while systemic infections in Group 3 were delayed for 2-3 days. Plants were photographed 5 days post systemic infection under visible light and long-wavelength ultraviolet (UV) illumination (FIG. 5C). The total protein of the upper leaves was extracted and analyzed by western blot using TSWV N-specific antibody, GFP polyclonal antibody, and mCherry polyclonal antibody, respectively. The TSWV N, GFP, and RFP proteins were detected in the total protein of all three experimental groups but not in the mock control group that lacks the L genomic plasmid (FIG. 5D).

The conclusions from the above experiments are: i) the L and S antigenomic RNA transcripts produced from the viral constructs can direct the translation of the RdRp and N proteins sufficient for genome encapsidation and replication; ii) for the M genome, both genomic cDNA vector (Group 1) and antigenomic cDNA vectors (Groups 2 and 3) can support recombinant virus rescue; iii) Providing additional viral movement protein does not facilitate but in fact delayed generation of recombinant virus (compare Group 2 with 3) ; iv) TSWV GP and *NSs* can be replaced by foreign genes without compromising plant systemic infection.

### Example 3: Role of RNA silencing suppressor in recombinant virus rescue

Four binary vectors for expression of viral suppressors of RNA silencing (VSRs), i.e., pGD-p19, pGD-HcPro, pGD-γb, and pGD-NSs, were included in the agrobacterial mixtures in Example 2. This implementation further tested the effect of expressing single or multiple combinations of VSRs on recombinant TSWV rescue. In the *Agrobacterium*mixtures*,* the final concentrations of the bacterial solution containing pL₍₊₎ₒₚₜ, pM_{(-)RFP}, and pS_{(+)GFP} plasmids were adjusted to OD₆₀₀=0.1, respectively, and the final concentrations of the bacterial solution of each of the following VSRs were adjusted to OD₆₀₀=0.05.
Experimental group 1: pGD-p19, pGD-HcPro, pGD-γb, pGD-NSs (pGD-VSRs)
Experimental group 2: pGD-p19
Experimental group 3: pGD-HcPro
Experimental group 4: pGD-γb
Experimental group 5: pGD-NSs
Experimental group 6: pGD-p19, pGD-HcPro
Experimental group 7: pGD (empty vector control)

Eight days after agroinoculation, about 16-18 plants of 20 inoculated plants showed systemic symptoms in experimental Group 1 (VSRs), Group 2 (p19), and Group 6 (p19 + HcPro), i.e., the rescue efficiencies were 80-90%. There were 7, 12, and 2 plants that developed systemic symptoms in experimental Group 3 (HcPro), Group 4 (γb), and Group 5 (NSs), respectively. According to the time and proportion of plants that showed systemic symptoms, the infection efficiencies from highest to lowest in the experiments were Group 2, 1, 6, 4, 3, and 5. Until 20 days after inoculation, none of the 20 infiltrated plants in experimental Group 7 showed systemic group (FIG. 6). The above results showed that co-expression of silencing suppressors promoted the rescue of recombinant TSWV vectors in different degrees, with p19 being the most effective and NSs being the least effective.

### Example 4: Design and construction of the TSWV-Cas12a-crRNA vectors

The CRISPR/Cas12a system contains the Cas12a protein and a CRISPR guide RNA (crRNA) that binds to the target. As shown in FIG. 7, the strategy for constructing the TSWV-Cas12a-crRNA vectors is as follows: i) the genomic cDNA vector pM_{(-)Cas12a} and the anti-genomic cDNA vector pM_{(+)Cas12a} were constructed by replacing the GP sequence in the TSWV M genome with the sequence encoding Cas12a; ii) the crRNA sequence was inserted into the pS_{(+)GFP} vector immediately upstream of the GFP start codon to construct the pS_{(+)crRNA:GFP} vector. The structure of the inserted crRNA sequence is "direct repeat - protospacer - direct repeat", so the two direct repeat regions are designed to allow the Cas12a protein to process the primary crRNA transcript to release the exact crRNA sequence. The specific construction steps are detailed below in detail.

### 1) Construction of the pM_{(-)Cas12a} and pM_{(+)Cas12a} vectors

The plasmid pYQ230 containing the rice codon-optimized Cas12a sequence flanked by a nuclear localization signal and 3× Flag antigen epitope sequence (SEQ ID NO: 10) was used as a template, and the 3× Flag-NLS-Cas12a-NLS fragment was amplified using the primers M UTR/Cas12a/F and Cas12a/R (Table 1). The M vector fragment, excluding the GP sequence, was amplified from the pM(-) plasmid using primers M 3'UTR/F and Cas12a/M IGR/R. The two fragments described above were ligated using an In-Fusion cloning reagent to generate the pM_{(-)Cas12a} vector.

Using pM_{(-)Cas12a} as a template, the recombinant M genomic cDNA fragment was amplified using the primers 35S/M 3'UTR/F and HDV/M 5'UTR/R and ligated with the StuI and SmaI digestedpCB301-2p-HDV vector fragment by In-Fusion cloning method to generate the M antigenomic cDNA vector pM_{(+)Cas12a}.

### 2) Construction of the pS_{(+)crRNA:GFP} series vectors

To facilitate crRNA insertion, a SpeI digest site was inserted upstream of the GFP start codon of the pS_{(+)GFP} plasmid. The fragment PacI-IGR-GFP and fragment SpeI-5'UTR-HDV-NOS fragment were amplified from the pS_{(+)GFR} vector with primer pairs SpeI/GFP/F and IGRPacI/R, NOS PvuI/FandS 5'UTR/R, respectively. The above fragments were inserted into a PacI/PvuI digested pS_{(+)GFP} vector to obtain pS_{(+) SpeI:GFP} intermediate vector with the introduced SpeI restriction site.

According to the CRISPR RGEN Tools (http://www.rgenome.net/), we selected the crPDS1 target site in the N. *benthamiana phytoene desaturase* gene *(NbPDS)* and tobacco *NtPDS* (5' -**TTTC**TGCCGTTAATTTGAGAGTCCAAG-3 ') (SEQID NO: 12) (bold letters are PAM same below), the crPDS2 target site in the tomato *SLPDS* (5'-**TTTC**TGCTGTTAACTTGAGAGTCCAAG-3') (SEQ ID NO: 13), the crPDS3 target site in the pepper *CaPDS* (5'-**TTTC**TGCTGTCAACTTGAGAGTCCAAG-3') (SEQIDNO: 14), thecrPDS4 target site in the peanut *AhPDS* loci and soybean *GmPDS* loci (5'-**TTTG**ACAGAAAACTGAAGAACACATAT-3') (SEQ ID NO: 15) . The crRNA sequences (as shown in SEQ ID NO: 18-21, respectively) that can target the above target sites have the structure of "direct repeat - protospacer - direct repeat" and contain SpeI cleavage sites on both sides. The above crRNA sequences were inserted into the SpeI site of pS_{(+)SpeI:GFP} vector to generate pS_{(+)crPDS1:GFP}, ps_{(+)crPDS2:GFPr}, pS _{(+) crPDS3: GFP}, and pS_{(+)crPDS4:GFP} vectors, respectively.

### Example 5: Recombinant TSWV-Cas12a-crRNA rescue system

Example 2 established the conditions for rescuing the TSWV-RFP-GFP vector from cloned cDNAs and found that both M genomic and anti-genomic cDNA constructs could be used to successfully recover the recombinant virus. A preliminary experiment showed that the insertion of Cas12a gene (nearly 4 kb) in the M genome significantly affected the rescue of the TSWV-Cas12a-crRNA vector. Therefore, the present embodiment systematically investigates the experimental conditions, including the polarity of the M genome (pM_{(-)Cas12a} or pM_{(+)Cas12a}) and the relative ratios of each *Agrobacterium*mixture containing each genomic component. *Agrobacterium* mixtures of different concentrations (OD₆₀₀) were formulated according to the eight combinations shown below, each containing L, M, and S cDNA constructs and four VSRs helper vectors, i.e., pGD-p19+ pGD-HcPro+ pGD-γb+ pGD-NSs. Groups 1-4 have the M genomic cDNA construct pM_{(-)Cas12a}, Groups 5-8 contain the M antigenomic cDNA construct pM_{(+)Cas12a}, and Groups 5-7 have the pGD-NSm expression vector.

| OD₆₀₀ | pL₍₊₎ₒₚₜ | pM_{(-)Cas12a} | pM_{(+)Cas12a} | pS_{(+)crPDS1:GFP} | VSRs | pGD-NSm | No. infected/ inoculated plants (%) |
|---|---|---|---|---|---|---|---|
| Group 1 | 0.1 | 0.2 | - | 0.2 | 0.05 | 0 | 0% (0/50) |
| Group 2 | 0.1 | 0.2 | - | 0.1 | 0.05 | 0 | 0 % (0/50) |
| Group 3 | 0.1 | 0.2 | - | 0.05 | 0.05 | 0 | 0% (0/50) |
| Group 4 | 0.1 | 0.5 | - | 0.05 | 0.05 | 0 | 4% (2/50) |
| Group 5 | 0.1 | - | 0.2 | 0.2 | 0.05 | 0.05 | 10% (5/50) |
| Group 6 | 0.1 | - | 0.2 | 0.1 | 0.05 | 0.05 | 28% (11/40) |
| Group 7 | 0.1 | - | 0.5 | 0.05 | 0.05 | 0.05 | 60% (24/40) |
| Group 8 | 0.1 | - | 0.5 | 0.05 | 0.05 | 0 | 83% (33/40) |

The above combinations of *Agrobacterium* mixtures were infiltrated into leaves of N. *benthamiana* for recovery of recombinant virus. GFP fluorescence was observed to widely distribute in the inoculated leaves in all groups by 5 days post inoculation (dpi) (FIG. 8A). The inoculated plants from some of the groups started to show systemic symptoms at 9 dpi, but none of plants in Groups 1, 2 and 3 showed recombinant virus systemic infection, and only 2 of the 50 (4%) inoculated plants in Group 4 eventually developed systemic symptoms. As shown in the table above, the infection rates in Groups 5-8 expressing the M antigenomic RNA were 10%, 28%, 60%, and 83%, significantly higher than those in Groups 1-4 expressing the M genomic RNA. The ratios of *Agrobacterium* cultures harboring the pM- and pS-derived plasmids in Groups 5-8 were 1:1, 2:1, 10:1, and 10:1, respectively, indicating that efficient recombinant virus rescue required relatively high concentrations of M antigenomic cDNA plasmids.

The systemically infected plants in the above groups exhibited typical TSWV symptoms (FIG. 8B) . Total protein samples from the upper leaves of the two systemically infected plants in Group 4 and from any two infected plants in Group 8 were extracted and analyzed by western blot using TSWV N polyclonal antibody, GFP polyclonal antibody, and Flag monoclonal antibody (to detect Cas12a protein), respectively. The expression of viral N and GFP proteins was detected in these samples . However, two samples in Group 8 had Cas12a protein bands of expected size. In contrast, both samples in Group 4 had no Cas12 expression (FIG. 8C). To further clarify the reason for the absence of Cas12a expression in Group 4 plants, total RNA samples from the leaves of the above four plants were extracted. Reverse transcription PCR (RT-PCR) was performed using the NSm-specific primersNSm/F (5'-ATGTTGACTTTTTTTTTGGTAATAAGGG-3') (SEQIDNO: 24) and NSm/R (5'-CTATATTTCATCAAAAGATAACTGAGC-3') (SEQ ID NO: 25), and the primers IGR/F (5'-TACTTTTAATACCACTTATTTAAGCTT-3') (SEQ ID NO: 26) and M UTR/R (5'-AGAGAGCAATCAGTGCAAACAAAAAC-3') (SEQ ID NO: 27) flanking the *Cas12a* sequence, to detect the *NSm* and *Cas12a* fragment in the M genome of the recombinant virus in the above four samples. The presence of the *NSm* gene of the expected size was detected in all four samples, and an expected Cas12a band of ~4.3 kb in size was detected in two plant samples in Group 8, but only a small band of about 0.5-0.7 kb was detected in two samples of Group 4 (FIG. 8D) . The above small bands were cloned and analyzed by Sanger sequencing, which showed that most of the *Cas12a* insert was lost in the recombinant virus in Group 4, and only the 5' and 3' partial sequences of *Cas12a* gene were retained (FIG. 8E).

From the above results, it is clear that unlike the TSWV-RFP-GFP vector rescue system, the M genomic cDNA vector pM_{(-)Cas12a} is not suitable to rescue TSWV-Cas12a-crRNA because it tends to lose most of the *Cas12a* sequence. In contrast, the M antigenomic cDNA vector pM_{(+) Cas12a} can be used to generate the intact TSWV-Cas12a-crRNA vector. The recombinant virus was most efficiently rescued when the ratio of *Agrobacterium* carrying pL, pM- and pS- was 2:10:1 (Group 8). In contrast, transient expression of the NSm protein did not contribute to the virus rescue (compare Groups 7 and 8). Therefore, the combination and concentration of *Agrobacterium* in Group 8 was used for recombinant virus rescue in all relevant embodiments described below.

### Example 6: Editing N. benthamiana NbPDS with TSWV-Cas12a-crRNA

The crPDS1 sequence (5'-**TTTC**TGCCGTTAATTTGAGAGTCCAAG-3'; SEQ ID NO: 12) (bold letters indicate PAM and the underlined bases refer to HinfI cleavage site) is conserved in both homoeologs in the allotetraploid genome of N. *benthamiana* (FIG. 9A) . As described in Example 4, the pS_{(+)crPDS1:GFP} vector contains a crRNA sequence that can target the crPDS1 site. Agrobacterial cultures were mixed according to Group 8 in Example 5, while the pS_{(+)GFP} without crRNA was used as a negative control, as detailed below,
Experimental group: pL₍₊₎ₒₚₜ, pM_{(+)Cas12a}, pS_{(+)crPDS1:GFP}, pGD-p19+ pGD-HcPro+ pGD-γb+ pGD-NSs
Control group: pL₍₊₎ₒₚₜ, pM_{(+)Cas12a}, pS_{(+)GFP}, pGD-p19+ pGD-HcPro+ pGD-γb+ pGD-NSs
The two groups of *Agrobacterium* mixtures were inoculated into *N. benthamiana* leaves and both produced TSWV symptoms such as leaf curling and crinkling as well as faint green mottling about 10 dpi and expressed GFP fluorescence in the upper uninoculated leaves (FIG. 9B) . Total protein was extracted from upper uninoculated leaves, and TSWV N, GFP, and Cas12a proteins could be detected using specific antibodies, respectively (FIG. 9C) .

Polymerase chain reaction/restriction endonuclease protection (PCR/RE) was used to detect gene editing, and if editing occurred at this target site, the restriction endonuclease recognition site that partially overlaps with the Cas12a cutting site is disrupted, and the target DNA PCR fragment is not susceptible to digestion by the corresponding endonuclease. The specific methods are described below:
1) Extraction of genomic DNA from upper uninoculated leaf tissues using PureLink^{®}Quick Gel Extraction Kit (Invitrogen).
2) PCR amplification of the genomic DNA fragment using specific primers flanking the target site in Table 2.
3) Digestion of PCR products using specific restriction endonucleases whose recognition site partially overlaps with the Cas12a cleveage site using the following general digestion system:

| | |
|---|---|
| 10×Fastdigest Buffer | 2 µl |
| restriction endonuclease | 1 µl |
| PCR product | 0.2 µg |
| dd H₂O | Fill up to 20 µl |

PCR/RE analysis showed that the mutation frequency in the *NbPDS* target locus was approximately 50%, whereas no mutation was found in Control group plants (FIG. 9D) . The above results indicate that the TSWV-Cas12a delivery system can systematically deliver the Cas12a/crRNA complex to N. *benthamiana* and produce high-efficiency editing at the target site.

### Example 7: Mechanical transmission and genetic stability of TSWV-Cas12a crRNA

Example 6 used agroinfiltration to initiate TSWV-Cas12a-crPDS1:GFP infection in N. *benthamiana,* but most plants were significantly less susceptible to *Agrobacterium* transformation. This embodiment explored the possibility of using recombinant virus particles to infect other plants through mechanical transmission with the following experimental steps (FIG. 10A):
(1) Agroinfiltrated N. *benthamiana* to rescue TSWV-Cas12a-crPDS1:GFP.
(2) Collect freshly infected N. *benthamiana* leaves and ground in inoculation buffer (0.01 mM sodium sulfite, 0.01 mM potassium phosphate, pH 7.0; 0.5% sodium sulfite [wt/vol]), 0.01 M 2-mercaptoethanol, 1% (wt/vol) Celite 545 (Thermo Fisher Scientific), and 1% (wt/vol) Carborundum 320 grit (Thermo Fisher Scientific)) to prepare leaf sap inocula.
(3) The leaves of the plants to be inoculated were first evenly dusted with Carborundum 320 grit.
(4) The leaves were gently rubbed by hand with saps kept on ice; Alternatively, the leaf saps were centrifuged at 12,000 rpm for 10 min, and 0.1% (v/v) surfactant silwet-70 was added to the supernatant and then loaded in a pressurized spray gun. The air pressure pump was adjusted to 40 PSI and then aimed at the plant leaf that had been dusted with Carborundum at a distance of 3-5 cm and sprayed the inocula.
(5) Spray sterile water to wash away the residual Carborundum on the leaves after sap inoculation.
(6) Detecte recombinant virus infection by symptom observation, fluorescence imaging, western blot, and RT-PCR, and detecting gene editing at target loci using PCR/RE and/or Sanger sequencing.

Healthy N. *benthamiana,* potato, ground cherry, cotton, and lettuce seedlings at appropriate ages were inoculated with TSWV-Cas12a-crPDS1:GFP saps according to the mechanical inoculation method described above. After 8-16 days of inoculation, N. *benthamiana,* potato, cotton, and lettuce plants that were inoculated through mechanical rubbing and pressurized spraying showed infection symptoms. Green fluorescent were detected in the upper uninoculated tissues under fluorescence microscopy (FIG. 10B) . Western blot analysis showed that TSWV N, Cas12a, and GFP proteins were detected in the leaf tissues of systemically infected N. *benthamiana* plants (FIG. 10C) . The PCR/RE method described in Example 6 was used to detect the editing efficiency in N. *benthamiana.* The mutation frequencies at the *PDS* target locus was 47-71% in two randomly selected plants inoculated by mechanical rubbing and pressurized spraying (FIG. 10D), which was comparable to the editing frequency in plants inoculated by agroinfiltration (43-55%; Example 6).

Since the crPDS1 target site was conserved in the *PDS-A* and *PDS-B* homoeologs in the allotetraploid genome of N. *benthamiana* and tobacco (FIG. 11A), the above recombinant virus leaf saps were also rub-inoculated onto tobacco plants. Approximately at 8 dpi, green fluorescence was observed in the upper uninoculated leaves. Symptoms such as green spots, yellowing of leaf veins and leaf crinkling appeared (FIG. 11B), and the expression of TSWV N, Cas12a and GFP proteins confirmed (FIG. 11C) . Using specific primers to amplify genomic DNA spanning the tobacco target loci (Table 2), PCR/RE analysis showed that the mutation frequency at the NtPDS target loci was up to 56% (FIG. 11D).

These results indicate that the TSWV-based vectors are broadly applicable to diverse crop hosts for somatic genome editing through mechanical inoculation.

### Example 8: Application of TSWV-Cas12a-crRNA-mediated gene editing in tomatoes and peppers.

The crPDS2 (5'-**TTTC**TGCTGTTAACTTGAGAGTCCAAG-3' (SEQ ID NO: 13) (bold letters indicate PAM and the underlined bases refer to HinfI restriction site) targeting the tomato *SlPDS* gene is shown in FIG. 12A. The pS_{(+)crPDS2:GFP} vector described in Example 4 contains a crRNA sequence that targets this site. *Agrobacterium tumefaciens* carrying pL₍₊₎ₒₚₜ, pM_{(+)Cas12a}, pS_{(+)crPDS2:GFP}, pGD-p19, pGD-HcPro, pGD-γb, and pGD-NSs vectors were mixed in appropriate ratio and infiltrated into leaf tissues of N. *benthamiana.* Freshly infected N. *benthamiana* plants were used to prepare leaf sap inocula for mechanical transmission to tomato seedlings . The tomato plants began to show symptoms of chlorosis, ring spots, and crinkling in upper uninoculated leaves at about 12 dpi, and GFP fluorescence was observed under fluorescence microscopy (FIG. 12B). At 15 dpi, total proteins of infected leaf tissues were extracted for western blot analysis, and TSWV N, Cas12a and GFP proteins were detected (FIG. 12C) . The leaves from six randomly selected infected tomato plants were used to amplify genomic DNA using the target site-specific primers (Table 2). PCR/RE analysis indicated that the mutation frequencies at the *PDS* target region in recombinant virus-infected tissues ranged from 53-89% (FIG. 12D).

The genus *Capsicum* includes several species, such as chili pepper (*C. frutescens*), sweet pepper (*C*. *annuum*), and habanero pepper (C. *chinense)* . Further, the crPDS3 target sequence (5'-**TTTC**TGCTGTCAACTTGAGAGTCCAAG -3', bold letters indicate PAM, and the underlined bases refer to HinfI restriction site) (SEQ ID NO: 14), which is conserved in all of the above pepper plants, was selected to target the *PDS* gene (FIG. 13A) . The pS_{(+)crPDS3:GFP}, as well as the pL₍₊₎ₒₚₜ, pM_{(+)Cas12a}, pGD-p19, pGD-HcPro, pGD-γb, and pGD-NSs vectors were delivered into the leaves of N. *benthamiana* by agroinfiltration as described in Example 6. The obtained infected leaf saps were used to inoculate pepper seedlings at the 2-leaf stage. The upper uninoculated leaves of these infected pepper plants showed chlorosis and etiolation with GFP expression seen at 12 dpi (FIG. 13B). Western blot analysis confirmed that the above symptoms were caused by recombinant virus infection (FIG. 13C). PCR/RE analysis (primers shown in Table 2) indicated that the mutation frequencies at the crPDS3 target loci ranged from 87-94% (FIG. 13D). High-throughput sequencing (HTS) analysis showed that the mutation frequencies at the target sites in infected chili pepper, sweet pepper, and habanero pepper were 51.5%, 64.0% and 49.3%, respectively.

The above experiment results demonstrated that TSWV-Cas12a-crRNA could infect a variety of *Solanaceae* crops by mechanical inoculation and produce somatic genome editing.

### Example 9: Application of TSWV-Cas-crRNA-mediated gene editing in ground cherry.

Ground cherry (*Physalis alkekengi* L.) is a plant in the genus *Physalis*, family Solanaceae, and is phylogenetically related to tomato. It has been shown that mutation of *ERECTA (ER)* genes in both tomato and *Physalis alkekengi* leads to shorter internodes in plants (Kwon et al. Rapid customization of Solanaceae fruit crops for urban agriculture. Nat Biotechnol, 2020, 38: 182-188 ) . The ground cherry *ER* gene target crER: 5'-**TTTA**ATGTTAAACCTTTAGTGTCCTTT -3' (bold letters are PAM, SEQ ID NO: 88) was selected to construct pS_{(+)crER:GFP} according to the method described in Example 4. The pS_{(+)crER:GFP}, pL₍₊₎ₒₚₜ, pM_{(+)Cas12a}, pGD-p19, pGD-HcPro, pGD-γb, and pGD-NSs vectors were delivered into the leaves of N. *benthamiana* by agroinfiltration as described in Example 6, and the obtained infected leaf saps were used to inoculate ground cherry seedlings at the two true-leaf stage through sap inoculation. The upper uninoculated leaves of the infected ground cherry plants showed leaf chlorosis and slight curling, with GFP expression detected around 10 dpi (FIG. 14A). Western blot analysis confirmed that the viral N, GFP and Cas12a proteins were detected in the virus-infected tissues (FIG. 14B). HTS analysis showed that the mutation frequency at the crER target site in the virus-infected tissues was 52.9% (FIG. 14C).

The above experimental results demonstrate that the recombinant TSWV-Cas12a-crRNA vector can infect ground cherry by mechanical inoculation and produce target gene editing.

### Example 10: Application of the TSWV-Cas12a-crRNA vector to peanut and soybean gene editing

Peanut (*Arachis hypogaea)* is an allotetraploid with genomes derived from two ancestral wild species, *Arachis duranensis,* and *Arachis ipaensis.* The designed target sequence crPDS4 (5'-**TTTG**ACAGAAAACTGAAGAACACACATAT (G) -3' (SEQ ID NO: 15) (bold letters are PAM; with the underlined sequence is a NdeI restriction site), is conserved in two homoeologs of the peanut PDSgene (FIG. 15A) . Following the method of the above embodiments, recombinant viruses carrying the Cas12a nuclease sequence were recovered from N. *benthamiana* leaves after agroinoculation and then transmitted to peanut seedlings at the four-leaf stage through sap inoculation. Since peanut leaves have a thick waxy layer, an additional 0.01 M of β-mercaptoethanol was added to the inoculum buffer to improve the permeability of the inoculum. At 10 day after mechanical inoculation, green fluorescence was observed in some systemic leaf tissues of peanut plants showing leaf chlorosis, yellowing, and plant dwarfing (FIG. 15B). Meanwhile, the expression of the TSWVN, Cas12a, and GFP proteins were detected in systemic leaf tissues (FIG. 15C). The PCR/RE method was used to detect the *PDS* target site editing, which showed that the insertion and deletion (indel) mutations frequencies varied among 48-59% in systemic leaf tissues of three randomly selected infected peanut plants (FIG. 15D).

Since the crPDS4 target locus is also conserved in the soybean *PDS* gene (FIG. 16A), the above recombinant viruses were also used to inoculate soybean plants mechanically. Symptomatic leaves of the N. *benthamiana* infected with recombinant virus were homogenized in inoculum buffer, and the supernatant was collected after centrifugation at 12,000 rpm for 1 min and injected into soybean cotyledons 4 days after germination using a 1-mL syringe with a needle. A distinct green fluorescence was observed near the cotyledon injection site at 4 dpi, and the fluorescence spread to the surrounding tissues by 8 dpi. In contrast, soybean cotyledons injected with healthy plant saps had only a background of red fluorescence (FIG. 16B). Expression of the TSWV N, Cas12a and GFP proteins were detected in total protein samples of the above-inoculated cotyledon tissues (FIG. 16C) . Meanwhile, the target site sequences were amplified from total DNA samples using the primers shown in Table 2. PCR/RE assays showed that the indel mutation frequencies of target genes were 27-46% in the cotyledon samples of the three plants (FIG. 16D).

The above experimental results indicate that the recombinant TSWV vector can infect plant hosts belonging to taxonomically diverse families by mechanical inoculation and efficiently produce targeted DNA editing.

### Example 11: Broad applicability of the TSWV vector for genome-editing of sweet pepper varieties

The crPDS 6 target sequence (5'-**TTTC**TGCTGTCAACTTGAGAGTCCAAG -3; bold letters are PAM, SEQ ID NO: 99) conserved in the *PDS* gene of all 10 varieties of sweet pepper (C. *annuum*) was selected and engineered into pS_{(+)crPDS6:GFP} vector as described in Example 4. V-Cas12a-crPDS6:GFP was recovered and inoculated to pepper plants according to the method described in Example 4 and Example 8. Symptoms, including leaf chlorosis and mottling, were observed in the upper non-inoculated leaves of these plants at approximately 7 dpi. Total proteins were extracted from systemically infected leaf tissues, and the expressions of TSWV N, Cas12a, and GFP proteins were detected by western blot analysis (FIG. 17A). All varieties were effectively systemically infected by the virus vector, although some differences in the infection efficiencies were noticed (FIG. 17B). Total DNA was extracted from young systemically infected leaf tissues, and DNA sequences spanning the target site region were amplified and determined by HTS. The sequencing results showed that the editing efficiencies were in the range of 50.7-64.4%, without a statistically significant difference among different varieties (FIG. 17B) . The above results show that the recombinant TSWV vector can effectively infect different sweet pepper varieties and produce targeted genome editing.

### Example 12: Broad applicability of the TSWV vector for genome-editing of peanut varieties

The crPDS5 target site sequence (5'-AATGCAATGTATATTGATTGCCT**TAAA** -3'; PAM sequence in bold, SEQ ID NO: 98) conserved in both homoeologs of *PDS* in all ten peanut varieties was selected and engineered into pS_{(+)crPDS5:GFP} vector as described in Example 4. Plants of 10 peanut varieties were inoculated by the method described in Example 10. Symptoms of TSWV were observed in non-inoculated peanut leaves approximately 7 days after inoculation (FIG. 18A) . Expression of viral N protein, as well as Cas12a and GFP, could be detected in systemically infected leaf tissues (FIG. 18B). Different peanut varieties were effectively infected by the viral vector systemically. However, there were some differences in the infection efficiencies (FIG. 18C) . Total DNA was extracted from infected young leaves, DNA sequences of the target locus region were amplified and high-throughput sequenced. The editing efficiencies ranged from 59.4-73.1% among different varieties with no statistical difference (FIG. 18C). These results indicate that recombinant TSWV gene editing vector can effectively infect different peanut varieties and produce targeted genome editing.

### Example 13: Multiplexed gene editing with TSWV-Cas12a-crRNA in N. benthamiana

Examples 6-12 above show that TSWV-Cas12a-crRNA can deliver Cas12a and a single crRNA, and this example provides a method for multiplexed gene editing using the vector delivering Cas12a and multiple crRNAs. The N. *benthamiana* crPDS1 target site shown in Example 6 was selected, as well as the crFucT (5'-**TTT**GGAACAGATTCCAATAAGAAGCCT - 3'; SEQ ID NO: 86) and crDCL2 (5'- **TTT**GAGAAGCTATGCTTATCTTCTTCG - 3'; SEQ ID NO: 87) target sites (Table 3) . According to the method described in Example 4, pS_{(+)crPDS1:GFP}, pS_{(+)crFucT:GFP} and pS_{(+)crDCL2:GFP} were constructed for targeting these individual sequences. Simultaneously, A tandem repeat of crRNA sequence in the configuration "DR-Spacer 1-DR-Spacer 2-DR" for targeting the crPDS1 and crFucT sites (SEQ ID NO: 135) was chemically synthesized. Additionally, a triplet crRNA repeats in the configuration "DR-Spacer 1-DR-Spacer 2-DR-Spacer 3-DR" for targeting the crPDS1, crFucT, and crDCL2 (SEQ ID NO: 136) were also synthesized (FIG. 19A) . The synthesized fragments were cloned into the pS_{(+)GFP} plasmid through SpeI digestion to produce the pS_{(+)crPDS1&FucT:GFP} and pS_{(+)crPDS1&FucT&DCL2:GFP} plasmids. After virus replication, the mRNA transcript produced from the S genome containing the crRNA sequences can be cleaved by the M genome-expressed Cas12a protein to release one or more mature crRNA molecules (FIG. 19A).

The above plasmids, along with pL₍₊₎ₒₚₜ, pM_{(+)Cas12a}, and binary plasmids encoding the VSRs were transformed into *Agrobacterium* strain EHA105. Mixtures of bacterial cultures were agroinoculated to N. *benthamiana* according to the methods described in Example 6. Plants were systemically infected starting at 7 dpi and showed leaf curling, crinkling, mottling, and chlorosis symptoms (FIG. 19B) . Total DNA was extracted from systemic leaves of infected plants. The genomic DNA sequences spanning the target loci were amplified, and the target gene editing efficiencies in virus-infected tissues were determined using HTS methods . The results showed that the editing efficiency of the multiple-gRNA vectors for each target was similar to that of the corresponding single-gRNA editing vectors (FIG. 19 C) . The above results indicate that multiplex crRNA can be efficiently expressed using this tandem expression approach to form multiple CRISPR/Cas12a complexes for multiplexed gene editing of plant genomes.

### Example 14: Design and construction of TSWV-Cas9-gRNA gene editing vector

The TSWV-Cas9-gRNA vector construction strategy involves two main steps. Firstly, the GP sequence in the TSWV M genome is replaced by the sequence encoding SpCas9 to produce the genomic cDNA vector pM_{(-)Cas9} and the antigenomic cDNA vector pM_{(+)Cas9}. Second, the RFP coding sequence and gRNA sequence are inserted into the pS₍₊₎ vector in place of the NSs coding frame to generate the pS_{(+ )RFP:sgRNA} vector. In this design, the gRNA sequence produced from the S genome mRNA transcripts will be embedded in viral non-coding sequences. It has been previously demonstrated that such gRNA sequences can bind to the Cas9 proteins and then be cleaved by cellular RNases to release the mature gRNA molecules (Mikami et al., Plant Cell Physiol, 2017, 58: 1857-1867; Ma et al., Nat Plants, 2020, 6: 773-779) . The methods for specific constructs are detailed below.

### 1) Construction of the pM_{(-)Cas9} and pM_{(+)Cas9} vectors

Using a plasmid containing the N. *benthamiana* codon-optimized Cas9 sequence (Yin et al., Scientific Reports, 2015, 5: 14926) as a template, which also includes the nuclear localization signal and the 3× Flag antigen epitope sequence (SEQ ID NO: 11) on each side of the Cas9 sequence, we amplified the 3× Flag-NLS-Cas9-NLS fragment using the primers Cas9/F and Cas9/R. The GP coding sequence in the pM₍₋₎ vector was removed by PCR amplification using the primers M 3'UTR/F and Cas9/M IGR/R. The above two fragments having a 15-bp homologous sequence at both ends were ligated to produce the pM_{(-)Cas9} vector using an In-Fusion cloning reagent.

Using pM_{(-)Cas9} as a template, the recombinant M genomic cDNA fragment was amplified using primers 35S/M 3'UTR/F and HDV/M 5'UTR/R and ligated with the StuI/SmaI-digested pCB301-2µ-HDV vector fragment by In-Fusion cloning to generate the antigenomic cDNA vector pM_{(+)Cas9}.

### 2) Construction of the pS_{(+)RFP:gRNA} series vectors

First, the S antigenomic cDNA vector pS_{(+)RFP:gGFP} was constructed for the expression of the gRNA molecule targeting the gGFP target loci (5'- GATACCCAGATCATATGAAGCGG-3'; bolded sequence is PAM) (SEQIDNO: 16) of the *mGFP5* in the N. *benthamiana* 16c line. The pS_{(+)GFP} was used as a template to amplify the fragment excluding the *GFP* gene using S primers 5'UTR/F and S IGR/R, and the pGD-RFP was used as a template to amplify the RFP fragment using primers S 5'UTR/RFP/F and RFP/R, and the gRNA sequence (SEQ IDNO: 22) targeting the gGFP loci was chemically synthesized, which contains SpeI/BamHI digestion sites on each sides and also contains 15 nt homologous sequence to facilitate In-Fusion cloning. The above three DNA fragments were ligated by an In-Fusion cloning reagent to generate pS_{(+)RFP:gGFP}.

The gPDS1 target sequence (5'-GGACTCTTGCCAGCAATGCT**TGG-**3'), which is conserved in the *PDS* genes of several *Solanaceae* plants, was selected according to the CRISPR-P (http://cbi.hzau.edu.cn/cgi-bin/CRISPR) online software and inserted into pS_{(+)RFP:gGFP} vector using the SpeI and BamHI restriction sites to obtain pS_{(+)RFP:gPDS1}.

### Example 15: Editing the mGFP5 with TSWV-Cas9-gRNAN. benthamiana 16c plants

The results of Example 4 demonstrate that efficient recovery of recombinant viral vectors necessitates the use of relatively high concentrations of the M antigenomic cDNA plasmids due to the insertion of the large Cas coding sequences that can affect the packaging of the M genome. Therefore, *Agrobacterium tumefaciens* EHA105 strains carrying pL₍₊₎ₒₚₜ, pM_{(-)Cas9} or pM_{(+)Cas9}, and pS_{(+)RFP:gGFP} were mixed in a ratio of 2: 10: 1, plus equal volume mixtures of *Agrobacterium tumefaciens* carrying the VSR expression vectors (pGD-p19+ pGD-HcPro+ pGD-γb+ pGD-NSs), in the final concentration (OD₆₀₀) detailed in the table shown below:

| OD₆₀₀ | pL_{(+)op t} | pM_{(-)Cas9} | pM_{(+)Cas9} | pS_{(+)RFP:gGF P} | VSRs |
|---|---|---|---|---|---|
| Group 1 | 0.1 | 0.5 | - | 0.05 | 0.05 |
| Group 2 | 0.1 | - | 0.5 | 0.05 | 0.05 |

At about 10 days post-inoculation, both group 1 and group 2 infiltrated plants began to exhibit symptoms of virus systemic infection, suggesting TSWV infection, including leaf mottling, chlorosis, and curling. The infiltrated plants were monitored for a period of 20 days, and it was observed that the virus infection rate in Group 1 was slightly higher than that of Group 2 (FIG. 21).

*N. benthamiana* 16c plants systemically infected with the recombinant virus displayed red fluorescence in upper leaves, whereas the healthy control plants did not. Consequently, the RFP-expression leaf tissues showed significantly suppressed *GFP* expression (FIG. 22A) . Western blot analysis revealed high levelsofexpressionofN, Cas9, andRFP in virus-infected tissues in groups 1 and 2, but the accumulation of GFP protein in the same tissues was notably lower than that in control plants (FIG. 22B) . These results suggest that the Cas9/gRNAcomplex expressed by the recombinant virus suppressed *mGFP5* expression.

To confirm the gene editing at the mGFP5 target site, the genomic DNA region spanning the gGFP target site (5'-GATACCCAGATCATATGAAG**CGG**-3';SEQ ID NO: 16; NdeI restriction site underlined) PCR was amplified using the specific primers listed in Table 2. PCR/RE analysis indicated that the TSWV-Cas9-RFP:gGFP-infected plant tissues had a mutation frequency of approximately 56-60%, while healthy control plants showed no editing (FIG. 22C). Sanger sequencing analysis of the amplified products revealed that the editingproducedmostly deletions ranging from 1-12 bases (FIG. 22D).

The above results indicate that expression of either M genomic or antigenomic RNAs can support recombinant viral rescue and Cas9 protein expression and that the RFP:gRNA fusion sequence inserted within the S genome expresses a fluorescent reporter gene for tracking viral infection and also produces functional gRNA forming a complex with Cas9 protein to yield gene editing at the target sequence.

### Example 16: Editing of endogenous PDS genes in N. benthamiana and tobacco with TSWV-Cas9-gRNA

As depicted in FIG. 23A, the gPDS1 target sequence (5'-GGACTCTTGCCAGCAATGCT**TGG**-3') ; PAM in boldfaced letters and BsrDI site underlined) (SEQ ID NO: 17) is conserved in both *PDSa* and *PDSb* homoeologs of N. *benthamiana.* To rescue the recombinant virus, pL₍₊₎ₒₚₜ, pM_{(-)Cas9}, pS_{(+)RFP:gPDS1}, and VSRs (pGD-p19+pGD-Hc-Pro+pGD-γb+pGD-NSs) vectors were agroinoculated into the leaves of N. *benthamiana* according to the method in Example 15. Approximately 10 days after agroinfiltration, the upper young leaves of N. *benthamiana* exhibited leaf curling and chlorosis (FIG. 23B). Meanwhile, the expressions of viral N and Cas9 proteins were detected (FIG. 23C). The primers listed in Table 2 were used to amplify the target site sequences, which can simultaneously amplify both *PDSa* and *PDSb* sequences . PCR/RE assays indicated that the editing frequency at the gPDS1 target site in the infected tissues from four plants was as high as 74-98% (FIG. 23D).

The above gPDS1 target was conserved in both homoeologs of tobacco *PDSa* and *PDSb* (FIG. 24A) . The recombinant virus produced in N. *benthamiana* was inoculated into tobacco by saps inoculation, resulting in the development of a systemic infection in approximately 8 dpi. The infected plants exhibited faded-green spots on systemic leaves, veins yellowing, and leaf crinkling (FIG. 24B) . Western blot analysis showed that both N and Cas9 proteins were expressed in infected plants (FIG. 24C). PCR amplification and BsrDI digestion analysis using the primers specific to the tobacco gPDS target regions (Table 2) revealed that the mutation frequency o in three randomly selected plants ranged from 84-94% (FIG. 24D), which was comparable to the efficiencies observed in N. *benthamiana.* These results demonstrate that the TSWV-Cas9-gRNA vector can effectively infect different hosts through agroinfiltration or mechanical inoculation and produce target gene editing at high efficiency.

### Example 17: Multiplexed gene editing with TSWV-Cas9-gRNA in N. benthamiana.

Examples 14-16 have demonstrated that the TSWV-Cas9-gRNA system can deliver Cas9 and a single gRNA. In this example, we present a method for multiplexed gene editing using the vector to deliver Cas9 protein and multiple gRNAs. Specifically, we targeted the N. *benthamiana* gPDS2 (5'-TTGGTAGTAGCGACTCCATG**GGG-**3'; SEQ ID NO: 89), gRDR6 (5'- **CCC**CTCCTGACTCTTACCCAACT- 3'; SEQ ID NO: 90), and gSGS3 (5'- ACAAGAGTGGAAGCAGTGCT**GGG** - 3'; SEQ ID NO: 91) target loci (Table 3) . To this end, we constructed the TSWV S plasmids pS_{(+)gRDS2:GFP}, pS_{(+)gRDR6:GFP}, and pS_{(+)gSGS3:GFP}, containing a single gRNA, as described in Example 14. Additionally, we chemically synthesized a tandem gRNA sequence targeting both gPDS2 and gRDR6 (SEQ ID NO: 137), and a triplet gRNA tandem sequence targeting gPDS2, gRDR6, and gSGS3 (SEQ ID NO: 138) (FIG. 25A). These synthesized DNA fragments were cloned into the SpeI site in the pS_{(+)GFP} plasmid to generate the pS_{(+)gNbPDS2&RDR6:GFP} and pS_{(+)gNbPDS2&RDR6&SGS3-1:GFP} plasmids. After virus replication, the primary gRNA transcripts produced by the S genome contain gRNA sequences. The Cas9 protein expressed by the M genome binds to the gRNA sequences, and the plant endogenous RNases cleave and process the precursor to release one or more mature gRNA molecules (FIG. 25A).

The plasmids mentioned above, along with pL₍₊₎ₒₚₜ, pM_{(+)Cas9}, and the VSRs constructs were transformed into *Agrobacterium* strain EHA105 and inoculated into N. *benthamiana* using agroinfiltration, as described in Example 14. By 7 dpi, some inoculated plants exhibited systemic infection and displayed symptoms such as leaf curling, crinkling, and mottling (FIG. 25B). The total DNA of the infected plant leaf tissues was extracted, and the genomic DNA sequences spanning the target genes were amplified. The editing efficiencies of the multi-gRNA vectors for each target in virus-infected tissues were determined by HTS methods . The results indicated that the editing efficiency of the multiple-gRNA vectors for each target was similar to that of the corresponding single-gRNA editing vectors (FIG. 25 C). These findings suggest that multiple gRNAs can be efficiently expressed using this tandem expression approach, and multiplex CRISPR/Cas9 complexes can be generated for multiplexed plant gene editing.

### Example 18: Design and construction of the base editing vector TSWV-ABE-gRNA

The adenine base editing system comprises an adenine base editor (ABE) fusion protein and a guide RNA (gRNA) that selectively binds to the target site. As depicted in FIG. 26, the TSWV-ABE-gRNA viral vector, used for delivering ABE, was constructed by: i) replacing the GP coding sequence in the TSWV M genome with the ABE encoding sequence to produce the anti-genomic cDNA vector pM_{(+)ABE}; and ii) inserting the fusion sequence containing the GFP coding sequence and gRNA into the pS₍₊₎ vector to displace the NSs coding frame, yielding the pS_{(+)gRNA:GFP} vector. In this design, the 3' non-coding region of the GFP mRNA sequence generated by S genome transcription contains the gRNA sequence. Previous studies have demonstrated that the gRNA sequence can bind to homologous Cas9 proteins and the terminal sequences are cleaved by endogenous RNA processing enzymes to release mature gRNA molecules (Mikami et al., Plant Cell Physiol, 2017, 58: 1857-1867; Ma et al., Nat Plants, 2020, 6: 773-779). The specific constructs are described in detail below (FIG. 26).

### 1) Construction of pM_{(+)ABE}

The ABE coding sequence (SEQ ID NO: 84) was chemically synthesized by optimizing the TadA8e (V106W) sequence for N. *benthamiana* codon usage and fused with the nCas9, which includes NLS and 3×Flag antigen epitope sequences at the N-terminal end and 3xSV40 NLS sequence at the C-terminal end. The NLS-3×Flag-TadA8e (V106W)-nCas9-3xNLS fragment was amplified with the primers SpeI/Flag/F (SEQ ID NO: 124) and ABE/R (SEQ ID NO: 125) primers using the synthesized ABE DNA fragment as a template. The pM_{(+)Cas9} plasmid backbone fragment, excluding the Cas9 sequence, was amplified using primer pair M 3'UTR /F (SEQ ID NO: 65) and Cas9/M IGR/R (SEQ ID NO: 66) . The wo fragments having a 15-bp overlapping homologous sequence at each end were circularized using In-Fusion reagent to generate the pM_{(+)ABE} vector.

### 2) Construction of pS_{(+)GFP:gRNA} series vector

We selected the gPDSa target sequence (5 '-TGCAAATTGAGTTGGGAGG**AGG**-3'; SEQ ID NO: 92) in the *NbPDSa* gene, the gFucTa1 target sequence (5'- **CCT**CTTGAGAATGTTGCTATTGG-3'; SEQ ID NO: 93) in the *NbFucTa* gene, and the gBBLd target sequence (5'- GAAATCAGAGTAAGGTGCGG**AGG**-3'; SEQ ID NO: 97) in the *NbBBLd* gene, according to CRISPR-P website (http://cbi.hzau.edu.cn/cgi-bin/CRISPR). The above gRNA sequence fragments were then inserted into the SpeI site in the pS_{(+)SpeI:GFP} vector, resulting in the generation of the pS_{(+)gPDSa:GFP}, pS_{(+)gFucTa1:GFP}, and pS_{(+)gBBLd:GFP} vectors.

### Example 19: Multiplexed base editing with TSWV-ABE-gRNA in N. benthamiana.

The pS_{(+)gPDSa:GFP}, pS_{(+)gFucTa1:GFP}, and pS_{(+)gBBLd:GFP} constructs in Example 18 contain sgRNA sequences that target the gPDSa, gFucTa1, and gBBLd loci, respectively. The selected gPDSa targets only the *PDSa* homoeolog in N. *benthamiana. N. benthamiana* has four *FucT* genes, and the gFucTa1 targets only *FucTa. N. benthamiana* has five *BBL* genes, and the selected gBBLd is conserved in all five genes. In this Example, only the *BBLd* gene target was analyzed for base editing. *Agrobacterium* strains containing the three plasmids were individually mixed with *Agrobacterium* strains carrying the pL₍₊₎ₒₚₜ, pM_{(+)ABE}, pGD-p19, pGD-HcPro, pGD-γb, and pGD-NSs vectors at suitable ratios and infiltrated into *N. benthamiana* leaves for recombinant virus rescue, as described in Example 15. Approximately 10 days after agroinfiltration, the upper young leaves of N. *benthamiana* showed symptoms such as leaf curling and yellowing (FIG. 27A), and the expression of viral N protein, GFP, and ABE fusion protein was detected (FIG. 27B) . The target site sequences were amplified using the primers listed in Table 2 (SEQ ID NO: 112 and 115, SEQ ID NO: 122 and 123), followed by HTS and Sanger sequencing analysis. The TSWV-ABE-gRNA vectors induced base editing frequencies at the three target sites of gPDSa, gFucTa1, and gBBLd at varied frequencies ranging from 28% to 39% (FIG. 27C), and targeted A:T to G:C conversions within the base editing window (bases 4 to 9) of the target sequences were detected (FIG. 27D) . These results demonstrate that the TSWV vector can deliver adenine base editors to plants and efficiently induce targeted base conversions in the infected plant tissues.

### Example 20: Design and construction of the TSWV-CBE-gRNA base editing vector

The cytosine base editing system requires a cytosine base editor (CBE) fusion protein and a guide RNA (gRNA) that binds to the target. As depicted in FIG. 28, the strategy for constructing the TSWV-CBE-gRNA vector for delivering CBE involved two steps: i) replacing the GP sequence in the TSWV M genome with the sequence encoding CBE to generate the antigenomic cDNA vector pM_{(+)CBE}; ii) inserting the fusion sequence containing the GFP coding sequence and gRNA into the pS₍₊₎ vector to replace the NSs coding frame to produce the pS_{(+)gRNA:GFP} vector. The details of the specific construction methods are provided below.

### 1) Construction of pM_{(+)CBE} vector

The nucleotide sequence of the CBE (SEQ ID NO: 85) containing the codon-optimized hA3A sequence with the nCas9 sequence and the UGI sequence was chemically synthesized. The resulting sequence contained NLS and 3×Flag antigen epitope sequence at the N-terminus of the hA3A sequence and NLS at the C-terminus of the UGI sequence. To construct the pM_{(+)CBE}, the NLS-3×Flag-hA3A-nCas9-UGI-NLS fragment was amplified using the primers SpeI/F (SEQ ID NO: 124) and CBE/R (SEQ ID NO: 126). The pM_{(+) Cas9} vector was used as a template to obtain the fragment excluding the Cas9 sequence via PCR using the primers M 3'UTR/F and Cas9/M IGR/R. The two resulting fragments shared a 15-bp homologous sequence at each end and were ligated using an In-Fusion reagent to generate the pM_{(+)CBE} vector.

### 2) Construction of the pS_{(+)GFP:gRNA} vectors

We selected the target sequence for the endogenous genes in N. *benthamiana* using the CRISPR-P software (http://cbi.hzau.edu.cn/cgi-bin/CRISPR) . The selected target sequences for the *RDR6a* gene were 5'-**CCA**ATATCTAGGCGTGAGGGATT-3 ' (gRDR6a1; SEQ ID NO: 95) and 5'- **CCC**GACTTCATGGGGAAGGAGGA-3' (gRDR6a2; SEQ ID NO: 96) . For the *FucTa* gene, the selected target sequence was 5'- CACACCTTCCTCCAGGAGAT**AGG**-3' (gFucTa2; SEQ ID NO: 94). The sgRNA sequence fragments were then inserted into the SpeI site of the pS_{(+)SpeI:GFP} vector to generate pS_{(+)gRDR6a1:GFP}, pS_{(+)gRDR6a2:GFP}, and pS_{(+)gFucTa2:GFP}•

### Example 21: Base editing endogenous genes with TSWV-CBE-gRNA in N. benthamiana

The pS_{(+)gRDR6a1:GFP}, pS_{(+)gRDR6a2:GFP}, and pS_{(+)gFucTa2:GFP} vectors described in Example 20 each contain gRNA sequence that can target a single target site. The selected gRDR6a1 and gRDR6a2 target only the N. *benthamiana RDR6a. N. benthamiana* has four *FucT* genes, and the selected gFucTa2 only targets *FucTa* gene. *Agrobacterium* strains containing the individual plasmids described above, and the pL₍₊₎ₒₚₜ, pM_{(+)CBE}, pGD-p19, pGD-HcPro, pGD-γb, and pGD-NSs vectors were mixed in appropriate ratios and infiltrated to leaves of N. *benthamiana* for recombinant virus rescue. Approximately 10 days after agroinfiltration, the upper young leaves of N. *benthamiana* showed symptoms of leaf curling and chlorosis (FIG. 29A) . The expression of N protein, GFP, and the CBE fusion protein were detected (FIG. 29B) . The target site sequences were amplified using the primers (SEQ ID NO: 116-121) shown in Table 2. HTS and Sanger sequencing analysis showed that the TSWV-CBE-gRNA vector induced base editing frequencies ranging from 55% to 82% at the three target sites of gRDR6a1, gRDR6a2, and gFucTa2 (FIG. 29C). Moreover, the base editing activity of the target sequences within the editing window (bases 2 to 13) produced a C to T (or G to A) base conversion (FIG. 29D). These experimental results demonstrate that the TSWV-CBE-gRNA vector can efficiently deliver cytosine base editors to plants and generate targeted base conversions in the infected plant tissues.

### Example 22: Regeneration of N. benthamiana mutant plants from leaf tissues infected with the TSWV vector

To clarify whether the tissues infected with the TSWV-Cas12a-crRNA vector could be regenerated to obtain mutant plants, infected leaves in Example 6 were collected for regeneration through tissue culture. The specific experimental procedure involved the following steps:
1) Sequentially rinse the leaves with sterile water, followed by surface disinfection with 70% ethanol for 15 s, surface disinfection with 0.1% mercuric chloride (v/v) for 8 min, and then rinse the leaves with sterile water 3 times.
2) Cut the leaves into discs of 1-cm² and placing the explants in antibiotic-free MS differentiation medium (4.4 g/L MS [Duchefa Biochemie], 30 g/L sucrose, 1 mg/L cytokinin 6-BA, 4 g/L agar, pH 5.8). The explants were then placed in a 25°C incubation room with 12 h light/12 h dark for about 2-3 weeks.
3) When the differentiated shoots have grown to a suitable size, transfer the differentiated shoots to MS rooting medium (4.4 g/L MS, 3 g/L sucrose, 7 g/L glucose, 4 g/L agar, pH 5.8) and continue to culture for about 3 weeks.
4) After the seedlings had produced robust roots, the culture flasks were acclimated with sterile double-distilled water (dd H₂O) for 4-5 days and then transplanted to soil.

After 10 days of culturing of virus-infected leaf tissues in a differentiation medium, calli were induced on some explants and further differentiated into leaves and shoots over time. The regenerated plants were either albino or green seedlings. The green seedlings rooted normally and were transplanted to soil. In contrast, albino seedlings failed to root (FIG. 30A) . Two green seedlings (M0-1/-2) and one albino seedling (M0-3) were selected, and genomic DNA was exacted from these plants for PCR/RE analysis. Consistent with the phenotype, the target site PCR products from the albino plants were resistant to HinfI digestion, indicating that the restriction sites in all four *PDS* homeoalleles were disrupted. PCR products amplified from two green seedlings were incompletely digested by HinfI, indicating the presence of partially mutated *PDS* alleles (FIG. 30B). Sanger sequencing and genotyping analysis of the PCR products further confirmed that all four *PDS* alleles of albino seedlings (M0-3) had base deletions causing frameshift. In contrast, one and two mutant *PDS* alleles were present in the M0-1 and M0-2 green seedlings, respectively (FIG. 30C) . These results suggest that *N*. *benthamiana* mutant plants can be regenerated from the TSWV-Cas12a-crRNA-infected somatic cells.

In a similar experiment, leaves were collected from the *N*. *benthamiana* plants systematically infected with the TSWV-Cas9-RFP:gGFP vector shown in Example 15 to regenerate mutant plants through tissue culture. The same tissue culture method mentioned above was employed to regenerate plants from the infected cells. Fluorescence imaging analysis revealed that none of the three regenerated plants expressed the *mGFP5* transgene (FIG. 31A) . Sanger sequencing analysis of the target sequences of the regenerated plants showed that both M0-1 and M0-2 had a homozygous 1-nucleotide insertion, and M0-3 had a homozygous 1-nucleotide deletion at the *mGFP5* target site (FIG. 31B) .

### Example 23: Regeneration of tomato mutant plants from leaf tissues infected with the TSWV vector

The tomato leaves systemically infected with V-Cas12a-crPDS2 : GFP in Example 8 were collected and rinsed with sterile water. The leaves were then surface disinfected using 70% ethanol for 15 seconds, followedby immersion in 0.1% mercuric chloride (v/v) for 8 minutes. The sterilized leaves were rinsed 3-5 times with sterile water and blotted with sterile filter paper. The main veins and leaf edges were trimmed off, and the remaining leaves were cut into 0.5-cm² pieces. The leaf pieces were placed in an antibiotic-free MS differentiation medium and cultured for 7 days at 25°C in darkness. Afterward, they were transferred to a 12-hour light/12-hour dark cycle and tested separately for the four combinations of plant hormones as follows.

| | **IAA** | **6-BA** |
|---|---|---|
| Group 1 | 0.1 mg/L | 2 mg/L |
| Group 2 | 0.2 mg/L | 2 mg/L |
| Group 3 | 0.1 mg/L | 3 mg/L |
| Group 4 | 0.2 mg/L | 3 mg/L |

After approximately 22 days of culture, the explants in experimental Groups 1 and 2 began to show signs of leaf differentiation. In contrast, in Groups 3 and 4, the edges of the leaves displayed enlarged calli that failed to differentiate into buds or leaves (FIG. 32A) . Additionally, some of the callus tissues exhibited an albino phenotype, indicating that the *PDS* gene had been edited and inactivated (FIG. 32B, upper panel) . The differentiated shoots grown to a suitable size were transferred to MS rooting medium (containing 0.1 mg/L IAA) for growth. After 2-3 weeks of further culture on the MS rooting medium, all shoots generated robust root systems (FIG. 32B, lower panel) .

Five regenerated tomato seedlings were randomly selected, and their genomic DNA was extracted. Subsequently, PCR/RE was performed to detect mutations at the *PDS* target loci. PCRproducts amplified from the M0-1 andM0-4 plants were partially resistant to HinfI digestion, indicating the presence of edited *PDS*genes. PCR products from the M0-2 plant, however, showed complete resistance to HinfI digestion, which indicated that all four *PDS* homeoalleles had been successfully edited. The PCR products from M0-3 and M0-5 plants were sensitive to HinfI digestion, indicating that the target genes were not edited (FIG. 32C).

### Example 24 : Improving regeneration of virus-free mutant plants by antiviral treatment

Plant regeneration through culturing the leaf tissues infected with the TSWV vectors are usually ineffective in eliminating the virus. The embodiment investigated the effect of antiviral treatment during plant regeneration on virus clearance using leaf tissues infected with the TSWV-Cas12a-crRNA vector targeting the *N. benthamiana* crPDS1 site. As depicted in FIG. 33, leaf tissues of *N. benthamiana* plants infected with this viral vector system were used as explants and grew on a medium containing 40 mg/L ribavirin, or 40 mg/L favipiravir, or 1.2 mg/L remdesivir, or no antiviral agent (Mock), according to the method described in Example 22. After 35 days of culturing, explants together with induced callus were transferred to an induction medium without antiviral agents until day 55, and then the regenerated seedlings were transferred to a rooting medium. Three independent biological replicates were included in each group, and 30 plants were randomly selected in each biological replicate of each group for subsequent analysis . Total RNAwas extracted fromM0 regenerated seedlings, and RT-PCR was conducted using the TSWV *N* specific primers N/F (CTATGGATTACCTCTTGATGATGC; SEQ ID NO: 131) and N/R (TTAAGCAAGTTCTGCAAGTTTTG; SEQ ID NO: 132) to examine the virus clearance rate. The primers Nb/actin/F (CAATCCAGACACCTGTACTTTCTCTC; SEQ ID NO: 133) and Nb/actin/R (AAGCTGCAGGTATCCATGAGACTA; SEQ ID NO: 134) for amplification of the *NbActin* cDNA served as an internal control. The results showed that TSWV was not detected in any regenerated plants in the ribavirin treatment group, indicating a 100% virus clearance rate. In contrast, the mock treatment had only 4.4% of virus-free regenerated plants. The remdesivir and favipiravir treatment groups had 15.6% and 28.9% virus clearance rates, respectively (FIG. 34A and B).

Albino plants were observed during tissue culture in the different treatment groups (FIG. 35A), indicating that all four alleles of the *PDS* gene were edited in these plants. To further evaluate the effect of drug treatment on the efficiency of gene editing in the regenerated plants, DNA sequences containing the target loci were PCR amplified using specific primers (SEQ ID NO: 70 and 71), and the resulting amplicons were high-throughput sequenced. The mutation type of each plant was analyzed, and the editing efficiency of each group was calculated. We used the following definitions to call the putative genotype: wild-type (WT) with editing efficiency (indels%) of 0-10.0%; chimeric (Chi) with 10% < indels% ≤ 35.0%; heterozygous (He) with 35.0% < indels% ≤ 80.0%, i.e., one allele is edited at the target site but the other allele is wild-type; homozygous (Ho) with indels% between 80.0% and 100.0% if both alleles have the same type of editing, and biallelic (Bi) if two alleles produced two different types of editing.

Statistical analysis of the mutant genotypes in the *PDSa* and *PDSb* in each group of M0 plants showed that the highest proportion of plants with mutations at the target site was observed in the ribavirin treatment group. The remdesivir treatment group had only a slight increase over the mock group, while the favipiravir group had the lowest mutation rates (FIG. 35B) . Further analysis found that, compared to the mock group, the proportion of Ho/Bi types in the ribavirin group increased in both homoeologs, while the proportion of Chi types decreased; the total editing efficiency of the remdesivir group was comparable to that of the mock group, but the percentage of Ho/Bi type was slightly increased in the target of *PDSb* gene and the percentage of Chi type was decreased in both homoeologs; conversely, the total editing efficiency in the favipiravir group was lower than that of the mock group, but the percentage of Ho/Bi types in all mutant genotypes was significantly higher than that of the mock group (FIG. 35B).

To investigate the inheritance pattern of the different mutation genotypes from the M0 generation to next generation (M1), 19 M0 plants covering the four genotypes as previously defined, were selected, and self-pollinated seeds were obtained for each plant. Fourteen to sixteen M1 seedlings from each M0 line were randomly selected and analyzed for mutation genotypes at their respective target loci of *PDSa* and *PDSb* genes using HTS. The results are presented in Table 4. For M0 plants with WT and Chi genotypes (0 < indels% ≤ 35.0%), their M1 progeny were found to be wild-type. When M0 plants were heterozygous (He), their offspring contained a 3:1 ratio of mutant/wild-type, consistent with Mendel's law. In contrast, when M0 plants were Bi/Ho genotypes, their M1 progeny all contained mutations . These results indicated that the mutation types of He and Ho/Bi in M0 plants were stably inherited by the offspring, while the Chi mutation was not heritable.

Furthermore, 10 M0 plants with He or Ho/Bi genotypes for both *PDSa* and *PDSb* genes were selected to set seeds, and the phenotypes of their progeny were counted after sowing the seedlings. M1 offspring plantsof some M0 lines showed a phenotype ratio of green seedlings to white seedlings that was approximately 3:1, such as RB-#54, while other lines had ratios of approximately 15:1, such as RD-#76 (FIG. 36A) . The phenotype ratios of each group were found to be consistent with Mendel's law, as confirmed by the chi-square goodness-of-fit test (FIG. 36B). In conclusion, the use of antiviral drugs during tissue culture can significantly enhance the efficiency of obtaining virus-free and mutation-bearing regenerated plants.

### Example 25: Regeneration of tobacco mutant plants from leaf tissues infected with the TSWV vector

This embodiment further investigates the impact of antiviral drug treatment on virus clearance and mutant plant recovery using tobacco leaf tissues infected with the TSWV vectors for tissue culture and regeneration. We used the recombinant TSWV-Cas12a-crRNA vector described in Example 24 as an example, which targets the crPDS1 target sites of both *PDS* genes in tobacco . The systemic leaves infected with this virus vector were used as explants for tissue culture and plant regeneration, following the same experimental protocol as described in Example 24. After 10 days of culturing virus-infected tissues in a differentiation medium, some explants showed induction of callus, which further developed into leaves and shoots over time. The regenerated plants exhibited two phenotypes: albino and green seedlings. The green seedlings rooted normally and were successfully transplanted to the soil, while the albino seedlings failed to root (FIG. 37A).

HTS analysis of the percentage of regenerated plants containing target editing in each drug treatment group indicated that, the percentage of regenerated plants carrying target mutations in the ribavirin treatment group was significantly higher than that in the control treatment and other drug treatment groups (FIG. 37B) . The distribution of mutant genotypes in the regenerated plants from each drug treatment group was further analyzed. Ribavirin treatment increased the proportion of various mutation types (FIG. 37C). Analysis of the presence of viral vectors in the regenerated plants revealed that similar to the results obtained in *N*. *benthamiana,* ribavirin treatment effectively cleared the virus from all of the regenerated plants. These findings suggest that TSWV-Cas12a-crRNA-infectedtobacco cells can be regenerated using tissue culture techniques to obtain mutant plants and that treatment with ribavirin greatly enhances virus clearance and increases the efficiency of obtaining regenerated plants with mutations.

### Example 26: Adverse effects of NSs on rescuing the recombinant TSWV vector and plant regeneration from virus-infected cells

In the above-described embodiment, the TSWV *NSs* gene encoding an RNA silencing suppressor (Takeda et al., FEES Lett, 2002, 532: 75-79) was replaced with a reporter gene. To investigate the effect of *NSs* on the rescue efficiency, virulence, and gene editing ability of the recombinant virus, the recombinant virus vector TSWV-Cas9-NSs: gPDS1 containing NSs gene was tested in this embodiment. This vector was similar to TSWV-Cas9-RFP: gPDS1, but the *NSs* gene was retained upstream of the crPDS1 gRNA sequence instead of the RFP reporter gene (FIG. 38A).

For this purpose, the S antigenomic vector pS_{(+)NSs:gPDS1} was constructed. The linearized plasmid fragment was obtained by PCR amplification of the pS₍₊₎ plasmid using the primers gPDS1/NSs/F and S IGR/R, and the fragment containing the gPDS1 gRNA sequence (SEQ ID NO: 23) was obtained by PCR amplification using the primers gPDS1/F and IGR/scaffold/R. These fragments were then ligated with an In-Fusion reagent to generate the pS_{(+)NSs:gPDS1} vector.

The following *Agrobacteri um* mixtures were infiltrated into *N. benthamiana* leaf tissues using the method described in Example 11.
TSWV-Cas9-RFP:gPDS1 rescue mixtures: pL₍₊₎ₒₚₜ, pM_{(-)Cas9}, pS_{(+)RFP:gPDS1}, pGD-p19 + pGD-HcPro + pGD-γb + pGD-NSs
TSWV-Cas9-NSs:gPDS1 rescue mixtures: pL(+)opt, pM(-)Cas9, pS(+)NSs:gPDS1, pGD-p19 + pGD-HcPro + pGD-γb
In this particular experiment, the TSWV-Cas9-NSs:gPDS1 vector was able to express the NSs protein, and therefore, the pGD-NSs transient expression vector was not included in the agrobacterial mixtures. The TSWV-Cas9-RFP:gPDS1 began to display systemic infection 8 days after agroinfiltration, while the TSWV-Cas9-NSs:gPDS1 did not show systemic infection until day 10. However, there was no significant difference in the symptoms produced by both recombinant viruses (FIG. 38B). By day 20 after agroinfiltration, all infiltrated plants were infected with TSWV-Cas9-RFP:gPDS1, whereas only 50% of the plants had rescued the recombinant TSWV-Cas9-NSs:gPDS1 (FIG. 38D). However, when saps from infected plants were use to mechanically inoculated healthy *N*. *benthamiana* plants, no significant difference in disease onset and infection efficiency was observed between the two recombinant virus systems (FIG. 38E). Furthermore, PCR/RE analysis showed that both recombinant viruses had comparable targeted gene editing efficiency in the leaves of infected plants (FIG. 38C).

Young leaf tissues infected with TSWV-Cas9-NSs:gPDS1 and TSWV-Cas9-RFP:gPDS1 were collected and cultured in media using the method described in Example 13. The TSWV-Cas9-NSs:gPDS1-infected leaf explants exhibited severe necrosis after 7 days of culturing and were essentially completely necrotic after 10 days of culturing. In contrast, leaf explants infected with TSWV-Cas9-RFP:gPDS1 did not exhibit severe necrosis and began to show callus differentiation by day 10 (FIG. 38F).

Based on the results described above, it can be concluded that the TSWV gene editing vector that retains the virulence factor gene *NSs* has a reduced rescue efficiency and induces severe necrosis of infected cells during tissue culture, ultimately hindering plant regeneration.

### Example 27: Recombinant TSWV vector deficient in GP is non-insect-transmissible

The TSWV glycoprotein (GP) is crucial for virion morphogenesis and insect transmission, as previously reported (Sin et al., Proc Natl Acad Sci U S A, 2005, 102: 5168-5173) . In the present invention, the GP gene is replaced by a heterologous sequence. To verify that the recombinant virus is unable to be transmitted by the insect vector thrips, *N*. *benthamiana* leaf tissues infected with the recombinant TSWV-Cas9-NSs:gPDS1 and wild-type TSWV (TSWV-WT) virus from Example 15 were used to inoculate sweet peppers plants mechanically. Pools of 24-hour-old first-instar thrips were given a 24-hour acquisition access period (AAP) on sweet peppers infected with TSWV or V-Cas12a-crCaPDS. Subsequently, groups of larvae were transferred to French kidney bean (*Phaseolus vulgaris*) pods to complete their development into adults. Adult thrips were transferred to test tubes containing healthy pepper leaf discs of 1.5 cm in diameter and allowed a 48-h inoculation entry period (IAP). After the IAP, thrips were collected in microcentrifuge tubes (10 thrips per tube) for RNA extraction, and the leaf discs were further incubated on the water surface of a microtiter plate at 27°C for 4 days before RNA extraction. The presence of TSWV was detected by RT-PCR using the primers listed in Table 1. As shown in the table below, the virus acquisition rate of thrips was 53%, and the transmission rate was 65% on sweet peppers infected with the wild-type virus, whereas the recombinant TSWV-Cas9-NSs:gPDS1 was unable to be acquired and transmitted by thrips.

| Virus | % of thrips infected with TSWV | % of transmission rate |
|---|---|---|
| TSWV-WT | 53% (21/40) | 65% (13/20) |
| TSWV-Cas9-NSs:gPDS1 | 0% (0/40) | 0% (0/20) |

**Table 1 Primers for plasmid construction and RT-PCR**

| **Name** | **Sequence (5' to 3')** |
|---|---|
| L(+)/F | ATATAAGGAAGTTCATTTCATTTGGAGAGGAGAGCAATCAGGTAACAACGATTTTAA (SEQ ID NO: 31) |
| L(+)/R | CGCGAGGAGGTGGAGATGCCATGCCGACCCAGAGCAATCAGGTACAACTAAAAC (SEQ ID NO: 32) |
| Lopt 5' UTR/F | GATTCTGATGAGGAAGAGGACACCGATTAGATTAAAAGTAATGCCTAACAATGC (SEQ ID NO: 33) |
| Lopt 3' UTR/R | CAATCAGCTTCTGGATTTTCTGGATGTTCATGTTTGCTTAAAATCGTTGTTACC (SEQ ID NO: 34) |
| M 5' UTR/F | TTTCATTTGGAGAGGAGAGCAATCAGTGCATCAG (SEQ ID NO: 35) |
| M 3' UTR/R | ATGCCATGCCGACCCAGAGCAATCAGTGCAAACAAAAACC (SEQ ID NO: 36) |
| M IGR/ScaI/F | TTGATGAAATATAGTACTTTTAATACCAC (SEQ ID NO: 37) |
| M IGR/R | CACCTGTTCCTGTAAGGCTGCTTGTCACAAGCT (SEQ ID NO: 38) |
| RFP/F | TGGCCTCCTCCGAGAACGTC (SEQ ID NO: 39) |
| RFP/R | TTACAGGAACAGGTGGTGGC (SEQ ID NO: 40) |
| RFP/M 3 ' UTR/ F | CTCGGAGGAGGCCATCTTATACGTCTGCTCAGATTATAATGG (SEQ ID NO: 41) |
| pCB-PmeI/R | AAACACTGATAGTTTAAACTGAAGGC (SEQ ID NO: 42) |
| 35S/M3'UTR/F | TTTCATTTGGAGAGGAGAGCAATCAGTGCAAACAAAAAC (SEQ ID NO: 43) |
| HDV/M5'UTR/R | ATGCCATGCCGACCCAGAGCAATCAGTGCATCAG (SEQ ID NO: 44) |
| 35S/S3'UTR/F | TTTCATTTGGAGAGGGAGCAATTGTGTCAATTTTATTCAAACC (SEQ ID NO: 45) |
| HDV/S 5' UTR/R | ATGCCATGCCGACCCAGAGCAATTGTGTCATAATTTTATTC (SEQ ID NO: 46) |
| S 5' UTR/F | TATGGTTATTGGTACTGTGTTC (SEQ ID NO: 47) |
| S IGR/R | TCTTGCCGTGTCCAGCTTTTC (SEQ ID NO: 48) |
| GFP/5' UTR/F | GTACCAATAACCATAATGGTGAGCAAGGGCG (SEQ ID NO: 49) |
| GFP/IGR/R | CTGGACACGGCAAGATTACTTGTACAGCTCGTCCATG (SEQ ID NO: 50) |
| BamHI/NSs/F | ATCTATCTCTGGATCCATGTCTTCAAGTGTTTATGAGTCG (SEQ ID NO: 51) |
| SalI/NSs/R | AACGAGCTCTGTCGACTTATTTTGATCCTGAAGCATATGC (SEQ ID NO: 52) |
| BamHI/NSm/F | ATCTCTGGATCCATGTTGACTTTTTTTGGTAATAAGGG (SEQ ID NO: 53) |
| SalI/NSm/R | CGAGCTCTGTCGACCTATATTTCATCAAAAGATAACTGAGC (SEQ ID NO: 54) |
| M UTR/Cas12a/F | GAGCAGACGTATAAGATGGATTACAAGGATGACGACGATAAGA (SEQ ID NO: 55) |
| Cas12a/R | TCACTTCTTTTTCTTAGCCTGTCCG (SEQ ID NO: 56) |
| Cas12a/M IGR/R | AAGAAAAAGAAGTGAGGCTGCTTGTCACAAGCTAAATTTG (SEQ ID NO: 57) |
| SpeI/GFP/F | ACTAGTATGGTGAGCAAGGGCGAGG (SEQ ID NO: 58) |
| IGR PacI/R | ATAAGTGTGTTTAATTAAAGTTTGC (SEQ ID NO: 59) |
| NOS Pvu1/F | CCAAATGTTTGAACGATCGGG (SEQ ID NO: 60) |
| S 5' UTR/R | ACTAGTTATGGTTATTGGTACTGTGTTCTTATTAC (SEQ ID NO: 61) |
| S UTR/RFP/F | GTACCAATAACCATAGGATCATGGCCTCCTCCGAGAACG (SEQ ID NO: 62) |
| Cas9/F | GAGCAGACGTATAAGGGATCCATGGATTACAAGGATCACGATG (SEQ ID NO: 63) |
| Cas9/R | CAAGCAGCCGTCGACTTACTTTTTCTTTTTTGCCTGGCC (SEQ ID NO: 64) |
| M 3' UTR/F | CTTATACGTCTGCTCAGATTATAATGG (SEQ ID NO: 65) |
| Cas9/M IGR/R | GTCGACGGCTGCTTGTCACAAGCTAAATTTG (SEQ ID NO: 66) |
| gPDS1/NSs/F | CAAGAGTCCGGATCCTTATTTTGATCCTGAAGCATATGC (SEQ ID NO: 67) |
| gPDS1/F | GGATCCGGACTCTTGCCAGCAATGCT (SEQ ID NO: 68) |
| IGR/scaffold/R | CTGGACACGGCAAGAACTAGTGCACCGACTCGGTGCCAC (SEQ ID NO: 69) |
| SpeI/Flag/F | GAGCAGACGTATAAGACTAGTATGGATTACAAGGACCACGA (SEQ ID NO:124) |
| ABE/R | GCAGCCGTCGACGCGTAGCTCCTACACCTTGCGC (SEQ ID NO:125) |
| CBE/R | GCAGCCGTCGACGCGTTACACTTTACGCTTCTTCTTAGG (SEQ ID NO:126) |
| N/F | CTATGGATTACCTCTTGATGATGC (SEQ ID NO: 131) |
| N/R | TTAAGCAAGTTCTGCAAGTTTTG (SEQ ID NO:132) |
| Actin/F | CAATCCAGACACCTGTACTTTCTCTC (SEQ ID NO: 133) |
| Actin/R | AAGCTGCAGGTATCCATGAGACTA (SEQ ID NO:134) |

**Table 2 Primers for mutation detection**

| **Target loci** | **Target plants** | **Forward primer sequence (5'-3')** | **Reverse primer sequence (5'-3')** | **Size (bp)** |
|---|---|---|---|---|
| crPDS1 | *N. benthamiana* | TAAAATGCCCCAAATTGGA CTTG (SEQ ID NO: 70) | GCTACTACCAAAACATAACAAA TTCC (SEQ ID NO: 71) | 145 |
| | *N. tabacum* | | | |
| crPDS2 | *S. lycopersicum* | GAGGTTTGTGGATCTTTTA GGC (SEQ ID NO: 72) | CCAGCAAAACATAACGAATTCC (SEQ ID NO: 73) | 323 |
| crPDS3 | *C. frutescens* | GCCTTCGGTTCAATTTGTT TGTC (SEQ ID NO: 74) | GTCACTACCACCAAAACATAAC G (SEQ ID NO: 75) | 441 |
| | *C. annuum* | | | |
| crPDS4 | *A. hypogaea* | TTGAGAAGTGAAACTGTGA AG (SEQ ID NO: 76) | TGTGAGGCACAAAGTTATAAGC (SEQ ID NO: 77) | 469 |
| crPDS4 | *G. max* | ATGGTTGTAATAGTTTTGG TAAATGG (SEQ ID NO: 78) | AGGGAGAGGATATCTTCTACAC (SEQ ID NO: 79) | 563 |
| gGFP1 | *N. benthamiana* | ATGAGTAAAGGAGAAGAAC TTTTCAC (SEQ ID NO: 80) | TTATTTGTATAGTTCATCCATGC C (SEQ ID NO: 81) | 717 |
| gPDS1 | *N. benthamiana* | ATGCGTTATTACTTCTACA GGAATTT (SEQ ID NO: 82) | ACTTGCTTTCTCATCCAGTCC (SEQ ID NO: 83) | 171 |
| | *N. tabacum* | | | |
| crFucT1 | *N. benthamiana* | CTGACTTTATGAATGTCCC ATC (SEQ ID NO: 100) | GCATAGTATTGAGCTGACTCC (SEQ ID NO: 101) | 194 |
| crDCL2 | *N. benthamiana* | AGTTAGAGGCATTGGAGAC G (SEQ ID NO: 102) | CTGAGCCACCAGAACAACC(SEQ ID NO: 103) | 173 |
| crER | *P. alkekengi* | GTTCTTGAGCTTTGGTCCT GTG (SEQ ID NO: 104) | AATGACAACTACAAACGTGG (SEQ ID NO: 105) | 170 |
| gPDS2 | *N. benthamiana* | GCTTATCTTTGGAGCTCGA G (SEQ ID NO: 106) | CGCATTCAAAACAAACCTTTAAA G (SEQ ID NO: 107) | 184 |
| gRDR6 | *N. benthamiana* | TGGGTTTGACAATGATGTC A (SEQ ID NO: 108) | GCATGAGGTACCACTTTTTC (SEQ ID NO: 109) | 180 |
| gSGS3 | *N. benthamiana* | CAGCAAAGCAGAAAGCTAT C (SEQ ID NO: 110) | ATCTGGATGACCCCAAGCTT (SEQ ID NO: 111) | 227 |
| gPDSa | *N. benthamiana* | GGGACATTTTTGGAGTGTA GC (SEQ ID NO: 112) | AGAGTTAAGAGTGGGAACTG (SEQ ID NO: 113) | 310 |
| gFucTa1 | *N. benthamiana* | CAACCCTCGATCACATTCA C (SEQ ID NO: 114) | ACACACACACACTAACACTAG (SEQ ID NO: 115) | 205 |
| gFucTa2 | *N. benthamiana* | TGGTGCATCTTCTAAGTAG CG (SEQ ID NO: 116) | ACTAAAAAAACCACGACATGC (SEQ ID NO: 117) | 179 |
| gRDR6a1 | *N. benthamiana* | TAGGTCCCAATGTCCATTC T (SEQ ID NO: 118) | AGCTAAGGATTGACCGACTC (SEQ ID NO: 119) | 175 |
| gRDR6a2 | *N. benthamiana* | GCAGAGCTTGCTGCTCTAG C (SEQ ID NO: 120) | CCCTCTGTATCACATACATC (SEQ ID NO: 121) | 242 |
| gBBL | *N. benthamiana* | AGCCAACTGTTATTATTGT ACC (SEQ ID NO: 122) | AACTGTTGGGCAAGAACCAG (SEQ ID NO: 123) | 305 |
| crPDS5 | *A. hypogaea* | GGTGTACCTGACCGAGTAA C (SEQ ID NO: 127) | GTGAGCTTGTCAAATTTATAC (SEQ ID NO: 128) | 184 |
| crPDS6 | *C. frutescens* | TATCCTCATGGCAGGTTTG AC (SEQ ID NO: 129) | CCACCAAAACATAACGAATTCC (SEQ ID NO: 130) | 175 |
| | *C. annuum* | | | |

**Table 3 List of target sites**

| **Guide RNA** | **Targeted sequence (5'→3')** | **Target plants** | **Targeted gene ID** | **SEQ ID NO.** |
|---|---|---|---|---|
| crPDS1 | TTTCTGCCGTTAATTTGAGAGTCCAAG | *N. benthamiana* | Niben101Scf14708g00023.1; | 12 |
| | | | Niben101Scf01283g02002.1; | |
| | | *N. tabacum* | Nitab4.5_0004950g0020.1; | |
| | | | Nitab4.5 0006338g0050.1 | |
| crFucT | **TTTG**GAACAGATTCCAATAAGAAGCCT | *N. benthamiana* | Niben101Scf01272g000140.1; | 86 |
| | | | Niben101Scf02631g00007.1 | |
| crDCL2 | **TTTG**AGAAGCTATGCTTATCTTCTTCG | *N. benthamiana* | Niben101Scf08272 | 87 |
| crPDS2 | **TTTC**TGCTGTTAACTTGAGAGTCCAAG | *S. lycopersicum* | Solyc03g123760.3.1 | 13 |
| crPDS3 | **TTTC**TGCTGTCAACTTGAGAGTCCAAG | *C**.** frutescens* | CA03g36860; | 14 |
| | | *C. annuum* | Capana03g000054 | |
| crPDS4 | **TTTG**ACAGAAAACTGAAGAACACATAT | *A. hypogaea* | AH14G02170; | 15 |
| | | | AH04G01420 | |
| crER | **TTTA**ATGTTAAACCTTTAGTGTCCTTT | *P. alkekengi* | Pg-t 64769 64772 | 88 |
| gGFP | GATACCCAGATCATATGAAG**CGG** | *N. benthamiana* 16c | GFP | 16 |
| gPDS1 | GGACTCTTGCCAGCAATGCT**TGG** | *N. benthamiana* | Niben101Scf14708g00023.1; | 17 |
| | | | Niben101Scf01283g02002.1; | |
| | | *N. tabacum* | Nitab4.5_0004950g0020.1; | |
| | | | Nitab4.5_0006338g0050.1 | |
| gPDS2 | TTGGTAGTAGCGACTCCATG**GGG** | *N. benthamiana* | Niben101Scf14708g00023.1; | 89 |
| | | | Niben101Scf01283g02002.1 | |
| gRDR6 | **CCC**CTCCTGACTCTTACCCAACT | *N. benthamiana* | Niben101Scf03832Ctg041; | 90 |
| | | | Niben101Scf12609Ctg016 | |
| gSGS3 | ACAAGAGTGGAAGCAGTGCT**GGG** | *N. benthamiana* | Niben101Scf03392Ctg069; | 91 |
| | | | Niben101Scf05468Ctg070 | |
| gPDSa | TGCAAATTGAGTTGGGAGTG**AGG** | *N. benthamiana* | Niben101Scf14708g00023.1 | 92 |
| gFucTa1 | **CCT**CTTGAGAATGTTGCTATTGG | *N. benthamiana* | Niben101Scf01272g000140.1 | 93 |
| gFucTa2 | CACACCTTCCTCCAGGAGAT**AGG** | *N. benthamiana* | Niben101Scf01272g000140.1 | 94 |
| gRDR6a1 | **CCA**ATATCTAGGCGTGAGGGATT | *N. benthamiana* | Niben101Scf03832Ctg041 | 95 |
| gRDR6a2 | **CCC**GACTTCATGGGGAAGGAGGA | *N. benthamiana* | Niben101Scf03832Ctg041 | 96 |
| gBBLd | GAAATCAGAGTAAGGTGCGG**AGG** | *N. benthamiana* | Niben101Scf05809Ctg001 | 97 |
| crPDS5 | AATGCAATGTATATTGATTGCCT**TAAA** | *A. hypogaea* | AH14G02170; | 98 |
| | | | AH04G01420 | |
| crPDS6 | **TTT**CTGCTGTCAACTTGAGAGTCCAAG | *C. frutescens* | CA03g36860; | 99 |
| | | *C. annuum* | Capana03g000054 | |
| Notes: | | | | |
| 1. For allotetraploid hosts (*N*. *benthamiana, N. tabacum, and A. hypogaea*), the most target sites are conserved in both homoeologs; However, the target sites marked with a are specific to a particular homoeolog, for example, NbPDSa-3 is not conserved in the NbPDS-B homoeolog. | | | | |
| 2. The PAM sequences are highlighted in bold. | | | | |

**Table 4 Summary of the mutation transmission rates from the M0 to M1 generation**

| M0 ID | Virus-infec ted | Genotype of M0 plants | | | Segregation of mutations in the M1 progeny | | | | |
|---|---|---|---|---|---|---|---|---|---|
| | | NbPDS- | Mutation type | Mutation rate (%) | No. of M1 plant analysed | WT | He | Ho/Bi | Transmission frequency (%) |
| CK-#27 | Yes | A | (-14/wt) He | 63.3 | 15 | 4 | 9 | 2 | 73.3 |
| | | B | Chi | 16.9 | 15 | 15 | 0 | 0 | 0.0 |
| CK-#46 | Yes | A | (-13/wt) He | 49.6 | 16 | 5 | 10 | 1 | 68.8 |
| | | B | WT | 0.0 | 16 | 16 | 0 | 0 | 0.0 |
| CK-#59 | Yes | A | (-13/-15) Bi | 100.0 | 16 | 0 | 0 | 16 | 100.0 |
| | | B | WT | 0.0 | 16 | 16 | 0 | 0 | 0.0 |
| CK-#75 | Yes | A | (-8/-9) Bi | 82.4 | 16 | 0 | 4 | 12 | 100.0 |
| | | B | (-8/wt) He | 42.9 | 16 | 8 | 7 | 1 | 50.0 |
| CK-#84 | Yes | A | Chi | 12.3 | 14 | 14 | 0 | 0 | 0.0 |
| | | B | (-1A/wt) He | 40.9 | 14 | 4 | 7 | 3 | 71.4 |
| RB-#17 | No | A | Chi | 32.9 | 16 | 16 | 0 | 0 | 0.0 |
| | | B | Chi | 18.8 | 16 | 16 | 0 | 0 | 0.0 |
| RB-#26 | No | A | WT | 0.0 | 15 | 15 | 0 | 0 | 0.0 |
| | | B | (-12/wt) He | 66.4 | 15 | 3 | 9 | 3 | 80.0 |
| RB-#36 | No | A | (-14/wt) He | 60.3 | 15 | 5 | 6 | 4 | 66.7 |
| | | B | (-8/-10) Bi | 100.0 | 15 | 0 | 0 | 15 | 100.0 |
| RB-#37 | No | A | (-7/-7) Bi | 82.8 | 15 | 0 | 0 | 15 | 100.0 |
| | | B | (-7/wt) He | 42.6 | 15 | 2 | 8 | 5 | 86.7 |
| RB-#41 | No | A | (-10/-14) Bi | 82.7 | 15 | 0 | 0 | 15 | 100.0 |
| | | B | (-7/-12) Bi | 100.0 | 15 | 0 | 0 | 15 | 100 |
| RB-#48 | No | A | (-6/-9) Bi | 100.0 | 14 | 0 | 0 | 14 | 100.0 |
| | | B | (-8/-8) Ho | 87.7 | 14 | 0 | 0 | 14 | 100.0 |
| RB-#54 | No | A | (-20/wt) He | 64.1 | 16 | 2 | 2 | 12 | 87.5 |
| | | B | (-10/wt) He | 59.6 | 16 | 3 | 10 | 3 | 81.3 |
| RB-#74 | No | A | (-8/-17) Bi | 81.9 | 14 | 0 | 7 | 7 | 100.0 |
| | | B | (-6/wt) He | 49.8 | 14 | 1 | 8 | 5 | 92.9 |
| RD-#35 | Yes | A | Chi | 24.4 | 16 | 16 | 0 | 0 | 0.0 |
| | | B | Chi | 24.4 | 16 | 16 | 0 | 0 | 0.0 |
| RD-#52 | Yes | A | WT | 0 | 16 | 16 | 0 | 0 | 0.0 |
| | | B | (-11/wt) He | 39.6 | 16 | 3 | 9 | 4 | 81.3 |
| RD-#67 | No | A | Chi | 24.0 | 16 | 16 | 0 | 0 | 0.0 |
| | | B | WT | 3.4 | 16 | 16 | 0 | 0 | 0.0 |
| RD-#75 | Yes | A | (-8/-9) Bi | 100.0 | 15 | 0 | 0 | 15 | 100.0 |
| | | B | (-9/wt) He | 66.8 | 15 | 4 | 6 | 5 | 73.3 |
| RD-#76 | Yes | A | (-11/-12) Bi | 100.0 | 16 | 0 | 0 | 16 | 100.0 |
| | | B | (-11/-14) Bi | 100.0 | 16 | 0 | 0 | 16 | 100.0 |
| RD-#84 | Yes | A | (-10/wt) He | 74.8 | 14 | 7 | 5 | 2 | 50.0 |
| | | B | (-13/-15) Bi | 100.0 | 14 | 0 | 0 | 14 | 100.0 |
| FP-#37 | No | A | (-7/wt) He | 59.7 | 14 | 6 | 4 | 4 | 57.1 |
| | | B | (-8/-18) Bi | 97.4 | 14 | 0 | 0 | 14 | 100 |
| Notes: | | | | | | | | | |
| 1. CK-, RB-, RD-, and FP- in M0 ID denote antiviral treatment, i.e., mock, ribavirin, and remdesivir, and favipiravir, respectively. WT, wild-type; Chi, chimeric; He, heterozygous; Bi, bi-allelic; Ho, homozygous. -x, deletion of x bases. | | | | | | | | | |
| 2. Mutation transmission frequency is expressed as the percentage of progeny with parent-derived mutations. According to Mendelian inheritance, M0 parents with germline heterozygous mutations would segregate at a ratio of 3:1 (75% transmission rate), while those with germline bi-allelic and/or homozygous edits would produce 100% of progeny with mutations. | | | | | | | | | |

## Claims

1. A method for modifying the genetic material of a plant cell without the need for introducing exogenous genetic material into the plant cell genome, comprising:
a) providing at least one plant cell to be modified;
b) providing a tospovirus vector comprising at least one polynucleotide sequence encoding a sequence-specific nucleic acid modifying enzyme;
c) introducing said tospovirus vector into said plant cell to express transiently said nucleic acid modifying enzyme;
d) wherein said sequence-specific nucleic acid modifying enzyme mediates the cleavage or modification of a specific genomic sequence in said plant cell, and wherein the cellular DNA repair machinery completes the modification of genetic material of said plant cell.

2. A method for producing a geneticallymodifiedplant without the need for introducing exogenous genetic material in the genome of the plant to be modified, comprising:
a) providing at least one cell of the plant to be modified;
b) providing a tospovirus vector comprising at least one polynucleotide sequence encoding a sequence-specific nucleic acid modifying enzyme;
c) introducing said tospovirus vector into said plant cell to express transiently said nucleic acid modifying enzyme;
d) wherein said sequence-specific nucleic acid modifying enzyme mediates the cleavage or modification of a specific genomic sequence in said plant cell, and wherein the cellular DNA repair machinery completes the modification of genetic material of said plant cell.
e) obtaining a genetically modified plant from said plant cell.

3. The method of claim 1 or 2, further comprising selecting at least a plant cell or a plant with genetic modifications, wherein said selection does not use a selectable marker, and wherein said modification comprises deletion, insertion, or substitution of one or more nucleotides, or a combination thereof, at the target DNA sequence.

4. The method of claim 1 or 2, wherein said sequence-specific nucleic acid modification enzyme is an endonuclease, its mutant forms or derivatives capable of achieving genome editing; preferably, wherein said sequence-specific nucleic acid modification enzyme is selected from the group of CRISPR/Cas nucleases; more preferably, *Streptococcus pyogenes* CRISPR/SpCas9 nuclease, or *Lachnospiraceae bacterium* CRISPR/LbCas12a (LbCpf1) nuclease, or an adenine base editor or cytosine base editor comprising a SpCas9 variant fused to a base deaminase.

5. The method of claim 1 or 2, wherein said tospovirus vector is derived from tomato spotted wilt virus (TSWV) ; preferably, said TSWV comprising the S, M, and L genome segments having at least 70%, at least 80%, at least 90%, at least 95%, at least 99% or 100% sequence homologies to the nucleic acid sequences as set forth in SEQ ID NO: 1, SEQ ID NO: 2, and SEQ ID NO: 3, respectively.

6. The method of claim 5, wherein the TSWV S segment comprises at least one heterologous polynucleotide sequence operably linked to viral mRNA transcriptional cis-element and encoding a component of sequence-specific nucleic acid modifying enzyme, preferably, wherein said nucleic acid modifying enzyme component is a chimeric CRISPR guide RNA polynucleotide molecule.

7. The method of claim 5 or 6, wherein the entire or partial sequence coding for non-structural protein (NSs) in said TSWV S genome segment is replaced with a heterologous chimeric polynucleotide sequence encoding said nucleic acid modifying enzyme component and a second heterologous polypeptide, optionally, a fluorescent reporter polypeptide.

8. The method of claim 6 or 7, wherein said heterologous polynucleotide sequence encodes a CRISPR guide RNA.

9. The method of claim 6 or 7, wherein said heterologous polynucleotide sequence encodes two or more guide RNAs.

10. The method of claim 5, wherein the TSWVM segment comprises at least one heterologous polynucleotide sequence operably linked to a viral mRNA transcriptional cis-element and encoding a component of the sequence-specific nucleic acid modifying enzyme; preferably a Cas polypeptide; more preferably, *Streptococcus pyogenes* SpCas9, *Lachnospiraceae bacterium* LbCas12a (LbCpf1), or an adenine base editor or cytosine base editor comprising a SpCas9 variant fused to a base deaminase.

11. The method of claim 5 or 10, wherein the entire or partial sequence coding for glycoprotein precursor (GP) in said TSWV M genome segment is replaced with a heterologous polynucleotide sequence encoding said nucleic acidmodifying enzyme component.

12. The method of claim 5, wherein the TSWVL segment comprises a polynucleotide sequence coding for a plant codon-optimized viral RNA-dependent RNA polymerase (RdRp), as preferably set forth in SEQ ID NO: 5.

13. The method of claim 1 or 2, wherein said method for introducing tospovirus vector into a plant cell is through delivery of polynucleotide constructs comprising the viral vector nucleic acid sequence into the plant cells under conditions sufficient to initiate recombinant virus infection; preferably, through agroinfection.

14. The method of claim 13, wherein said polynucleotide constructs further comprise one or more constructs encoding viral suppressor suppressors of RNA silencing, optionally selected from the group consisting of tomato bushy stunt virus p19, tobacco etch virus P1/Hc-Pro, barley stripe mosaic virus γb, or TSWV NSs.

15. The method of claim 13, wherein said polynucleotide constructs comprising the tospoviral vector nucleic acid sequence produce positive- or negative-sense viral RNA transcripts upon delivery into a plant cell; preferably, positive-sense RNA transcripts.

16. The method of claim 13, wherein the polynucleotide constructs comprising the TSWV L, M, and S segments are delivered into a plant cell in relative molar ratios of 1-2 (L): 2-10 (M): 1-2 (S), preferably in a relative ration of 1: 10: 2.

17. The method of any one of claims 13-16, wherein said polynucleotide constructs are plasmids, preferably *Agrobacterium* binary vectors; wherein the construct ratios are the ratios of *Agrobacterium* strains harboring the corresponding binary vectors.

18. The method of claim 1 or 2, wherein said method for introducing tospovirus vector into a plant cell is through infection of plant with recombinant virus particles, by optionally mechanical rubbing, pressurized spraying, or graft inoculation.

19. The method of any one of claims 1-18, wherein said tospovirus vector is an NSs-deficient, attenuated infectious viral vector.

20. The method of any one of claims 1-19, wherein said tospovirus vector is a GP-deficient, non-insect-transmissible viral vector.

21. The method of claim 1 or 2, wherein said plant to be geneticallymodified is selected from a natural or experimental host plant capable of supporting local or systemic infection of a tospovirus; preferably, selected from the group of *Nicotiana benthamiana, N. tabacum,* tomato (*Solanum lycopersicum*), sweet pepper (*Capsicum annuum*), chili pepper (*C. frutescens*), habanero pepper (*C*. *chinense*), peanut (*Arachis hypogaea*), potato (*Solanum tuberosum*)*,* ground cherry (*Physalisalkekengi*), soybean (*Glycine max),* cotton (*Gossypium hirsutum*), lettuce (*Lactuca sativa*), spinach (*Spinacia oleracea*), watermelon (*Citrullus lanatus*), cucumber (*Cucumis sativus*), broad bean (*Vicia faba*), black mung bean (*Vigna mungo*), green bean (*Vigna radiata*), or cowpea (*Vigna unguiculata*).

22. The method of claim 21, further comprising various plant varieties or genotypes of the tospovirus host plant species.

23. A method for obtaining virus-freeplants by tissue culture from cells infected by a tospovirus, comprising:
a) providing at least one plant cell infected with a tosposvirus;
b) subjecting said plant cells to tissue culture and regeneration on a medium containing an antiviral compound;
c) obtaining and characterizing a regenerated plant free of the virus.

24. The method of claim 23, wherein said antiviral compound is ribavirin.

25. The method of claim 23, wherein said antiviral compound is favipiravir.

26. The method of claim 23, wherein said antiviral compound is remdesivir.

27. A recombinant virus vector system, comprising:
a) a polynucleotide sequence of a tospovirus, and
b) at least a polynucleotide sequence encoding a sequence-specific nucleic acid modifying enzyme operably linked to a viral transcriptional cis-element;
wherein said tospoviral vector replicates and expresses said sequence-specific nucleic acid modifying enzyme upon introduction into a plant cell, and wherein said nucleic acid modifying enzyme mediates the cleavage or modification of a specific genomic sequence in said plant cell.

28. The recombinant viral vector system of claim 27, wherein said sequence-specific nucleic acid modification enzyme is a nuclease, its mutant form or derivatives capable of achieving genome editing; preferably, wherein said sequence-specific nucleic acid modification enzyme is selected from the group of CRISPR/Cas nucleases; more preferably, *Streptococcus pyogenes* CRISPR/SpCas9 nuclease, or *Lachnospiraceae bacterium* CRISPR/LbCas12a (LbCpf1) nuclease, or an adenine base editor or cytosine base editor comprising a SpCas9 variant fused to a base deaminase.

29. The recombinant viral vector system of claim 27, wherein said tospovirus vector is derived from tomato spotted wilt virus (TSWV) ; preferably, said TSWV comprising the S, M, and L genome segments having at least 70%, at least 80%, at least 90%, at least 95%, at least 99% or 100% sequence homologies to the nucleic acid sequences as set forth in SEQ ID NO: 1, SEQ ID NO: 2, and SEQ ID NO: 3, respectively.

30. The recombinant viral vector system of claim 27 or 29, wherein the TSWV S segment comprises at least one heterologous polynucleotide sequence operably linked to viral mRNA transcriptional cis-element and encoding a component of sequence-specific nucleic acid modifying enzyme, preferably, wherein said nucleic acid modifying enzyme component is a chimeric CRISPR guide RNA polynucleotide molecule.

31. The recombinant viral vector system of any one of claims 27, 29 or 30, wherein the entire or partial sequence coding for non-structural protein (NSs) in said TSWV S genome segment is replaced with a heterologous chimeric polynucleotide sequence encoding said nucleic acid modifying enzyme component and a second heterologous polypeptide, optionally, a fluorescent reporter polypeptide.

32. The method of claim 30 or 31, wherein said heterologous polynucleotide sequence encodes a CRISPR guide RNA.

33. The method of claim 30 or 31, wherein said heterologous polynucleotide sequence encodes two or more guide RNAs.

34. The recombinant viral vector system of claim 27 or 29, wherein the TSWV M segment comprises at least one heterologous polynucleotide sequence operably linked to a viral mRNA transcriptional cis-element and encoding a component of the sequence-specific nucleic acid modifying enzyme; preferably, a Cas polypeptide; more preferably, *Streptococcus pyogenes* SpCas9, *Lachnospiraceae bacterium* LbCas12a (LbCpf1), or an adenine base editor or cytosine base editor comprising a SpCas9 variant fused to a base deaminase.

35. The recombinant viral vector system of any one of claims 27, 29, or 34, wherein the entire or partial sequence coding for glycoprotein precursor (GP) in said TSWVM genome segment is replaced with a heterologous polynucleotide sequence encoding said nucleic acid modifying enzyme component.

36. The recombinant viral vector system of claim 27 or 29, wherein the TSWV L segment comprises a polynucleotide sequence coding for a plant codon-optimized viral RNA-dependent RNA polymerase (RdRp), as preferably set forth in SEQ ID NO: 5.

37. The recombinant viral vector system of claim 27 or 29, wherein said viral vector comprises polynucleotide constructs capable of forming an infectious tospovirus upon introduction into a plant cell.

38. The recombinant viral vector system of claim 37, wherein the polynucleotide constructs further comprise one or more constructs encoding viral suppressors of RNA silencing, optionally selected from the group consisting of tomato bushy stunt virus p19, tobacco etch virus P1/Hc-Pro, barley stripe mosaic virus γb, or TSWV NSs.

39. The recombinant viral vector system of claim 37, wherein saidpolynucleotide constructs comprising the tospoviral vector nucleic acid sequence produce positive- or negative-sense viral RNA transcripts upon delivery into a plant cell; preferably, positive-sense RNA transcripts.

40. The recombinant viral vector system of claim 37, wherein the polynucleotide constructs comprising the TSWV L, M, and S segments are delivered into a plant cell in relative molar ratios of 1-2 (L): 2-10 (M) : 1-2 (S), preferably in a relative ration of 1: 10: 2.

41. The recombinant viral vector system of any one of claims 37-40, wherein said polynucleotide constructs are plasmids, preferably *Agrobacterium* binary vectors; wherein the construct ratios are the ratios of *Agrobacterium* strains harboring the corresponding binary vectors.

42. The recombinant virus vector system of claim 27 or 29, wherein said method for introducing tospovirus vector into a plant cell is through plant infection with recombinant virus particles, by optionally mechanical rubbing, pressurized spraying, or graft inoculation.

43. The recombinant viral vector system of any one of claims 27-42, said tospovirus vector is an NSs-deficient, attenuated infectious viral vector.

44. The recombinant viral vector system of any one of claims 27-43, wherein said tospovirus vector is a GP-deficient, non-insect-transmissible viral vector.

45. Bacterial strains or recombinant viral particles comprising the recombinant viral vector system of any one of claims 27-44.

46. A kit comprising the recombinant viral vector system of any one of claims 27-44,
a) a polynucleotide construct composition comprising the nucleic acid sequence of the TSWV S, M and L genome segments comprising at least one heterologous polynucleotide sequence encoding a sequence-specific nucleic acid modifying enzyme;
b) Supporting expression constructs encoding one or more viral suppressors of RNA silencing; optionally selected from the group consisting of tomato bushy stunt virus p19, tobacco etch virus P1/Hc-Pro, barley stripe mosaic virus γb, or TSWV NSs;
wherein the viral vector components may be provided separately or in combination.

47. The kit of claim 46, further comprising a user manual or instruction.

48. Use of the recombinant viral vector system of any one of claims 27-44, the bacterial strains or recombinant viral particles of claim 45, the kit of any claims 46 or 47, or the method of any one of claims 1-26, in plant genome editing.

49. Genetically modified plants and their progeny produced by using the recombinant viral vector system of any one of claims 27-44 or by using the method of any one of claims 1-26.
